# EUROPEAN PATENT APPLICATION

(11) **EP 3 474 013 A1**
(43) Date of publication of application: **24.04.2019**
(21) Application number: 17813033.2
(22) Date of filing: 28.04.2017
(51) Int. Cl.: G01N 33/53, A23L 17/00, A61K 39/00, A61P 37/08, C12Q 1/48, C07K 14/47, C12N 15/09

(54) **FISH ALLERGY ANTIGEN**

(30) Priority: 16.06.2016 JP 2016119919
(71) Applicant: Hoyu Co., Ltd., Nagoya-shi, Aichi 461-8650 (JP)
(72) Inventor: MATSUNAGA, Kayoko, Toyoake-shi Aichi 470-1192 (JP); YAGAMI, Akiko, Toyoake-shi Aichi 470-1192 (JP); SHIMOJO, Naoshi, Nagakute-shi Aichi 480-1136 (JP)
(74) Representative: Mewburn Ellis LLP
(86) International application number: PCT/JP2017/017018
(87) International publication number: WO 2017/217128

(57) **Abstract**

The present invention provides novel antigens of an allergy to fish, methods and kits for diagnosing an allergy to fish, a pharmaceutical composition comprising the antigen, fish or fish eggs in which the antigen is eliminated, a processed product of the fish or the fish eggs or fish which lay the fish eggs or have hatched from the eggs, and a tester for determining the presence or absence of a fish antigen in an object of interest.

SEQUENCE LISTING

<110>  FUJITA ACADEMY
       Hoyu Co., Ltd.

<120>  Antigens that causes allergy to fish

<130>  FA1621-17013

<150>  JP2016-119919
<151>  2016-06-16

<160>  149

<170>  PatentIn version 3.5

<210>  1
<211>  2685
<212>  DNA
<213>  Artificial Sequence

<220>
<223>  PREDICTED: alpha-actinin-3

<400>  1
atgacggcaa tcgaaactca ggtgcaatat ggctcctaca tgacttcgga gcaagtctac      60

atgacccaag aggatgattg ggacagggac cttctgttgg accctgcctg ggagaaacag     120

cagcgcaaga ccttcaccgc ctggtgcaac tctcacctgc gtaaagcggg cacacagatt     180

gagaacattg aggaggactt caggaatggg ctcaagctca tgttgctgtt agaggtcatc     240

tcaggtgaaa ggcttcccaa accagacaaa ggcaaaatgc gtttccacaa aatcgccaac     300

gtgaacaagg ccctggattt catctgcagc aagggagtca agctggtgtc catcggtgcc     360

gaggagattg tggatggtaa tgtgaagatg accctgggga tgatctggac catcattctg     420

cgtttcgcca tccaggacat ctctgtagag gagacctctg ctaaggaggg tctgttgctg     480

tggtgccaga ggaagactgc cccctacagg aatgtcaatg tgcagaactt ccacatcagt     540

tggaaggatg gcctggcact gtgtgccctc atccacagac acagacctga cctcattgac     600

tactccaaac tgcgcaagga tgaccccatg ggcaatctca acactgcctt tgaggtggca     660

gagaagtacc tggacatccc caagatgttg gatgcagaag atattgtgaa caccccgaag     720

cccgacgaga aagccatcat gacctatgtg tcctgcttct atcatgcctt cgctggagcc     780

gagcaggccg agacagctgc caataggatc tgcaaggtgt tggctgtcaa ccaggagaac     840

gagaagctca tggaggagta tgagaagctg gccagtgagc tgctggagtg gatccgtcgc     900

accatcccct ggctggagaa ccgcgtggct gagcagacca tgcgcgccat gcagcagaag     960

ctggaggact tccgtgacta ccgtcgcgtc cacaagcctc ccaaggtgca ggagaagtgt    1020

cagctggaga ttaacttcaa caccctgcag accaagctga ggctgagcaa caggcccgcc    1080

ttcatgccct ccgagggcaa gatggtgtcg gacattgcca atgcctggaa gggtctggag    1140

caggtagaga agggctatga ggagtggctg ctcactgaga tcagacgcct ggagaggctg 1200

gaccacctgg ctgagaagtt caagcagaag tcttccctgc atgagtcctg gacctcaggt 1260

aaggtggagc tgctgtccat gaaggactat gagtctgcct cactgatgga gatccgtgcc 1320

ctgatgagga agcacgaggc gtttgagagc gacctggctg cccaccagga cagagtggag 1380

cagattgctg ccatcgccca ggagctcaat gagctggact atcatgatgc cgtcaccatc 1440

aacgcccgct gccagggtat ttgtgaccag tgggacaacc tgggcacctt gacccagaag 1500

aggagagact cactggagcg tgtggagaag ctgtgggaga ccatcgacca gctgtacctg 1560

gagtttgcca agagagcagc ccccttcaac aactggatgg acggagccat ggaggactta 1620

caggacatgt tcattgtgca cagcattgag gagatccaga gtctgatcac agcccatgac 1680

cagttcaagg ccaccctgcc agaggcagat aaggagcgcg ttgcaaccat gggaatccag 1740

aatgagatcg tgaagatcgc tcagacctac ggcatcaagc tgtcaggagt caacccctac 1800

accaaccttt ccccccagga catcaccgac aaatgggatg ctgtgaaaca cctggtgccc 1860

ctcagggatc agatgctgca ggaggaggtg gccaggcagc agtccaacga gaggctgagg 1920

cgccagttcg ctgcccaggc caacatcatc ggaccctgga tccagaccaa gatggaggag 1980

atcagccatg tgtctgtgga catcgccggc tccctggagg aacagatgag caacctgaag 2040

cagtacgagc agaacatcat caactacaag tgcaacatcg acaagctgga gggagaccac 2100

caactcagcc aggagtccct catctttgac aacaagcaca ccaactacac catggagcat 2160

gtgcgtgtgg gctgggagca gctcctcacc accatcgccc gcaccatcaa cgaggtggag 2220

aaccagatcc tgacccgaga tgccaagggc atcagccagg agcagctcaa cgagttcaga 2280

gcctccttca accactttga caggaagaga aatggtatga tggacccaga tgacttccgt 2340

gcctgtctca tctccatggg ctacgatctg ggtgaggtgg agtttgcccg catcatgacg 2400

ctggtggatt ccaacaacac aggtgtggtg accttccagg ccttcatcga cttcatgacc 2460

cgcgagacgg ccgagacaga caccgccgat caggtcatgg cctcattcaa gatcctggcc 2520

tctgacaaga catacatcac agtggaagaa ctgcgcaggg agctgccccc agagcaggcc 2580

gactactgca tcagccgcat gaccagttac atcggcagcg gcgcaccccc aggcgccctg 2640

gactacatct ccttctccag tgccctgtac ggagagagcg actta 2685

<210> 2
<211> 895
<212> PRT
<213> Artificial Sequence

<220>
<223> PREDICTED: alpha-actinin-3

<400> 2

Met Thr Ala Ile Glu Thr Gln Val Gln Tyr Gly Ser Tyr Met Thr Ser
1               5                   10                  15

Glu Gln Val Tyr Met Thr Gln Glu Asp Asp Trp Asp Arg Asp Leu Leu
            20                  25                  30

Leu Asp Pro Ala Trp Glu Lys Gln Gln Arg Lys Thr Phe Thr Ala Trp
        35                  40                  45

Cys Asn Ser His Leu Arg Lys Ala Gly Thr Gln Ile Glu Asn Ile Glu
    50                  55                  60

Glu Asp Phe Arg Asn Gly Leu Lys Leu Met Leu Leu Leu Glu Val Ile
65                  70                  75                  80

Ser Gly Glu Arg Leu Pro Lys Pro Asp Lys Gly Lys Met Arg Phe His
                85                  90                  95

Lys Ile Ala Asn Val Asn Lys Ala Leu Asp Phe Ile Cys Ser Lys Gly
            100                 105                 110

Val Lys Leu Val Ser Ile Gly Ala Glu Glu Ile Val Asp Gly Asn Val
        115                 120                 125

Lys Met Thr Leu Gly Met Ile Trp Thr Ile Ile Leu Arg Phe Ala Ile
    130                 135                 140

Gln Asp Ile Ser Val Glu Glu Thr Ser Ala Lys Glu Gly Leu Leu Leu
145                 150                 155                 160

Trp Cys Gln Arg Lys Thr Ala Pro Tyr Arg Asn Val Asn Val Gln Asn
                165                 170                 175

Phe His Ile Ser Trp Lys Asp Gly Leu Ala Leu Cys Ala Leu Ile His
            180                 185                 190

Arg His Arg Pro Asp Leu Ile Asp Tyr Ser Lys Leu Arg Lys Asp Asp
        195                 200                 205

Pro Met Gly Asn Leu Asn Thr Ala Phe Glu Val Ala Glu Lys Tyr Leu
    210                 215                 220

Asp Ile Pro Lys Met Leu Asp Ala Glu Asp Ile Val Asn Thr Pro Lys
225                 230                 235                 240

Pro Asp Glu Lys Ala Ile Met Thr Tyr Val Ser Cys Phe Tyr His Ala
                245                 250                 255

Phe Ala Gly Ala Glu Gln Ala Glu Thr Ala Ala Asn Arg Ile Cys Lys
            260                 265                 270

Val Leu Ala Val Asn Gln Glu Asn Glu Lys Leu Met Glu Glu Tyr Glu
        275                 280                 285

Lys Leu Ala Ser Glu Leu Leu Glu Trp Ile Arg Arg Thr Ile Pro Trp
    290                 295                 300

Leu Glu Asn Arg Val Ala Glu Gln Thr Met Arg Ala Met Gln Gln Lys
305                 310                 315                 320

Leu Glu Asp Phe Arg Asp Tyr Arg Arg Val His Lys Pro Pro Lys Val
                325                 330                 335

Gln Glu Lys Cys Gln Leu Glu Ile Asn Phe Asn Thr Leu Gln Thr Lys
            340                 345                 350

Leu Arg Leu Ser Asn Arg Pro Ala Phe Met Pro Ser Glu Gly Lys Met
        355                 360                 365

Val Ser Asp Ile Ala Asn Ala Trp Lys Gly Leu Glu Gln Val Glu Lys
    370                 375                 380

Gly Tyr Glu Glu Trp Leu Leu Thr Glu Ile Arg Arg Leu Glu Arg Leu
385                 390                 395                 400

Asp His Leu Ala Glu Lys Phe Lys Gln Lys Ser Ser Leu His Glu Ser
                405                 410                 415

Trp Thr Ser Gly Lys Val Glu Leu Leu Ser Met Lys Asp Tyr Glu Ser
            420                 425                 430

Ala Ser Leu Met Glu Ile Arg Ala Leu Met Arg Lys His Glu Ala Phe
        435                 440                 445

Glu Ser Asp Leu Ala Ala His Gln Asp Arg Val Glu Gln Ile Ala Ala
    450                 455                 460

Ile Ala Gln Glu Leu Asn Glu Leu Asp Tyr His Asp Ala Val Thr Ile
465                 470                 475                 480

Asn Ala Arg Cys Gln Gly Ile Cys Asp Gln Trp Asp Asn Leu Gly Thr
                485                 490                 495

Leu Thr Gln Lys Arg Arg Asp Ser Leu Glu Arg Val Glu Lys Leu Trp
            500                 505                 510

Glu Thr Ile Asp Gln Leu Tyr Leu Glu Phe Ala Lys Arg Ala Ala Pro
        515                 520                 525

Phe Asn Asn Trp Met Asp Gly Ala Met Glu Asp Leu Gln Asp Met Phe
    530                 535                 540

Ile Val His Ser Ile Glu Glu Ile Gln Ser Leu Ile Thr Ala His Asp
545                 550                 555                 560

Gln Phe Lys Ala Thr Leu Pro Glu Ala Asp Lys Glu Arg Val Ala Thr
                565                 570                 575

Met Gly Ile Gln Asn Glu Ile Val Lys Ile Ala Gln Thr Tyr Gly Ile
            580                 585                 590

Lys Leu Ser Gly Val Asn Pro Tyr Thr Asn Leu Ser Pro Gln Asp Ile
        595                 600                 605

Thr Asp Lys Trp Asp Ala Val Lys His Leu Val Pro Leu Arg Asp Gln
    610                 615                 620

Met Leu Gln Glu Glu Val Ala Arg Gln Gln Ser Asn Glu Arg Leu Arg
625                 630                 635                 640

Arg Gln Phe Ala Ala Gln Ala Asn Ile Ile Gly Pro Trp Ile Gln Thr
                645                 650                 655

Lys Met Glu Glu Ile Ser His Val Ser Val Asp Ile Ala Gly Ser Leu
            660                 665                 670

Glu Glu Gln Met Ser Asn Leu Lys Gln Tyr Glu Gln Asn Ile Ile Asn
        675                 680                 685

Tyr Lys Cys Asn Ile Asp Lys Leu Glu Gly Asp His Gln Leu Ser Gln
    690                 695                 700

Glu Ser Leu Ile Phe Asp Asn Lys His Thr Asn Tyr Thr Met Glu His
705                 710                 715                 720

Val Arg Val Gly Trp Glu Gln Leu Leu Thr Thr Ile Ala Arg Thr Ile
                725                 730                 735

Asn Glu Val Glu Asn Gln Ile Leu Thr Arg Asp Ala Lys Gly Ile Ser
            740                 745                 750

Gln Glu Gln Leu Asn Glu Phe Arg Ala Ser Phe Asn His Phe Asp Arg

755                 760                 765

Lys Arg Asn Gly Met Met Asp Pro Asp Asp Phe Arg Ala Cys Leu Ile
    770                 775                 780

Ser Met Gly Tyr Asp Leu Gly Glu Val Glu Phe Ala Arg Ile Met Thr
785                 790                 795                 800

Leu Val Asp Ser Asn Asn Thr Gly Val Val Thr Phe Gln Ala Phe Ile
                805                 810                 815

Asp Phe Met Thr Arg Glu Thr Ala Glu Thr Asp Thr Ala Asp Gln Val
            820                 825                 830

Met Ala Ser Phe Lys Ile Leu Ala Ser Asp Lys Thr Tyr Ile Thr Val
        835                 840                 845

Glu Glu Leu Arg Arg Glu Leu Pro Pro Glu Gln Ala Asp Tyr Cys Ile
    850                 855                 860

Ser Arg Met Thr Ser Tyr Ile Gly Ser Gly Ala Pro Pro Gly Ala Leu
865                 870                 875                 880

Asp Tyr Ile Ser Phe Ser Ser Ala Leu Tyr Gly Glu Ser Asp Leu
                885                 890                 895

<210>  3
<211>  14
<212>  PRT
<213>  Artificial Sequence

<220>
<223>  PREDICTED: alpha-actinin-3 fragment

<400>  3

Glu Arg Val Ala Thr Met Gly Ile Gln Asn Glu Ile Val Lys
1               5                   10

<210>  4
<211>  3591
<212>  DNA
<213>  Artificial Sequence

<220>
<223>  EEF1A2 binding protein-like

<400>  4
atgtggaaaa agaaatcaaa gctcacggac cagacggcca ctggccaagt tgggatcaag      60

aagagatcaa aagtccctgg agttatgatc acgcagtatg tggagaaaat accagatggg     120

aaaagccacc ctgacttcac ccgcaagcct atcgcgttga ccattcagga gggtaaattc     180

gccttcttca aagccctggt tattggagat ccagaaccaa ccgtgacatg gggcagaaat 240

aatggagatg tgtcagatac atcaaaatat gtgacaaaat atgaccctgc tacacgtgag 300

cacttatttg agatggccaa tgtaaaacca gaacaagcag acacctacaa atgctttgca 360

gctaatgagt ttggaagagc agtggtcaca gtggtcctca atgttattga agttgggttc 420

aagaaagcac aagcggactc acaggtgcaa ccagaggcag ctgttgcaga ttttaaatct 480

gtattgaaga gaaaaagtaa aattcagccc aaaatggaaa agaaagaaga tggagaaata 540

gatccaagat tttgggaact cttgataagt gctgacaaga aagactatga gagcctcatg 600

ttggagtttg gagtcactga cttccgcttt atgctgaaga cactgaatga gattaagaag 660

gaaagagagg aagagcaagc acagttcatt gaaaacctag ctaacctgaa acctattgaa 720

gttggacccg atggctgtgc aaccttttca atagacatgg atctcattga acaaagcagc 780

aggatcttcc tctacaagga tggagtgatg attccataca gcaaggagtt gggagataca 840

atcaaacaca gcctaaagat agtgggccga aagtatcagt tcagcataag ggatctgttt 900

cctgatgatg ctgggctcta ccaggtggat gttgaggacg taaatgtatt ctccaccgat 960

tttaagattc ccatggtgga cttcctggtc aagattcagg agtgtaaggc catggagaga 1020

gaggatgctg tgtttgagtg tgtcctgtca cagcccttcg gcaagatcat gtgggttggc 1080

aagaacttac cattggaggc aggggataaa tatgatattg aggtttcaga agacaagctc 1140

atccacagac taatcattaa agacgttgct atggtggaca aaggcatcta tgccgctgtg 1200

gcaggaatca aatcttgcaa tgcctttctt gtggttgaag ccgacaaggg tgaacccggc 1260

aaaaagaaac aacgtaaaac cacaagggca ggaggagctg gagttgacct gacggcgatt 1320

gcccaagagc aggcagttaa aaacacggca gacagagagg tgctgaagga gaaggtgaaa 1380

gcaatcaatg acgagagagc ggctaatgcc actgcagcac ctgagacttc agctgaagct 1440

aaagctaaag ttaaggtggt agaggcttcc caaacaggag ctccaaaaca gggaccggca 1500

gttaaagggt ctgaccataa atccgtggat aatgagggat acagcattaa gagtggactc 1560

tcagatgtct ttgctctccg tggcaagaaa ggtgaactgg tttgtgagat gagccatgaa 1620

gttgatgggg cctggttcaa ggatggagag aagttatcca ccacagatgg aatagccata 1680

gtgaaagatg gaacgagaca cacgatgacc attcatagca gtagtgaaga cgacactgga 1740

gtctatcaca ttgaagcggg gggatttaaa tcagaggcaa aagtcactgt gggagaatta 1800

cctgccattg atgctgatga cctccacaag ttttctaagc ctgtgacaat aaaagtgggc 1860

cagaatgcat cctggaagat gccttataca ccacaggaca acttggaggt gaaatggttt 1920

aaggatggca aagagttgaa ggatggtggt ggggtgaagt tggtgaagga ggtcaagcac 1980

agccggctgc tgctccggga gtgtctgcgt tctgacgctg gggagatcaa gattcaactc 2040

aaaaacccct ttggcactat agaggccaca tctcgactga ttgtccttga caagcccggc 2100

ccaccagaag gcccggtgga aatcttggag accacctcca ctgtgattga gctgcagtgg 2160

ggcgctccta aggacgacgg tggctccccg gtgactaact acatcattga gcgccagcag 2220

ctgggacaga ccgtgtggaa gaagatgggt gatgtcgcag ctgacaaaac cacctacagg 2280

gacaggaatg tggtccatgg gaaactgtac atctacaata tctacgcagt gaacccagag 2340

gggaccagtg atgcactgca gactgaggaa acaatggctg gcgtattgat atttgctggc 2400

cgacctggag caccaaaagt ggtcagcgca tcaaagacct gcatcaacct gaaatgggag 2460

cccccagaag atgacggagg aattaagatt gatggctatc aactggaaaa acgcaaaaag 2520

gacacagctc agtggattgc tttgaaccca gtaactgagc ctattgaagt gctggagtac 2580

gcagtgaagg atgttgttga gggggcggag tatgagttca gggtatcggc catcaacgtt 2640

tctggggcgg gagagttcag tctcccctct gtgatggtga ctgcaaagaa tcccaacatg 2700

aggcctacat tcaaagatcc agaggacttc atggtgatca gggcgggaaa ctctgtgaga 2760

atcaaagttc tctatgaggc tgagcctcca ccggagatca cttggatgaa ggacaatgag 2820

cctgtatcca gttttataca gatcatcaac acagagggct gttcccagct tgtgatcccc 2880

tcaacaaaac gctctgattc aggaaattac accattgtgg ctaagaataa agttggagag 2940

gccagctttg acattgaggt cctagtcaca gatgagccaa agcctcctgg tgcagtggag 3000

ctggagcaga ttgtccatgg caaagttatt gtgtcctggg aggcctctcc agaccaggag 3060

ctggacaaca ggctgtatta catggtggcc gagcacgact ccagcacacg catgtggcac 3120

actgtggcag accgcatctt tgacaattca tacacagcca ataacatcat gcctggaagg 3180

gagtaccact tcaaaatcta tgccaagaac gacatgggca tgtcagaccc ctccatgtca 3240

cccacctggg gcatcaacag caacagaatt cctataaaca caaacacgcc agtggaagtc 3300

agctttgaga aaccaccatc tgtcctggtc cctctgaaga tgcactcgcc accaaaagga 3360

ttccagatgt acatgacctg cgccatccgg ggatgcccca cacccagcgt cacgtggcac 3420

ctgaacaacg tctgcatcaa tggtgacagc aactactaca tcaccaactc gtatggcgtg 3480

tgctccatgt acatccttag ggtcaggccc aaggacgccg gagaatacaa agttgtcgca 3540

gttaactcct tcggcaaggc agaatgctcc actaaacttg ttgttaaaga c 3591

<210> 5
<211> 1197
<212> PRT
<213> Artificial Sequence

<220>
<223> EEF1A2 binding protein-like

<400> 5

Met Trp Lys Lys Lys Ser Lys Leu Thr Asp Gln Thr Ala Thr Gly Gln
1               5                   10                  15

Val Gly Ile Lys Lys Arg Ser Lys Val Pro Gly Val Met Ile Thr Gln
            20                  25                  30

Tyr Val Glu Lys Ile Pro Asp Gly Lys Ser His Pro Asp Phe Thr Arg
        35                  40                  45

Lys Pro Ile Ala Leu Thr Ile Gln Glu Gly Lys Phe Ala Phe Phe Lys
    50                  55                  60

Ala Leu Val Ile Gly Asp Pro Glu Pro Thr Val Thr Trp Gly Arg Asn
65                  70                  75                  80

Asn Gly Asp Val Ser Asp Thr Ser Lys Tyr Val Thr Lys Tyr Asp Pro
                85                  90                  95

Ala Thr Arg Glu His Leu Phe Glu Met Ala Asn Val Lys Pro Glu Gln
            100                 105                 110

Ala Asp Thr Tyr Lys Cys Phe Ala Ala Asn Glu Phe Gly Arg Ala Val
        115                 120                 125

Val Thr Val Val Leu Asn Val Ile Glu Val Gly Phe Lys Lys Ala Gln
    130                 135                 140

Ala Asp Ser Gln Val Gln Pro Glu Ala Ala Val Ala Asp Phe Lys Ser
145                 150                 155                 160

Val Leu Lys Arg Lys Ser Lys Ile Gln Pro Lys Met Glu Lys Lys Glu
                165                 170                 175

Asp Gly Glu Ile Asp Pro Arg Phe Trp Glu Leu Leu Ile Ser Ala Asp
            180                 185                 190

Lys Lys Asp Tyr Glu Ser Leu Met Leu Glu Phe Gly Val Thr Asp Phe
        195                 200                 205

Arg Phe Met Leu Lys Thr Leu Asn Glu Ile Lys Lys Glu Arg Glu Glu
    210                 215                 220

Glu Gln Ala Gln Phe Ile Glu Asn Leu Ala Asn Leu Lys Pro Ile Glu
225                 230                 235                 240

Val Gly Pro Asp Gly Cys Ala Thr Phe Ser Ile Asp Met Asp Leu Ile
                245                 250                 255

Glu Gln Ser Ser Arg Ile Phe Leu Tyr Lys Asp Gly Val Met Ile Pro
            260                 265                 270

Tyr Ser Lys Glu Leu Gly Asp Thr Ile Lys His Ser Leu Lys Ile Val
        275                 280                 285

Gly Arg Lys Tyr Gln Phe Ser Ile Arg Asp Leu Phe Pro Asp Asp Ala
    290                 295                 300

Gly Leu Tyr Gln Val Asp Val Glu Asp Val Asn Val Phe Ser Thr Asp
305                 310                 315                 320

Phe Lys Ile Pro Met Val Asp Phe Leu Val Lys Ile Gln Glu Cys Lys
                325                 330                 335

Ala Met Glu Arg Glu Asp Ala Val Phe Glu Cys Val Leu Ser Gln Pro
            340                 345                 350

Phe Gly Lys Ile Met Trp Val Gly Lys Asn Leu Pro Leu Glu Ala Gly
        355                 360                 365

Asp Lys Tyr Asp Ile Glu Val Ser Glu Asp Lys Leu Ile His Arg Leu
    370                 375                 380

Ile Ile Lys Asp Val Ala Met Val Asp Lys Gly Ile Tyr Ala Ala Val
385                 390                 395                 400

Ala Gly Ile Lys Ser Cys Asn Ala Phe Leu Val Val Glu Ala Asp Lys
                405                 410                 415

Gly Glu Pro Gly Lys Lys Lys Gln Arg Lys Thr Thr Arg Ala Gly Gly
            420                 425                 430

Ala Gly Val Asp Leu Thr Ala Ile Ala Gln Glu Gln Ala Val Lys Asn
        435                 440                 445

Thr Ala Asp Arg Glu Val Leu Lys Glu Lys Val Lys Ala Ile Asn Asp
    450                 455                 460

Glu Arg Ala Ala Asn Ala Thr Ala Ala Pro Glu Thr Ser Ala Glu Ala
465                 470                 475                 480

Lys Ala Lys Val Lys Val Val Glu Ala Ser Gln Thr Gly Ala Pro Lys
                485                 490                 495

Gln Gly Pro Ala Val Lys Gly Ser Asp His Lys Ser Val Asp Asn Glu
            500                 505                 510

Gly Tyr Ser Ile Lys Ser Gly Leu Ser Asp Val Phe Ala Leu Arg Gly
        515                 520                 525

Lys Lys Gly Glu Leu Val Cys Glu Met Ser His Glu Val Asp Gly Ala
    530                 535                 540

Trp Phe Lys Asp Gly Glu Lys Leu Ser Thr Thr Asp Gly Ile Ala Ile
545                 550                 555                 560

Val Lys Asp Gly Thr Arg His Thr Met Thr Ile His Ser Ser Ser Glu
                565                 570                 575

Asp Asp Thr Gly Val Tyr His Ile Glu Ala Gly Gly Phe Lys Ser Glu
            580                 585                 590

Ala Lys Val Thr Val Gly Glu Leu Pro Ala Ile Asp Ala Asp Asp Leu
        595                 600                 605

His Lys Phe Ser Lys Pro Val Thr Ile Lys Val Gly Gln Asn Ala Ser
    610                 615                 620

Trp Lys Met Pro Tyr Thr Pro Gln Asp Asn Leu Glu Val Lys Trp Phe
625                 630                 635                 640

Lys Asp Gly Lys Glu Leu Lys Asp Gly Gly Gly Val Lys Leu Val Lys
                645                 650                 655

Glu Val Lys His Ser Arg Leu Leu Leu Arg Glu Cys Leu Arg Ser Asp
            660                 665                 670

Ala Gly Glu Ile Lys Ile Gln Leu Lys Asn Pro Phe Gly Thr Ile Glu
        675                 680                 685

Ala Thr Ser Arg Leu Ile Val Leu Asp Lys Pro Gly Pro Pro Glu Gly
    690                 695                 700

Pro Val Glu Ile Leu Glu Thr Thr Ser Thr Val Ile Glu Leu Gln Trp
705                 710                 715                 720

Gly Ala Pro Lys Asp Asp Gly Gly Ser Pro Val Thr Asn Tyr Ile Ile
                725                 730                 735

Glu Arg Gln Gln Leu Gly Gln Thr Val Trp Lys Lys Met Gly Asp Val
            740                 745                 750

Ala Ala Asp Lys Thr Thr Tyr Arg Asp Arg Asn Val Val His Gly Lys

        755                 760                 765

Leu Tyr Ile Tyr Asn Ile Tyr Ala Val Asn Pro Glu Gly Thr Ser Asp
    770                 775                 780

Ala Leu Gln Thr Glu Glu Thr Met Ala Gly Val Leu Ile Phe Ala Gly
785                 790                 795                 800

Arg Pro Gly Ala Pro Lys Val Val Ser Ala Ser Lys Thr Cys Ile Asn
                805                 810                 815

Leu Lys Trp Glu Pro Pro Glu Asp Asp Gly Gly Ile Lys Ile Asp Gly
            820                 825                 830

Tyr Gln Leu Glu Lys Arg Lys Lys Asp Thr Ala Gln Trp Ile Ala Leu
        835                 840                 845

Asn Pro Val Thr Glu Pro Ile Glu Val Leu Glu Tyr Ala Val Lys Asp
    850                 855                 860

Val Val Glu Gly Ala Glu Tyr Glu Phe Arg Val Ser Ala Ile Asn Val
865                 870                 875                 880

Ser Gly Ala Gly Glu Phe Ser Leu Pro Ser Val Met Val Thr Ala Lys
                885                 890                 895

Asn Pro Asn Met Arg Pro Thr Phe Lys Asp Pro Glu Asp Phe Met Val
            900                 905                 910

Ile Arg Ala Gly Asn Ser Val Arg Ile Lys Val Leu Tyr Glu Ala Glu
        915                 920                 925

Pro Pro Pro Glu Ile Thr Trp Met Lys Asp Asn Glu Pro Val Ser Ser
    930                 935                 940

Phe Ile Gln Ile Ile Asn Thr Glu Gly Cys Ser Gln Leu Val Ile Pro
945                 950                 955                 960

Ser Thr Lys Arg Ser Asp Ser Gly Asn Tyr Thr Ile Val Ala Lys Asn
                965                 970                 975

Lys Val Gly Glu Ala Ser Phe Asp Ile Glu Val Leu Val Thr Asp Glu
            980                 985                 990

Pro Lys Pro Pro Gly Ala Val Glu Leu Glu Gln Ile Val His Gly Lys
        995                 1000                1005

Val Ile Val Ser Trp Glu Ala Ser Pro Asp Gln Glu Leu Asp Asn
    1010                1015                1020

Arg Leu Tyr Tyr Met Val Ala Glu His Asp Ser Ser Thr Arg Met
    1025                1030                1035

Trp His Thr Val Ala Asp Arg Ile Phe Asp Asn Ser Tyr Thr Ala
    1040                1045                1050

Asn Asn Ile Met Pro Gly Arg Glu Tyr His Phe Lys Ile Tyr Ala
    1055                1060                1065

Lys Asn Asp Met Gly Met Ser Asp Pro Ser Met Ser Pro Thr Trp
    1070                1075                1080

Gly Ile Asn Ser Asn Arg Ile Pro Ile Asn Thr Asn Thr Pro Val
    1085                1090                1095

Glu Val Ser Phe Glu Lys Pro Pro Ser Val Leu Val Pro Leu Lys
    1100                1105                1110

Met His Ser Pro Pro Lys Gly Phe Gln Met Tyr Met Thr Cys Ala
    1115                1120                1125

Ile Arg Gly Cys Pro Thr Pro Ser Val Thr Trp His Leu Asn Asn
    1130                1135                1140

Val Cys Ile Asn Gly Asp Ser Asn Tyr Tyr Ile Thr Asn Ser Tyr
    1145                1150                1155

Gly Val Cys Ser Met Tyr Ile Leu Arg Val Arg Pro Lys Asp Ala
    1160                1165                1170

Gly Glu Tyr Lys Val Val Ala Val Asn Ser Phe Gly Lys Ala Glu
    1175                1180                1185

Cys Ser Thr Lys Leu Val Val Lys Asp
    1190                1195

<210>  6
<211>  17
<212>  PRT
<213>  Artificial Sequence

<220>
<223>  EEF1A2 binding protein-like fragment

<400>  6

Ala Gln Ala Asp Ser Gln Val Gln Pro Glu Ala Ala Val Ala Asp Phe

1               5                   10                  15

Lys

<210>  7
<211>  10
<212>  PRT
<213>  Artificial Sequence

<220>
<223>  EEF1A2 binding protein-like fragment

<400>  7

Gly Ile Tyr Ala Ala Val Ala Gly Ile Lys
1               5                   10

<210>  8
<211>  21
<212>  PRT
<213>  Artificial Sequence

<220>
<223>  EEF1A2 binding protein-like fragment

<400>  8

Thr Thr Arg Ala Gly Gly Ala Gly Val Asp Leu Thr Ala Ile Ala Gln
1               5                   10                  15

Glu Gln Ala Val Lys
            20

<210>  9
<211>  2532
<212>  DNA
<213>  Artificial Sequence

<220>
<223>  Alpha-1,4 glucan phosphorylase

<400>  9
atgtcaaaac cgttgtcaga tcacgataga aagaagcaga tctccgtgag aggtcttgct      60

ggtgtggaaa atgtggcaga actgaaggtc gctttcaaca ggcatctcca ttttacgctg     120

gtcaaggaca gaaatgtggc atccaaacgg gattactact ttgctctcgc caacaccgtg     180

cgtgaccact tggtgggcag gtggatcaga acccagcaat actactatga gaaagatccc     240

aaacgtgtgt actacatctc cctggagttc tacatgggtc gcaccctgca gaacaccatg     300

gtgaacctgg cgctggaaaa cgcctgtgat gaggccatat accagctggg tctggacatg     360

gaggagctgg aggacatgga ggaggacgca ggcctgggaa acggtggtct tggacgtctt     420

gccgcctgct tcctggactc tatggcttct ctgggtctgg ctgcgtatgg ctacggtatc     480

cgctatgagt ttggcatctt taaccagaag atcgtcaatg gatggcaggt tgaggaggct 540

gatgactggc tgcgttacgg caacccctgg gagaaggccc gacccgagta catgcgcccc 600

gtcaagttct atggcagaac cgagcacacc ccagagggtg tgaaatgggt tgacactcag 660

gtagtgttgg ctctgccata tgacacccct atccccgggt acagaaacaa cattgtcaac 720

accatgagac tgtggtctgc aaaggcccca tgcgacttca acctgaaaga cttcaacgtt 780

ggtgggtaca ttcaggctgt gttggacaga aacctatgcg agaacatttc ccgtgtgctg 840

taccccaatg ataacttctt tgagggcaag gagctgcgtc tgaagcagga gtactttgtg 900

gtggccgcca cccttcagga catcgtccgt cgtttcaagg cctctaagtt tggctccaga 960

gagatcgtcc gcacagactt cgcccagctg cccaacaaag ttgccatcca gctgaatgac 1020

actcaccctg ccatggccat tcctgagctg atgagggtac tggttgatga ggagaagctg 1080

gagtgggaca aggcctggga cgtgtgtgtc cgtacctgtg cctacacaaa ccacaccgtg 1140

ctgcctgagg ccctggagcg ctggcccatt gacctgttcc atcacctgct gccccgtcac 1200

ctggagatta tctacgagat caaccgtcgc ttcctgcagt acgtcgcctc gaagttccct 1260

ggcgacaacg accgtctgcg ccgcatgtcc ctgattgagg agggagaatg caagaaagtc 1320

aacatggctc atatgtgtat cgttggatcc catgctgtca acggcgtggc ccgcatccac 1380

tcggagatcc tcgtcgccac tctgttcaag gacttttatg agttggaccc acacaagttc 1440

cagaacaaga ccaatggcat caccccccgt cgctggctgg ttatgtgcaa ccccggcttg 1500

gctgaggtca tcgcagagag aattggagag gagtttgtcc gtgaccttga ccagctgaag 1560

aaactgttga agttcattga tgatgatgct ttcatccgtg acatagccaa agtcaagcag 1620

gagaacaagc tgaagttcgc tgtgcacctg gaagaacact acaaagtaaa gatcaacccc 1680

cagtccatgt ttgacttcca agtcaaaaga atccacgagt acaagagaca gctgctcaac 1740

tgtctgcaca tgatcaccta ctacaaccgt atcaagaagg agccaaacaa gcactggacc 1800

ccaagaacca tcatggtcgg aggaaaggct gccccaggct accacacagc caagatgatc 1860

attcgtctca tcaccgctat cggtgaggtt gtcaaccacg accccgtgat cggcgaccgc 1920

ctcaaagtca tcttcttgga gaactacaga gtcaccctgg ctgagaaagc catcccctct 1980

gctgacctgt ctgagcagat ctctacagct ggcactgagg cctctggcac tggcaacatg 2040

aagttcatgc tgaatggcgc tctgaccatc ggcaccatgg acggagccaa cgtggagatg 2100

gccgaggagg ccggagagaa gaacctcttc atcttcggca tgagagtgga ggaagtggac 2160

gcaatggacg ccggcaaagg ataccacgcc tctgagtact acaaccgtat tcccgagctg 2220

aaacaggcca tggaccagat ctctggtggc ttcttcagcc ataagcagcc agacctcttc 2280

aaggaacttg tggacctgct gatgcaccac gacaggttca aggtgtttgc tgactacgaa 2340

gcctacatca aaagtcagga taaggtcaac gaactgtaca agaaacccaa ggaatggacc 2400

aagatggtga tccataacat tgcaggctgt ggtaaattct ccagcgaccg caccatttcc 2460

cagtacgccc gggagatctg gggcatggag cccagcctgg agaagatccc tgcccccgat 2520

gagcaactca aa 2532

<210> 10
<211> 844
<212> PRT
<213> Artificial Sequence

<220>
<223> Alpha-1,4 glucan phosphorylase

<400> 10

Met Ser Lys Pro Leu Ser Asp His Asp Arg Lys Lys Gln Ile Ser Val
1               5                   10                  15

Arg Gly Leu Ala Gly Val Glu Asn Val Ala Glu Leu Lys Val Ala Phe
            20                  25                  30

Asn Arg His Leu His Phe Thr Leu Val Lys Asp Arg Asn Val Ala Ser
        35                  40                  45

Lys Arg Asp Tyr Tyr Phe Ala Leu Ala Asn Thr Val Arg Asp His Leu
    50                  55                  60

Val Gly Arg Trp Ile Arg Thr Gln Gln Tyr Tyr Tyr Glu Lys Asp Pro
65                  70                  75                  80

Lys Arg Val Tyr Tyr Ile Ser Leu Glu Phe Tyr Met Gly Arg Thr Leu
                85                  90                  95

Gln Asn Thr Met Val Asn Leu Ala Leu Glu Asn Ala Cys Asp Glu Ala
            100                 105                 110

Ile Tyr Gln Leu Gly Leu Asp Met Glu Glu Leu Glu Asp Met Glu Glu
        115                 120                 125

Asp Ala Gly Leu Gly Asn Gly Gly Leu Gly Arg Leu Ala Ala Cys Phe
    130                 135                 140

Leu Asp Ser Met Ala Ser Leu Gly Leu Ala Ala Tyr Gly Tyr Gly Ile
145                 150                 155                 160

Arg Tyr Glu Phe Gly Ile Phe Asn Gln Lys Ile Val Asn Gly Trp Gln
                165                 170                 175

Val Glu Glu Ala Asp Asp Trp Leu Arg Tyr Gly Asn Pro Trp Glu Lys
            180                 185                 190

Ala Arg Pro Glu Tyr Met Arg Pro Val Lys Phe Tyr Gly Arg Thr Glu
        195                 200                 205

His Thr Pro Glu Gly Val Lys Trp Val Asp Thr Gln Val Val Leu Ala
    210                 215                 220

Leu Pro Tyr Asp Thr Pro Ile Pro Gly Tyr Arg Asn Asn Ile Val Asn
225                 230                 235                 240

Thr Met Arg Leu Trp Ser Ala Lys Ala Pro Cys Asp Phe Asn Leu Lys
                245                 250                 255

Asp Phe Asn Val Gly Gly Tyr Ile Gln Ala Val Leu Asp Arg Asn Leu
            260                 265                 270

Cys Glu Asn Ile Ser Arg Val Leu Tyr Pro Asn Asp Asn Phe Phe Glu
        275                 280                 285

Gly Lys Glu Leu Arg Leu Lys Gln Glu Tyr Phe Val Val Ala Ala Thr
    290                 295                 300

Leu Gln Asp Ile Val Arg Arg Phe Lys Ala Ser Lys Phe Gly Ser Arg
305                 310                 315                 320

Glu Ile Val Arg Thr Asp Phe Ala Gln Leu Pro Asn Lys Val Ala Ile
                325                 330                 335

Gln Leu Asn Asp Thr His Pro Ala Met Ala Ile Pro Glu Leu Met Arg
            340                 345                 350

Val Leu Val Asp Glu Glu Lys Leu Glu Trp Asp Lys Ala Trp Asp Val
        355                 360                 365

Cys Val Arg Thr Cys Ala Tyr Thr Asn His Thr Val Leu Pro Glu Ala
    370                 375                 380

Leu Glu Arg Trp Pro Ile Asp Leu Phe His His Leu Leu Pro Arg His
385                 390                 395                 400

Leu Glu Ile Ile Tyr Glu Ile Asn Arg Arg Phe Leu Gln Tyr Val Ala
                405                 410                 415

Ser Lys Phe Pro Gly Asp Asn Asp Arg Leu Arg Arg Met Ser Leu Ile
            420                 425                 430

Glu Glu Gly Glu Cys Lys Lys Val Asn Met Ala His Met Cys Ile Val
        435                 440                 445

Gly Ser His Ala Val Asn Gly Val Ala Arg Ile His Ser Glu Ile Leu
    450                 455                 460

Val Ala Thr Leu Phe Lys Asp Phe Tyr Glu Leu Asp Pro His Lys Phe
465                 470                 475                 480

Gln Asn Lys Thr Asn Gly Ile Thr Pro Arg Arg Trp Leu Val Met Cys
                485                 490                 495

Asn Pro Gly Leu Ala Glu Val Ile Ala Glu Arg Ile Gly Glu Glu Phe
            500                 505                 510

Val Arg Asp Leu Asp Gln Leu Lys Lys Leu Leu Lys Phe Ile Asp Asp
        515                 520                 525

Asp Ala Phe Ile Arg Asp Ile Ala Lys Val Lys Gln Glu Asn Lys Leu
    530                 535                 540

Lys Phe Ala Val His Leu Glu Glu His Tyr Lys Val Lys Ile Asn Pro
545                 550                 555                 560

Gln Ser Met Phe Asp Phe Gln Val Lys Arg Ile His Glu Tyr Lys Arg
                565                 570                 575

Gln Leu Leu Asn Cys Leu His Met Ile Thr Tyr Tyr Asn Arg Ile Lys
            580                 585                 590

Lys Glu Pro Asn Lys His Trp Thr Pro Arg Thr Ile Met Val Gly Gly
        595                 600                 605

Lys Ala Ala Pro Gly Tyr His Thr Ala Lys Met Ile Ile Arg Leu Ile
    610                 615                 620

Thr Ala Ile Gly Glu Val Val Asn His Asp Pro Val Ile Gly Asp Arg
625                 630                 635                 640

Leu Lys Val Ile Phe Leu Glu Asn Tyr Arg Val Thr Leu Ala Glu Lys
                645                 650                 655

Ala Ile Pro Ser Ala Asp Leu Ser Glu Gln Ile Ser Thr Ala Gly Thr
            660                 665                 670

Glu Ala Ser Gly Thr Gly Asn Met Lys Phe Met Leu Asn Gly Ala Leu
        675                 680                 685

Thr Ile Gly Thr Met Asp Gly Ala Asn Val Glu Met Ala Glu Glu Ala
    690                 695                 700

Gly Glu Lys Asn Leu Phe Ile Phe Gly Met Arg Val Glu Glu Val Asp
705                 710                 715                 720

Ala Met Asp Ala Gly Lys Gly Tyr His Ala Ser Glu Tyr Tyr Asn Arg
                725                 730                 735

Ile Pro Glu Leu Lys Gln Ala Met Asp Gln Ile Ser Gly Gly Phe Phe
            740                 745                 750

Ser His Lys Gln Pro Asp Leu Phe Lys Glu Leu Val Asp Leu Leu Met
        755                 760                 765

His His Asp Arg Phe Lys Val Phe Ala Asp Tyr Glu Ala Tyr Ile Lys
    770                 775                 780

Ser Gln Asp Lys Val Asn Glu Leu Tyr Lys Lys Pro Lys Glu Trp Thr
785                 790                 795                 800

Lys Met Val Ile His Asn Ile Ala Gly Cys Gly Lys Phe Ser Ser Asp
                805                 810                 815

Arg Thr Ile Ser Gln Tyr Ala Arg Glu Ile Trp Gly Met Glu Pro Ser
            820                 825                 830

Leu Glu Lys Ile Pro Ala Pro Asp Glu Gln Leu Lys
        835                 840

<210>  11
<211>  9
<212>  PRT
<213>  Artificial Sequence

<220>
<223>  Alpha-1,4 glucan phosphorylase fragment

<400>  11

Ile Pro Ala Pro Asp Glu Gln Leu Lys
1               5

<210>  12
<211>  17
<212>  PRT
<213>  Artificial Sequence

<220>
<223>  Alpha-1,4 glucan phosphorylase fragment

<400>  12

Gln Ile Ser Val Arg Gly Leu Ala Gly Val Glu Asn Val Ala Glu Leu
1               5                   10                  15

Lys

<210>  13
<211>  25
<212>  PRT
<213>  Artificial Sequence

<220>
<223>  Alpha-1,4 glucan phosphorylase fragment

<400>  13

Ala Ile Pro Ser Ala Asp Leu Ser Glu Gln Ile Ser Thr Ala Gly Thr
1               5                   10                  15

Glu Ala Ser Gly Thr Gly Asn Met Lys
            20                  25

<210>  14
<211>  9
<212>  PRT
<213>  Artificial Sequence

<220>
<223>  Alpha-1,4 glucan phosphorylase fragment

<400>  14

Asp Phe Tyr Glu Leu Asp Pro His Lys
1               5

<210>  15
<211>  10
<212>  PRT
<213>  Artificial Sequence

<220>
<223>  Alpha-1,4 glucan phosphorylase fragment

<400>  15

Phe Ala Val His Leu Glu Glu His Tyr Lys
1               5                   10

<210>  16
<211>  15
<212>  PRT
<213>  Artificial Sequence

<220>

<223>  Alpha-1,4 glucan phosphorylase fragment

<400>  16

Gly Tyr His Ala Ser Glu Tyr Tyr Asn Arg Ile Pro Glu Leu Lys
1               5                   10                  15


<210>  17
<211>  9
<212>  PRT
<213>  Artificial Sequence

<220>
<223>  Alpha-1,4 glucan phosphorylase fragment

<400>  17

Ile Pro Ala Pro Asp Glu Gln Leu Lys
1               5


<210>  18
<211>  2574
<212>  DNA
<213>  Artificial Sequence

<220>
<223>  Elongation factor 2

<400>  18
atggtgaact ttacagtgga ccagatccgt gccatcatgg acaagaaatc caacattcgt      60

aacatgtctg tgatcgctca cgtggaccac ggcaagtcca cgctgaccga ctccctggtg     120

tctaaagccg ggatcatcgc ggggtctcgc gccggagaga cacgcttcac agacactcgc     180

aaagacgagc aggagcgctg tattaccatc aagtccacgg ctatctcgat gtactatgag     240

ctgggggaaa acgacatggc cttcatcaag cagtctaagg atgggcttgg cttcctcatc     300

aacctgattg actcaccggg ccatgtggac ttctcctctg aggtcacagc cgcccttagg     360

gtcaccgacg gcgccctggt ggtggttgac tgcgtctcag gtgtgtgtgt gcagacagag     420

accgtgttga ggcaggccat tgctgagcgc atcaagccag tgctgatgat gaacaagatg     480

gaccgggccc tgctggagct gcagctggag cctgaggacc tgttccagac cttccagcgc     540

atcgtggaga atgtcaacgt catcattgcc acctacggag aggatgaagc gggaccaatg     600

ggtgccatca tgattgaccc tgtgattggt accgtggggt ttgggtctgg cctccacggc     660

tgggccttca ctctaaagca gtttgctgag atgtacgtga caaagttttc tgccggcaaa     720

gacacccagc tgggatcggc ggagaggtgt aagaaggtgg aggacatgat gaagaagctg     780

tggggggaga ggttttttga cccagccact gggaagttca gtaagtccaa cctcggccct     840

gacggtaaga agctgccccg caccttctct cagctggtcc tggaccctat cttcaaggta     900

tttgatgcca tcatgaactt caagaaggat gagacagcca agctgataga gaagctggac     960

atcaagctgg actctgagga caaggagaag gagggcaagc ccctgttgaa ggcagtgatg 1020

cgtcgctggc tcccagccgg agaagccctg ctccagatga tcaccatcca cctgccctcc 1080

cccgtcacgg cccagaagta ccgctgtgag ctgctctacg agggaccagg agacgacgag 1140

gccgccatgg gtatcaagaa ctgcgacccc aaggctcccc tgatgatgta catatctaag 1200

atggtgccca ccacagacaa gggtcgcttc tatgcctttg gccgtgtgtt ctctggctgt 1260

gtgtccaccg gtctgaaggt gcgcatcatg ggaccaaact tcacccctgg gaagaaggaa 1320

gacctctaca tcaagcccat ccagaggacc attctgatga tggggcgtta tgtggagccc 1380

attgaggatg taccatgtgg gaacatcgtt gggctggttg gagttgacca gtatctgatt 1440

aagactggga ccatcaccac ctttgaacag gcccacaaca tgcgtgtgat gaagttcagc 1500

gtcagccctg tggtgagggt ggctgtggag gccaagaacc ctgctgacct gcccaagctg 1560

gtggaggggc tgaagcgtct ggccaagtct gaccccatgg tgcagtgtat catcgaggag 1620

tctggagagc acatcatcgc cggggccgga gagcttcatc tggagatctg tctcaaggat 1680

ctggaggagg accacgccgg cattcccctg aagaaatctg atccagtggt gtcctacagg 1740

gagactgtgt ctgaggagtc tgaagtgatg tgtctatcca agtcccctaa caagcacaac 1800

cgtctgtaca tgcgggctaa acctttccct gacggcctgg ccgaggacat cgagaagggg 1860

gacgtcagcc cccgacagga gctgaaaatc cgcgcccgtt tcctggctga caagtacgag 1920

tgggacgtgt cggaggcccg taagatctgg tgcttcggcc ctgacggtac cggtcccaac 1980

ctgctgatgg atgttaccaa gggagtccag tacctgaatg agatcaagga cagtgttgtg 2040

gctggcttcc agtgggctgt caaggagggt gtgttgtgtg aggagaacat gcgtgcagtc 2100

cgcttcgaca tccacgacgt gaccctgcac acagacgcaa ttcaccgcgg tggcggacag 2160

atcatcccca cggcccgcag agtgctgtat gcctgccagc tcaccgccca gccacgactc 2220

atggagccgg tctacttagt ggagatccag tgcccagagc aggtagtggg tgggatctac 2280

ggcgtgctga acaggaagcg aggccatgtg ttcgaggagt cccaggtgat gggcacgccc 2340

atgttcatcg tcaaggccta cctgcctgtc aacgagtcat ttgggttcac cgctgacctg 2400

cgctccaaca cgggcggcca ggctttcccc cagtgtgtgt ttgatcactg gcagatcctc 2460

cagggagatc cccaggaccc caccaccaag accgccattg tggtggccga gaccaggaaa 2520

cgcaaggggc tgaaagaggg catcccggcc ctggacaact acctggacaa attg 2574

<210> 19
<211> 858
<212> PRT
<213> Artificial Sequence

<220>
<223> Elongation factor 2

<400>  19

Met Val Asn Phe Thr Val Asp Gln Ile Arg Ala Ile Met Asp Lys Lys
1               5                   10                  15

Ser Asn Ile Arg Asn Met Ser Val Ile Ala His Val Asp His Gly Lys
            20                  25                  30

Ser Thr Leu Thr Asp Ser Leu Val Ser Lys Ala Gly Ile Ile Ala Gly
        35                  40                  45

Ser Arg Ala Gly Glu Thr Arg Phe Thr Asp Thr Arg Lys Asp Glu Gln
    50                  55                  60

Glu Arg Cys Ile Thr Ile Lys Ser Thr Ala Ile Ser Met Tyr Tyr Glu
65                  70                  75                  80

Leu Gly Glu Asn Asp Met Ala Phe Ile Lys Gln Ser Lys Asp Gly Leu
                85                  90                  95

Gly Phe Leu Ile Asn Leu Ile Asp Ser Pro Gly His Val Asp Phe Ser
            100                 105                 110

Ser Glu Val Thr Ala Ala Leu Arg Val Thr Asp Gly Ala Leu Val Val
        115                 120                 125

Val Asp Cys Val Ser Gly Val Cys Val Gln Thr Glu Thr Val Leu Arg
    130                 135                 140

Gln Ala Ile Ala Glu Arg Ile Lys Pro Val Leu Met Met Asn Lys Met
145                 150                 155                 160

Asp Arg Ala Leu Leu Glu Leu Gln Leu Glu Pro Glu Asp Leu Phe Gln
                165                 170                 175

Thr Phe Gln Arg Ile Val Glu Asn Val Asn Val Ile Ile Ala Thr Tyr
            180                 185                 190

Gly Glu Asp Glu Ala Gly Pro Met Gly Ala Ile Met Ile Asp Pro Val
        195                 200                 205

Ile Gly Thr Val Gly Phe Gly Ser Gly Leu His Gly Trp Ala Phe Thr
    210                 215                 220

Leu Lys Gln Phe Ala Glu Met Tyr Val Thr Lys Phe Ser Ala Gly Lys
225                 230                 235                 240

Asp Thr Gln Leu Gly Ser Ala Glu Arg Cys Lys Lys Val Glu Asp Met

                245                 250                 255

Met Lys Lys Leu Trp Gly Glu Arg Phe Phe Asp Pro Ala Thr Gly Lys
            260                 265                 270

Phe Ser Lys Ser Asn Leu Gly Pro Asp Gly Lys Lys Leu Pro Arg Thr
        275                 280                 285

Phe Ser Gln Leu Val Leu Asp Pro Ile Phe Lys Val Phe Asp Ala Ile
    290                 295                 300

Met Asn Phe Lys Lys Asp Glu Thr Ala Lys Leu Ile Glu Lys Leu Asp
305                 310                 315                 320

Ile Lys Leu Asp Ser Glu Asp Lys Glu Lys Glu Gly Lys Pro Leu Leu
                325                 330                 335

Lys Ala Val Met Arg Arg Trp Leu Pro Ala Gly Glu Ala Leu Leu Gln
            340                 345                 350

Met Ile Thr Ile His Leu Pro Ser Pro Val Thr Ala Gln Lys Tyr Arg
        355                 360                 365

Cys Glu Leu Leu Tyr Glu Gly Pro Gly Asp Asp Glu Ala Ala Met Gly
    370                 375                 380

Ile Lys Asn Cys Asp Pro Lys Ala Pro Leu Met Met Tyr Ile Ser Lys
385                 390                 395                 400

Met Val Pro Thr Thr Asp Lys Gly Arg Phe Tyr Ala Phe Gly Arg Val
                405                 410                 415

Phe Ser Gly Cys Val Ser Thr Gly Leu Lys Val Arg Ile Met Gly Pro
            420                 425                 430

Asn Phe Thr Pro Gly Lys Lys Glu Asp Leu Tyr Ile Lys Pro Ile Gln
        435                 440                 445

Arg Thr Ile Leu Met Met Gly Arg Tyr Val Glu Pro Ile Glu Asp Val
    450                 455                 460

Pro Cys Gly Asn Ile Val Gly Leu Val Gly Val Asp Gln Tyr Leu Ile
465                 470                 475                 480

Lys Thr Gly Thr Ile Thr Thr Phe Glu Gln Ala His Asn Met Arg Val
                485                 490                 495

Met Lys Phe Ser Val Ser Pro Val Val Arg Val Ala Val Glu Ala Lys
        500                 505                 510

Asn Pro Ala Asp Leu Pro Lys Leu Val Glu Gly Leu Lys Arg Leu Ala
    515                 520                 525

Lys Ser Asp Pro Met Val Gln Cys Ile Ile Glu Glu Ser Gly Glu His
    530                 535                 540

Ile Ile Ala Gly Ala Gly Glu Leu His Leu Glu Ile Cys Leu Lys Asp
545                 550                 555                 560

Leu Glu Glu Asp His Ala Gly Ile Pro Leu Lys Lys Ser Asp Pro Val
                565                 570                 575

Val Ser Tyr Arg Glu Thr Val Ser Glu Glu Ser Glu Val Met Cys Leu
            580                 585                 590

Ser Lys Ser Pro Asn Lys His Asn Arg Leu Tyr Met Arg Ala Lys Pro
        595                 600                 605

Phe Pro Asp Gly Leu Ala Glu Asp Ile Glu Lys Gly Asp Val Ser Pro
    610                 615                 620

Arg Gln Glu Leu Lys Ile Arg Ala Arg Phe Leu Ala Asp Lys Tyr Glu
625                 630                 635                 640

Trp Asp Val Ser Glu Ala Arg Lys Ile Trp Cys Phe Gly Pro Asp Gly
                645                 650                 655

Thr Gly Pro Asn Leu Leu Met Asp Val Thr Lys Gly Val Gln Tyr Leu
            660                 665                 670

Asn Glu Ile Lys Asp Ser Val Val Ala Gly Phe Gln Trp Ala Val Lys
        675                 680                 685

Glu Gly Val Leu Cys Glu Glu Asn Met Arg Ala Val Arg Phe Asp Ile
    690                 695                 700

His Asp Val Thr Leu His Thr Asp Ala Ile His Arg Gly Gly Gly Gln
705                 710                 715                 720

Ile Ile Pro Thr Ala Arg Arg Val Leu Tyr Ala Cys Gln Leu Thr Ala
                725                 730                 735

Gln Pro Arg Leu Met Glu Pro Val Tyr Leu Val Glu Ile Gln Cys Pro
            740                 745                 750

Glu Gln Val Val Gly Gly Ile Tyr Gly Val Leu Asn Arg Lys Arg Gly
        755                 760                 765

His Val Phe Glu Glu Ser Gln Val Met Gly Thr Pro Met Phe Ile Val
    770                 775                 780

Lys Ala Tyr Leu Pro Val Asn Glu Ser Phe Gly Phe Thr Ala Asp Leu
785                 790                 795                 800

Arg Ser Asn Thr Gly Gly Gln Ala Phe Pro Gln Cys Val Phe Asp His
                805                 810                 815

Trp Gln Ile Leu Gln Gly Asp Pro Gln Asp Pro Thr Thr Lys Thr Ala
            820                 825                 830

Ile Val Val Ala Glu Thr Arg Lys Arg Lys Gly Leu Lys Glu Gly Ile
        835                 840                 845

Pro Ala Leu Asp Asn Tyr Leu Asp Lys Leu
    850                 855

<210>  20
<211>  10
<212>  PRT
<213>  Artificial Sequence

<220>
<223>  Elongation factor 2 fragment

<400>  20

Thr Ala Ile Val Val Ala Glu Thr Arg Lys
1               5                   10

<210>  21
<211>  12
<212>  PRT
<213>  Artificial Sequence

<220>
<223>  Elongation factor 2 fragment

<400>  21

Asp Leu Glu Glu Asp His Ala Gly Ile Pro Leu Lys
1               5                   10

<210>  22
<211>  13
<212>  PRT
<213>  Artificial Sequence

<220>

<223>  Elongation factor 2 fragment

<400>  22

Asp Leu Glu Glu Asp His Ala Gly Ile Pro Leu Lys Lys
1               5                   10


<210>  23
<211>  12
<212>  PRT
<213>  Artificial Sequence

<220>
<223>  Elongation factor 2 fragment

<400>  23

Asp Ser Val Val Ala Gly Phe Gln Trp Ala Val Lys
1               5                   10


<210>  24
<211>  10
<212>  PRT
<213>  Artificial Sequence

<220>
<223>  Elongation factor 2 fragment

<400>  24

Gly Asp Val Ser Pro Arg Gln Glu Leu Lys
1               5                   10


<210>  25
<211>  1989
<212>  DNA
<213>  Artificial Sequence

<220>
<223>  Heat shock cognate 70 kDa protein

<400>  25
atgtcaaagg gaccagcagt tggcattgac ctgggcacca cctactcctg tgtgggtgtg      60

tttcagcatg gcaaagtgga gatcatagcc aacgaccagg gaaacaggac cacacccagt     120

tatgtcgcct tcacagacac agaaaggctg attggggacg cagccaagaa ccaagtggcc     180

atgaacccca caaacacagt gtttgatgct aagcggctga tagggcggaa gtttgacgac     240

agtgtcgtcc aggcagacat gaaacactgg ccgtttacag tgatcaacga ctcgacacgg     300

cccaaggtcc aagtggagta caagggagag accaaggcct tctacccaga ggagatctcc     360

tccatggtgc tggtcaagat gaaggagatt gcagaggcct acctgggcaa gactataacc     420

aatgcagtgg tcactgtgcc agcttacttc aacgactctc agcgccaggc caccaaagat     480

gcagggacta tctcaggact caacgtactc cgcatcatca atgagccaac tgctgctgct     540

atcgcctatg gcctggacaa gaaggtggga gtggagagaa acgtcctaat ctttgaccta 600

ggcggaggta cgtttgatgt gtctatcctg accatcgaag acgggatctt tgaggtgaag 660

tccacggccg gagacaccca tcttggagga gaggacttcg acaaccgcat ggtcaaccac 720

ttcatctctg agttcaagcg caaatacaag aaggacatca gcgataacaa gagggccgtg 780

cgtcgtctgc gcaccgcctg tgaacgtgcc aagcgcaccc tgtcctccag tacccaggcc 840

agcattgaga ttgactctct gtatgagggc gtcgacttct acacctccat caccagggct 900

cgctttgagg agctgaacgc tgacctgttc agaggcaccc tggaccccgt ggagaagtct 960

ctgagggacg ccaagatgga caaggcccag gttcacgaca tcgtcctagt gggaggctcc 1020

acacgcatcc ccaagatcca aaagttgctg caggacttct tcaatgggaa ggagctcaac 1080

aagagcatca accctgatga ggcggttgcc tatggtgcag ctgttcaggc ggccatcttg 1140

tccggagaca agtctgagaa cgtgcaggac ctgctgctgc tggacgtcac gccactgtcc 1200

ctgggcattg agacggccgg aggggtcatg accgtgctca tcaagaggaa caccaccatc 1260

cccaccaagc agacgcagac cttcacaaca tactcagaca accagcctgg ggtgctcatt 1320

caggtatatg agggggaaag agccatgacc aaagacaaca acctactggg gaagtttgag 1380

ttgtgtggga ttccaccagc cccccggggt gtgcctcaga tcgaggtcac ctttgacatt 1440

gacgccaacg gcatcatgaa cgtgtctgcc gctgacaaga gcaccggcaa ggagaataag 1500

atcaccatca ccaatgacaa aggtcgtctg agtaaggagg acatagagcg catggtccag 1560

gaggctgaac agtacaaagc tgcggacgat gtccagaggg acaaggtggc gtccaagaat 1620

ggcctggagt catacgcctt caacatgaag tccaccgtgg aggacgagaa gctcaagggc 1680

aagctcagcg acgaggacaa acagaagatc ctggacaaat gcaatgaggt catcagctgg 1740

ctggacaaga accagtctgc agagaaggag gagtttgagc accaccagaa ggagctggag 1800

aaggtctgta accccatcat caccaagctg taccagggtg ctggggggat gcctggaggg 1860

atgcctggag ggatgcctgg agggatgcct ggagggatgc ctggggggat gcctggaggg 1920

atgcccgggg ctggtggtgc cgcacccgga ggaggtggat cctctggacc aacaattgag 1980

gaagtcgac 1989

<210> 26
<211> 663
<212> PRT
<213> Artificial Sequence

<220>
<223> Heat shock cognate 70 kDa protein

<400> 26

Met Ser Lys Gly Pro Ala Val Gly Ile Asp Leu Gly Thr Thr Tyr Ser
1               5                   10                  15

Cys Val Gly Val Phe Gln His Gly Lys Val Glu Ile Ile Ala Asn Asp
        20                  25                  30

Gln Gly Asn Arg Thr Thr Pro Ser Tyr Val Ala Phe Thr Asp Thr Glu
    35                  40                  45

Arg Leu Ile Gly Asp Ala Ala Lys Asn Gln Val Ala Met Asn Pro Thr
    50                  55                  60

Asn Thr Val Phe Asp Ala Lys Arg Leu Ile Gly Arg Lys Phe Asp Asp
65                  70                  75                  80

Ser Val Val Gln Ala Asp Met Lys His Trp Pro Phe Thr Val Ile Asn
                85                  90                  95

Asp Ser Thr Arg Pro Lys Val Gln Val Glu Tyr Lys Gly Glu Thr Lys
            100                 105                 110

Ala Phe Tyr Pro Glu Glu Ile Ser Ser Met Val Leu Val Lys Met Lys
        115                 120                 125

Glu Ile Ala Glu Ala Tyr Leu Gly Lys Thr Ile Thr Asn Ala Val Val
    130                 135                 140

Thr Val Pro Ala Tyr Phe Asn Asp Ser Gln Arg Gln Ala Thr Lys Asp
145                 150                 155                 160

Ala Gly Thr Ile Ser Gly Leu Asn Val Leu Arg Ile Ile Asn Glu Pro
                165                 170                 175

Thr Ala Ala Ala Ile Ala Tyr Gly Leu Asp Lys Lys Val Gly Val Glu
            180                 185                 190

Arg Asn Val Leu Ile Phe Asp Leu Gly Gly Gly Thr Phe Asp Val Ser
        195                 200                 205

Ile Leu Thr Ile Glu Asp Gly Ile Phe Glu Val Lys Ser Thr Ala Gly
    210                 215                 220

Asp Thr His Leu Gly Gly Glu Asp Phe Asp Asn Arg Met Val Asn His
225                 230                 235                 240

Phe Ile Ser Glu Phe Lys Arg Lys Tyr Lys Lys Asp Ile Ser Asp Asn
                245                 250                 255

Lys Arg Ala Val Arg Arg Leu Arg Thr Ala Cys Glu Arg Ala Lys Arg

            260                 265                 270

Thr Leu Ser Ser Ser Thr Gln Ala Ser Ile Glu Ile Asp Ser Leu Tyr
        275                 280                 285

Glu Gly Val Asp Phe Tyr Thr Ser Ile Thr Arg Ala Arg Phe Glu Glu
    290                 295                 300

Leu Asn Ala Asp Leu Phe Arg Gly Thr Leu Asp Pro Val Glu Lys Ser
305                 310                 315                 320

Leu Arg Asp Ala Lys Met Asp Lys Ala Gln Val His Asp Ile Val Leu
                325                 330                 335

Val Gly Gly Ser Thr Arg Ile Pro Lys Ile Gln Lys Leu Leu Gln Asp
            340                 345                 350

Phe Phe Asn Gly Lys Glu Leu Asn Lys Ser Ile Asn Pro Asp Glu Ala
        355                 360                 365

Val Ala Tyr Gly Ala Ala Val Gln Ala Ala Ile Leu Ser Gly Asp Lys
    370                 375                 380

Ser Glu Asn Val Gln Asp Leu Leu Leu Leu Asp Val Thr Pro Leu Ser
385                 390                 395                 400

Leu Gly Ile Glu Thr Ala Gly Gly Val Met Thr Val Leu Ile Lys Arg
                405                 410                 415

Asn Thr Thr Ile Pro Thr Lys Gln Thr Gln Thr Phe Thr Thr Tyr Ser
            420                 425                 430

Asp Asn Gln Pro Gly Val Leu Ile Gln Val Tyr Glu Gly Glu Arg Ala
        435                 440                 445

Met Thr Lys Asp Asn Asn Leu Leu Gly Lys Phe Glu Leu Cys Gly Ile
    450                 455                 460

Pro Pro Ala Pro Arg Gly Val Pro Gln Ile Glu Val Thr Phe Asp Ile
465                 470                 475                 480

Asp Ala Asn Gly Ile Met Asn Val Ser Ala Ala Asp Lys Ser Thr Gly
                485                 490                 495

Lys Glu Asn Lys Ile Thr Ile Thr Asn Asp Lys Gly Arg Leu Ser Lys
            500                 505                 510

Glu Asp Ile Glu Arg Met Val Gln Glu Ala Glu Gln Tyr Lys Ala Ala
        515                 520                 525

Asp Asp Val Gln Arg Asp Lys Val Ala Ser Lys Asn Gly Leu Glu Ser
    530                 535                 540

Tyr Ala Phe Asn Met Lys Ser Thr Val Glu Asp Glu Lys Leu Lys Gly
545                 550                 555                 560

Lys Leu Ser Asp Glu Asp Lys Gln Lys Ile Leu Asp Lys Cys Asn Glu
                565                 570                 575

Val Ile Ser Trp Leu Asp Lys Asn Gln Ser Ala Glu Lys Glu Glu Phe
            580                 585                 590

Glu His His Gln Lys Glu Leu Glu Lys Val Cys Asn Pro Ile Ile Thr
        595                 600                 605

Lys Leu Tyr Gln Gly Ala Gly Gly Met Pro Gly Gly Met Pro Gly Gly
    610                 615                 620

Met Pro Gly Gly Met Pro Gly Gly Met Pro Gly Gly Met Pro Gly Gly
625                 630                 635                 640

Met Pro Gly Ala Gly Gly Ala Ala Pro Gly Gly Gly Gly Ser Ser Gly
                645                 650                 655

Pro Thr Ile Glu Glu Val Asp
            660


<210>  27
<211>  14
<212>  PRT
<213>  Artificial Sequence

<220>
<223>  Heat shock cognate 70 kDa protein fragment

<400>  27

Ala Phe Tyr Pro Glu Glu Ile Ser Ser Met Val Leu Val Lys
1               5                   10


<210>  28
<211>  14
<212>  PRT
<213>  Artificial Sequence

<220>
<223>  Heat shock cognate 70 kDa protein fragment

<400>  28

Glu Asp Ile Glu Arg Met Val Gln Glu Ala Glu Gln Tyr Lys
1               5                   10

<210>  29
<211>  14
<212>  PRT
<213>  Artificial Sequence

<220>
<223>  Heat shock cognate 70 kDa protein fragment

<400>  29

His Trp Pro Phe Thr Val Ile Asn Asp Ser Thr Arg Pro Lys
1               5                   10

<210>  30
<211>  14
<212>  PRT
<213>  Artificial Sequence

<220>
<223>  Heat shock cognate 70 kDa protein fragment

<400>  30

Asn Gln Ser Ala Glu Lys Glu Glu Phe Glu His His Gln Lys
1               5                   10

<210>  31
<211>  22
<212>  PRT
<213>  Artificial Sequence

<220>
<223>  Heat shock cognate 70 kDa protein fragment

<400>  31

Thr Ile Thr Asn Ala Val Val Thr Val Pro Ala Tyr Phe Asn Asp Ser
1               5                   10                  15

Gln Arg Gln Ala Thr Lys
            20

<210>  32
<211>  2061
<212>  DNA
<213>  Artificial Sequence

<220>
<223>  Serotransferrin

<400>  32
atgaaactgc ttctcgtctc agcgctgctg gggtgcttcg ctacggtgta tgctgcccca        60

gctgagggaa tggttagatg gtgcgtaaag tcggaaaaag agctgaagaa atgccacgat       120

ctcgcagcca atgtggcggg gttttcatgc gtgaggaggg acgactctct tgaatgcatc 180

caagccatca agagggaaga ggctgatgct atcactctgg atggaggaga tatttacata 240

gctggcctcc acaactacaa cctgcagccc atcattgcag aggactatgg tgaggactct 300

gacacctgct attatgctgt ggccgtggcc aaaaagggca ctgaatttgg gttcctcgac 360

ctccgtggca agaagtcctg ccacaccggg ttgggcaaat ctgcaggctg gaacattccc 420

atcggtaccc tggtgactgt gggccagatc caatgggccg gcatcgagga cagacctgtg 480

gagtcggcgg tgagcgactt cttcaatgcc agctgtgctc caggagccaa tagggattct 540

caactgtgcc agttgtgtat gggagactgc tccagatctc acaacgagcc ctactatgac 600

tattccgggg ccttccagtg cctgaaggat ggagcaggag aggttgcctt catcaagcac 660

ctgactgtac ctgccgcaga gaaggcaagc tatgagttgc tgtgcaagga taacaccaga 720

gctcccatcg acagctacaa gacctgccac ctggccagag tacccgccca cgctgtggtc 780

agccgcaagg accccaggct ggccaatctc atctacagca agctgatggc cgtcacgaac 840

ttcaacctgt tctcctccga tggttatgct gccaagaacc tgatgttcaa ggactccact 900

cagaatctag tacagctgcc aatgaccacc gactccttcc tctacctggg agctgagtac 960

atgagcacta tacgctccct gacaaaagcg caggccacag gtgtcacctc cagggccatc 1020

aaatggtgtg ccgtgggcca taaggagaag gtcaagtgtg acgcctggac aatcaacagc 1080

ttcacagatg gtgactccag gatcgaatgc caggacgcac ccacagtgga tgaatgcatc 1140

aagaagatca tgcgtaaaga ggcagatgcc atagcggtgg atggtgggga ggtgttcact 1200

gctggaaaat gtggtctggt ccctgtcatg gtggagcagt atgatgaagt tcggtgcagc 1260

gcccctggtg aggcgtcatc ctactttgcg gtggcggtgg caaagagggg atctgggttg 1320

acctggacaa ccctgaaggg caagaggtca tgccacaccg gcttgggcag gaccgcaggc 1380

tggaacatac ccatgggtct tatccatagg aggactatga actgcgactt caccacatac 1440

ttcagtaagg gctgtgctcc tggatttgag gtggactctc ccttctgtgc ccagtgtaag 1500

ggcagtggga agtccgtggg aggagatggg tccaagtgca aagccagctc tgaagagcag 1560

tactacggct ataacggagc attcagatgt ctggttgaag atgctggaga tgttgccttc 1620

attaaacaca ctattgtacc agagatgact gatggtagtg gtccagtttg ggcacagaac 1680

ctgatgtcct ctgactttga actactctgc caggatggta ctaccaagcc agtcacacat 1740

ttccgtgagt gccacctggc caaggtgccc gcccatgctg tgataacacg cccagagtcc 1800

cgtggagagg ttgtgtccat ccttctggag cagcaggcca ggttcggctc aagcggcagc 1860

gattcctcat ttaatatgtt caagccagat tttggaaaga acttgctctt caaggattcc 1920

acaaagtgtc tccaggagat cccaagcggc accaaattcc agggtttcct tggggaagag 1980

tacatgatcg ccatgcaatc gctcagggag tgctccaaca gtacctcaga tttggagaag 2040

gcatgcactt tccattcctg c 2061

<210> 33
<211> 687
<212> PRT
<213> Artificial Sequence

<220>
<223> Serotransferrin

<400> 33

Met Lys Leu Leu Leu Val Ser Ala Leu Leu Gly Cys Phe Ala Thr Val
1               5                   10                  15

Tyr Ala Ala Pro Ala Glu Gly Met Val Arg Trp Cys Val Lys Ser Glu
            20                  25                  30

Lys Glu Leu Lys Lys Cys His Asp Leu Ala Ala Asn Val Ala Gly Phe
        35                  40                  45

Ser Cys Val Arg Arg Asp Asp Ser Leu Glu Cys Ile Gln Ala Ile Lys
    50                  55                  60

Arg Glu Glu Ala Asp Ala Ile Thr Leu Asp Gly Gly Asp Ile Tyr Ile
65                  70                  75                  80

Ala Gly Leu His Asn Tyr Asn Leu Gln Pro Ile Ile Ala Glu Asp Tyr
                85                  90                  95

Gly Glu Asp Ser Asp Thr Cys Tyr Tyr Ala Val Ala Val Ala Lys Lys
            100                 105                 110

Gly Thr Glu Phe Gly Phe Leu Asp Leu Arg Gly Lys Lys Ser Cys His
        115                 120                 125

Thr Gly Leu Gly Lys Ser Ala Gly Trp Asn Ile Pro Ile Gly Thr Leu
    130                 135                 140

Val Thr Val Gly Gln Ile Gln Trp Ala Gly Ile Glu Asp Arg Pro Val
145                 150                 155                 160

Glu Ser Ala Val Ser Asp Phe Phe Asn Ala Ser Cys Ala Pro Gly Ala
                165                 170                 175

Asn Arg Asp Ser Gln Leu Cys Gln Leu Cys Met Gly Asp Cys Ser Arg
            180                 185                 190

Ser His Asn Glu Pro Tyr Tyr Asp Tyr Ser Gly Ala Phe Gln Cys Leu
        195                 200                 205

Lys Asp Gly Ala Gly Glu Val Ala Phe Ile Lys His Leu Thr Val Pro
    210                 215                 220

Ala Ala Glu Lys Ala Ser Tyr Glu Leu Leu Cys Lys Asp Asn Thr Arg
225                 230                 235                 240

Ala Pro Ile Asp Ser Tyr Lys Thr Cys His Leu Ala Arg Val Pro Ala
                245                 250                 255

His Ala Val Val Ser Arg Lys Asp Pro Arg Leu Ala Asn Leu Ile Tyr
            260                 265                 270

Ser Lys Leu Met Ala Val Thr Asn Phe Asn Leu Phe Ser Ser Asp Gly
        275                 280                 285

Tyr Ala Ala Lys Asn Leu Met Phe Lys Asp Ser Thr Gln Asn Leu Val
    290                 295                 300

Gln Leu Pro Met Thr Thr Asp Ser Phe Leu Tyr Leu Gly Ala Glu Tyr
305                 310                 315                 320

Met Ser Thr Ile Arg Ser Leu Thr Lys Ala Gln Ala Thr Gly Val Thr
                325                 330                 335

Ser Arg Ala Ile Lys Trp Cys Ala Val Gly His Lys Glu Lys Val Lys
            340                 345                 350

Cys Asp Ala Trp Thr Ile Asn Ser Phe Thr Asp Gly Asp Ser Arg Ile
        355                 360                 365

Glu Cys Gln Asp Ala Pro Thr Val Asp Glu Cys Ile Lys Lys Ile Met
    370                 375                 380

Arg Lys Glu Ala Asp Ala Ile Ala Val Asp Gly Gly Glu Val Phe Thr
385                 390                 395                 400

Ala Gly Lys Cys Gly Leu Val Pro Val Met Val Glu Gln Tyr Asp Glu
                405                 410                 415

Val Arg Cys Ser Ala Pro Gly Glu Ala Ser Ser Tyr Phe Ala Val Ala
            420                 425                 430

Val Ala Lys Arg Gly Ser Gly Leu Thr Trp Thr Thr Leu Lys Gly Lys
        435                 440                 445

Arg Ser Cys His Thr Gly Leu Gly Arg Thr Ala Gly Trp Asn Ile Pro
    450                 455                 460

Met Gly Leu Ile His Arg Arg Thr Met Asn Cys Asp Phe Thr Thr Tyr
465                 470                 475                 480

Phe Ser Lys Gly Cys Ala Pro Gly Phe Glu Val Asp Ser Pro Phe Cys
                485                 490                 495

Ala Gln Cys Lys Gly Ser Gly Lys Ser Val Gly Gly Asp Gly Ser Lys
            500                 505                 510

Cys Lys Ala Ser Ser Glu Glu Gln Tyr Tyr Gly Tyr Asn Gly Ala Phe
        515                 520                 525

Arg Cys Leu Val Glu Asp Ala Gly Asp Val Ala Phe Ile Lys His Thr
    530                 535                 540

Ile Val Pro Glu Met Thr Asp Gly Ser Gly Pro Val Trp Ala Gln Asn
545                 550                 555                 560

Leu Met Ser Ser Asp Phe Glu Leu Leu Cys Gln Asp Gly Thr Thr Lys
                565                 570                 575

Pro Val Thr His Phe Arg Glu Cys His Leu Ala Lys Val Pro Ala His
            580                 585                 590

Ala Val Ile Thr Arg Pro Glu Ser Arg Gly Glu Val Val Ser Ile Leu
        595                 600                 605

Leu Glu Gln Gln Ala Arg Phe Gly Ser Ser Gly Ser Asp Ser Ser Phe
    610                 615                 620

Asn Met Phe Lys Pro Asp Phe Gly Lys Asn Leu Leu Phe Lys Asp Ser
625                 630                 635                 640

Thr Lys Cys Leu Gln Glu Ile Pro Ser Gly Thr Lys Phe Gln Gly Phe
                645                 650                 655

Leu Gly Glu Glu Tyr Met Ile Ala Met Gln Ser Leu Arg Glu Cys Ser
            660                 665                 670

Asn Ser Thr Ser Asp Leu Glu Lys Ala Cys Thr Phe His Ser Cys
        675                 680                 685

<210>  34
<211>  12

<212>  PRT
<213>  Artificial Sequence

<220>
<223>  Serotransferrin fragment

<400>  34

Ala Gln Ala Thr Gly Val Thr Ser Arg Ala Ile Lys
1               5                   10


<210>  35
<211>  10
<212>  PRT
<213>  Artificial Sequence

<220>
<223>  Serotransferrin fragment

<400>  35

Ala Thr Gly Val Thr Ser Arg Ala Ile Lys
1               5                   10


<210>  36
<211>  11
<212>  PRT
<213>  Artificial Sequence

<220>
<223>  Serotransferrin fragment

<400>  36

Asp Pro Arg Leu Ala Asn Leu Ile Tyr Ser Lys
1               5                   10


<210>  37
<211>  12
<212>  PRT
<213>  Artificial Sequence

<220>
<223>  Serotransferrin fragment

<400>  37

Gly Thr Glu Phe Gly Phe Leu Asp Leu Arg Gly Lys
1               5                   10


<210>  38
<211>  13
<212>  PRT
<213>  Artificial Sequence

<220>
<223>  Serotransferrin fragment

<400>  38

Lys Gly Thr Glu Phe Gly Phe Leu Asp Leu Arg Gly Lys
1               5                   10

<210>  39
<211>  14
<212>  PRT
<213>  Artificial Sequence

<220>
<223>  Serotransferrin fragment

<400>  39

Lys Gly Thr Glu Phe Gly Phe Leu Asp Leu Arg Gly Lys Lys
1               5                   10

<210>  40
<211>  18
<212>  PRT
<213>  Artificial Sequence

<220>
<223>  Serotransferrin fragment

<400>  40

Leu Met Ala Val Thr Asn Phe Asn Leu Phe Ser Ser Asp Gly Tyr Ala
1               5                   10                  15

Ala Lys

<210>  41
<211>  11
<212>  PRT
<213>  Artificial Sequence

<220>
<223>  Serotransferrin fragment

<400>  41

Arg Gly Ser Gly Leu Thr Trp Thr Thr Leu Lys
1               5                   10

<210>  42
<211>  1518
<212>  DNA
<213>  Artificial Sequence

<220>
<223>  Myosin binding protein H-like

<400>  42
atgcctgcga agcctgcccc tatcaagaag gcagccccga aagagcctgc tcctaagaag        60

gaggaagcac ctgcacctgc acccgcggag gcggctcctg ctgaagttgc tccccctgcc       120

gaggtcgaag ctccaccggc agaggctgcc cctgccgaag cagctgcccc tgctgaggca 180

gctccaaccg aagaaaaggc tcccgcagaa cccgcgcctg agcagcccaa agcacccaca 240

ccccctcctc cagatgagcc caccagtgag cccctggaag tgacagtgga cgacaagagc 300

gacacttcag tgaccatcac ctggaggcct ccaaagacta ttccagccaa ctctggcctg 360

gatggataca ctgtggaaat caccaaggag ggaactgaag actggaaagc agccaacgag 420

gatctgaccg tgtcctgccg ttatgtcatc aagaaccaga ctaccggcga ccgtctgctg 480

atccgagtgg ttgctgtaaa ccctggtggc cgcagccccc ctgctaccat cgccgacccc 540

gttctggtca aggaggttgg ggctcgcccc aaagtccgcc tccctcgttt cctgaggcag 600

aggtatgtca agaaagtcgg cgagaagatc aacattgtca tcccgtactc tggcaaacct 660

aagccagtgg ttagctggtt aaaggatgga cagcccctcg actcaaagag ggccaacatc 720

cgtacatctg acagagacag catcctgttc atccgtcaag cagagagggt ggactctggc 780

tcatacgaaa tgtgcgtgaa ggtggacgac tttgaggaca aagctgctat catcatccag 840

attattgagt tacccgggcc accagccagc attaagattg tggatgtctg gggattcaac 900

gttgctttgg agtggaccgt acccaaagat aatggaaaca cagagatcac aggctacact 960

gtccagaaag ctgacaagaa gaccggggac tggttcaaca ttttggagca ctacgccaga 1020

ctaaatgcca ccatctcaga cctcattatg ggcaacacct acaccttcag ggtctttgca 1080

gagaacaagt gcggactcag tgaggaatgt gccgtgacca agggagaagc caccattgtg 1140

aaggaggtta ttgactacaa acctaccccg ttcgtggagc atgacttcac cgaggctccc 1200

aagttcacca ctgttctgaa cgacaggtcc accactgttg gctacagcac caagctgctg 1260

tgttctgtga ggggatgccc caagcctaag attatgtgga tgaagaacaa gatgattctc 1320

aacaacatgg acaaccccaa gtacaggatg atcagcacag ggggcatctg caccctggag 1380

atccgtaagc ctggcccata cgacggtggt gagtatgtct gcagagccga gaatacactg 1440

gggaaggtcg acaccggctg caagctggag gtcagaaagc ctgggcaagc agatgctgac 1500

aaagacaaga aggaacag 1518

<210> 43
<211> 506
<212> PRT
<213> Artificial Sequence

<220>
<223> Myosin binding protein H-like

<400> 43

Met Pro Ala Lys Pro Ala Pro Ile Lys Lys Ala Ala Pro Lys Glu Pro
1               5                   10                  15

Ala Pro Lys Lys Glu Glu Ala Pro Ala Pro Ala Pro Ala Glu Ala Ala
            20                  25                  30
Pro Ala Glu Val Ala Pro Pro Ala Glu Val Glu Ala Pro Pro Ala Glu
        35                  40                  45
Ala Ala Pro Ala Glu Ala Ala Ala Pro Ala Glu Ala Ala Pro Thr Glu
    50                  55                  60
Glu Lys Ala Pro Ala Glu Pro Ala Pro Glu Gln Pro Lys Ala Pro Thr
65                  70                  75                  80
Pro Pro Pro Pro Asp Glu Pro Thr Ser Glu Pro Leu Glu Val Thr Val
                85                  90                  95
Asp Asp Lys Ser Asp Thr Ser Val Thr Ile Thr Trp Arg Pro Pro Lys
            100                 105                 110
Thr Ile Pro Ala Asn Ser Gly Leu Asp Gly Tyr Thr Val Glu Ile Thr
        115                 120                 125
Lys Glu Gly Thr Glu Asp Trp Lys Ala Ala Asn Glu Asp Leu Thr Val
    130                 135                 140
Ser Cys Arg Tyr Val Ile Lys Asn Gln Thr Thr Gly Asp Arg Leu Leu
145                 150                 155                 160
Ile Arg Val Val Ala Val Asn Pro Gly Gly Arg Ser Pro Pro Ala Thr
                165                 170                 175
Ile Ala Asp Pro Val Leu Val Lys Glu Val Gly Ala Arg Pro Lys Val
            180                 185                 190
Arg Leu Pro Arg Phe Leu Arg Gln Arg Tyr Val Lys Lys Val Gly Glu
        195                 200                 205
Lys Ile Asn Ile Val Ile Pro Tyr Ser Gly Lys Pro Lys Pro Val Val
    210                 215                 220
Ser Trp Leu Lys Asp Gly Gln Pro Leu Asp Ser Lys Arg Ala Asn Ile
225                 230                 235                 240
Arg Thr Ser Asp Arg Asp Ser Ile Leu Phe Ile Arg Gln Ala Glu Arg
                245                 250                 255
Val Asp Ser Gly Ser Tyr Glu Met Cys Val Lys Val Asp Asp Phe Glu
            260                 265                 270

Asp Lys Ala Ala Ile Ile Ile Gln Ile Ile Glu Leu Pro Gly Pro Pro
        275                 280                 285

Ala Ser Ile Lys Ile Val Asp Val Trp Gly Phe Asn Val Ala Leu Glu
    290                 295                 300

Trp Thr Val Pro Lys Asp Asn Gly Asn Thr Glu Ile Thr Gly Tyr Thr
305                 310                 315                 320

Val Gln Lys Ala Asp Lys Lys Thr Gly Asp Trp Phe Asn Ile Leu Glu
                325                 330                 335

His Tyr Ala Arg Leu Asn Ala Thr Ile Ser Asp Leu Ile Met Gly Asn
            340                 345                 350

Thr Tyr Thr Phe Arg Val Phe Ala Glu Asn Lys Cys Gly Leu Ser Glu
        355                 360                 365

Glu Cys Ala Val Thr Lys Gly Glu Ala Thr Ile Val Lys Glu Val Ile
    370                 375                 380

Asp Tyr Lys Pro Thr Pro Phe Val Glu His Asp Phe Thr Glu Ala Pro
385                 390                 395                 400

Lys Phe Thr Thr Val Leu Asn Asp Arg Ser Thr Thr Val Gly Tyr Ser
                405                 410                 415

Thr Lys Leu Leu Cys Ser Val Arg Gly Cys Pro Lys Pro Lys Ile Met
            420                 425                 430

Trp Met Lys Asn Lys Met Ile Leu Asn Asn Met Asp Asn Pro Lys Tyr
        435                 440                 445

Arg Met Ile Ser Thr Gly Gly Ile Cys Thr Leu Glu Ile Arg Lys Pro
    450                 455                 460

Gly Pro Tyr Asp Gly Gly Glu Tyr Val Cys Arg Ala Glu Asn Thr Leu
465                 470                 475                 480

Gly Lys Val Asp Thr Gly Cys Lys Leu Glu Val Arg Lys Pro Gly Gln
                485                 490                 495

Ala Asp Ala Asp Lys Asp Lys Lys Glu Gln
            500                 505

<210>  44

<211>  18
<212>  PRT
<213>  Artificial Sequence

<220>
<223>  Myosin binding protein H-like fragment

<400>  44

Ala Ala Ile Ile Ile Gln Ile Ile Glu Leu Pro Gly Pro Pro Ala Ser
1               5                   10                  15

Ile Lys

<210>  45
<211>  18
<212>  PRT
<213>  Artificial Sequence

<220>
<223>  Myosin binding protein H-like fragment

<400>  45

Ala Ala Pro Ala Glu Ala Ala Ala Pro Ala Glu Ala Ala Pro Thr Glu
1               5                   10                  15

Glu Lys

<210>  46
<211>  11
<212>  PRT
<213>  Artificial Sequence

<220>
<223>  Myosin binding protein H-like fragment

<400>  46

Ala Pro Ala Glu Pro Ala Pro Glu Gln Pro Lys
1               5                   10

<210>  47
<211>  14
<212>  PRT
<213>  Artificial Sequence

<220>
<223>  Myosin binding protein H-like fragment

<400>  47

Asp Asn Gly Asn Thr Glu Ile Thr Gly Tyr Thr Val Gln Lys
1               5                   10

<210>  48
<211>  20
<212>  PRT
<213>  Artificial Sequence

<220>
<223>  Myosin binding protein H-like fragment

<400>  48

Glu Val Ile Asp Tyr Lys Pro Thr Pro Phe Val Glu His Asp Phe Thr
1               5                   10                  15

Glu Ala Pro Lys
            20

<210>  49
<211>  17
<212>  PRT
<213>  Artificial Sequence

<220>
<223>  Myosin binding protein H-like fragment

<400>  49

Phe Thr Thr Val Leu Asn Asp Arg Ser Thr Thr Val Gly Tyr Ser Thr
1               5                   10                  15

Lys

<210>  50
<211>  10
<212>  PRT
<213>  Artificial Sequence

<220>
<223>  Myosin binding protein H-like fragment

<400>  50

Ile Asn Ile Val Ile Pro Tyr Ser Gly Lys
1               5                   10

<210>  51
<211>  19
<212>  PRT
<213>  Artificial Sequence

<220>
<223>  Myosin binding protein H-like fragment

<400>  51

Ile Asn Ile Val Ile Pro Tyr Ser Gly Lys Pro Lys Pro Val Val Ser
1               5                   10                  15

Trp Leu Lys

<210> 52
<211> 33
<212> PRT
<213> Artificial Sequence

<220>
<223> Myosin binding protein H-like fragment

<400> 52

Asn Gln Thr Thr Gly Asp Arg Leu Leu Ile Arg Val Val Ala Val Asn
1               5                   10                  15

Pro Gly Gly Arg Ser Pro Pro Ala Thr Ile Ala Asp Pro Val Leu Val
            20                  25                  30

Lys

<210> 53
<211> 13
<212> PRT
<213> Artificial Sequence

<220>
<223> Myosin binding protein H-like fragment

<400> 53

Ser Asp Thr Ser Val Thr Ile Thr Trp Arg Pro Pro Lys
1               5                   10

<210> 54
<211> 17
<212> PRT
<213> Artificial Sequence

<220>
<223> Myosin binding protein H-like fragment

<400> 54

Thr Ile Pro Ala Asn Ser Gly Leu Asp Gly Tyr Thr Val Glu Ile Thr
1               5                   10                  15

Lys

<210> 55
<211> 1377
<212> DNA
<213> Artificial Sequence

<220>
<223>  Desmin (fragment)

<400>  55
atttcctctt accgccgcac tttcggttct ggtattggat ccaccccagg catgtcctcc      60

atgttctccc acggtggacg cggctcctcc ggctcggccc acatgtcctc cagagtctac     120

gagatgacca aaagctccgc ccgccccagc tactcctccg gcagtattcg ctcctcctcc     180

ggcggcgcga tgcgctcgta cgccgggatg ggcgagaagc tggacttcaa cctggccgac     240

gccactaacc gcgacttcct ggacaccaga accaatgaga aggcagagtt gcagcacctg     300

aacgaccggt tcgccagcta catcgagaag gtccgtttcc tggagcagca gaacgctact     360

ctggtggtgg agatcgagag gctgaggggt cacgagccga cccgcgtggc cgagatgtac     420

gaggaggaga tgagagagct gaggcgtcag gtggacggca tgtccaatga ccgagcccgc     480

atggaggtgg agagagacaa cttggctgac gacctgcaga aactcaaact cagactgcag     540

gaggtgatcc accagaggga agaggcagag aacaacctgt ctgccttcag agctgacgtg     600

gactctgcca cgctggccag gctggacctg gagagacgca ttgaaagcct gcaggaggag     660

atcaccttcc tcaagaagat ccacgaggag gagatccatg agctgacgag ccagatgcag     720

gagacctcgg tgcaggtcca gatggacatg tccaaaccag acctgaccgt tgccctcagg     780

gacatccgta tgcagtacga gggtatcgca gccaagaaca tctctgaggc agaggactgg     840

tacaagtcca aggtgtcaga cctgaaccag gctgtgaaca agaacaacga tgctctgaga     900

caggccaaac aggagagcat ggagttcagg catcagatcc agtcctacac ctgtgagatc     960

gactctctca agggaaccaa cgagtctctg ctgaggcaga tgagggagat ggaggatcgt    1020

ctgggaaacg aggctggagg ttaccaggac tccgtcaccc gtctggaggc tgagatcgcc    1080

aagatgaagg atgagatggc tcgtcacctc agagagtacc aggacctcct caatgtcaag    1140

atggccctgg atatagagat cgctacctac aggaagctac tggagggaga ggagagccga    1200

atcactgtat ctggctccaa gtcttctcac tctggctccc actctgctgc ctcattatat    1260

tccactgttg gattcagaga gaccagccca gacgtcggac gatcagcaga ggtccactcc    1320

aagaagactg tcctgatcaa gaccatcgag acccgcgatg gagaggtcgt cagcgag       1377

<210>  56
<211>  459
<212>  PRT
<213>  Artificial Sequence

<220>
<223>  Desmin (fragment)

<400>  56

Ile Ser Ser Tyr Arg Arg Thr Phe Gly Ser Gly Ile Gly Ser Thr Pro

1               5                   10                  15

Gly Met Ser Ser Met Phe Ser His Gly Gly Arg Gly Ser Ser Gly Ser
            20                  25                  30

Ala His Met Ser Ser Arg Val Tyr Glu Met Thr Lys Ser Ser Ala Arg
        35                  40                  45

Pro Ser Tyr Ser Ser Gly Ser Ile Arg Ser Ser Ser Gly Gly Ala Met
    50                  55                  60

Arg Ser Tyr Ala Gly Met Gly Glu Lys Leu Asp Phe Asn Leu Ala Asp
65                  70                  75                  80

Ala Thr Asn Arg Asp Phe Leu Asp Thr Arg Thr Asn Glu Lys Ala Glu
                85                  90                  95

Leu Gln His Leu Asn Asp Arg Phe Ala Ser Tyr Ile Glu Lys Val Arg
            100                 105                 110

Phe Leu Glu Gln Gln Asn Ala Thr Leu Val Val Glu Ile Glu Arg Leu
        115                 120                 125

Arg Gly His Glu Pro Thr Arg Val Ala Glu Met Tyr Glu Glu Glu Met
    130                 135                 140

Arg Glu Leu Arg Arg Gln Val Asp Gly Met Ser Asn Asp Arg Ala Arg
145                 150                 155                 160

Met Glu Val Glu Arg Asp Asn Leu Ala Asp Asp Leu Gln Lys Leu Lys
                165                 170                 175

Leu Arg Leu Gln Glu Val Ile His Gln Arg Glu Glu Ala Glu Asn Asn
            180                 185                 190

Leu Ser Ala Phe Arg Ala Asp Val Asp Ser Ala Thr Leu Ala Arg Leu
        195                 200                 205

Asp Leu Glu Arg Arg Ile Glu Ser Leu Gln Glu Glu Ile Thr Phe Leu
    210                 215                 220

Lys Lys Ile His Glu Glu Glu Ile His Glu Leu Thr Ser Gln Met Gln
225                 230                 235                 240

Glu Thr Ser Val Gln Val Gln Met Asp Met Ser Lys Pro Asp Leu Thr
                245                 250                 255

Val Ala Leu Arg Asp Ile Arg Met Gln Tyr Glu Gly Ile Ala Ala Lys
            260                 265                 270

Asn Ile Ser Glu Ala Glu Asp Trp Tyr Lys Ser Lys Val Ser Asp Leu
        275                 280                 285

Asn Gln Ala Val Asn Lys Asn Asn Asp Ala Leu Arg Gln Ala Lys Gln
    290                 295                 300

Glu Ser Met Glu Phe Arg His Gln Ile Gln Ser Tyr Thr Cys Glu Ile
305                 310                 315                 320

Asp Ser Leu Lys Gly Thr Asn Glu Ser Leu Leu Arg Gln Met Arg Glu
                325                 330                 335

Met Glu Asp Arg Leu Gly Asn Glu Ala Gly Gly Tyr Gln Asp Ser Val
            340                 345                 350

Thr Arg Leu Glu Ala Glu Ile Ala Lys Met Lys Asp Glu Met Ala Arg
        355                 360                 365

His Leu Arg Glu Tyr Gln Asp Leu Leu Asn Val Lys Met Ala Leu Asp
    370                 375                 380

Ile Glu Ile Ala Thr Tyr Arg Lys Leu Leu Glu Gly Glu Glu Ser Arg
385                 390                 395                 400

Ile Thr Val Ser Gly Ser Lys Ser Ser His Ser Gly Ser His Ser Ala
                405                 410                 415

Ala Ser Leu Tyr Ser Thr Val Gly Phe Arg Glu Thr Ser Pro Asp Val
            420                 425                 430

Gly Arg Ser Ala Glu Val His Ser Lys Lys Thr Val Leu Ile Lys Thr
        435                 440                 445

Ile Glu Thr Arg Asp Gly Glu Val Val Ser Glu
    450                 455

<210>  57
<211>  17
<212>  PRT
<213>  Artificial Sequence

<220>
<223>  A part of Desmin (fragment)

<400>  57

Asp Glu Met Ala Arg His Leu Arg Glu Tyr Gln Asp Leu Leu Asn Val

1               5                   10                  15

Lys

<210>  58
<211>  21
<212>  PRT
<213>  Artificial Sequence

<220>
<223>  A part of Desmin (fragment)

<400>  58

Leu Asp Phe Asn Leu Ala Asp Ala Thr Asn Arg Asp Phe Leu Asp Thr
1               5                   10                  15

Arg Thr Asn Glu Lys
            20

<210>  59
<211>  10
<212>  PRT
<213>  Artificial Sequence

<220>
<223>  A part of Desmin (fragment)

<400>  59

Asn Ile Ser Glu Ala Glu Asp Trp Tyr Lys
1               5                   10

<210>  60
<211>  822
<212>  DNA
<213>  Artificial Sequence

<220>
<223>  Capping protein (Actin filament) muscle Z-line beta

<400>  60
atgaatgacc agcagctgga ctgtgctctg gacctgatga ggcgtcttcc tccccagcag      60

atcgagaaga atctcagtga cctcatcgac ctggtgccca gtctgtgtga ggacctactc     120

tcctctgtgg accagcctct gaagatcgcc cgggacaagg tagtggggaa agactacctg     180

ctctgtgact ataacagaga cggtgactcc tacagatccc catggagtaa taagtatgag     240

ccacccatcg acgatggtgc catgccatct gcccgcctgc gcaaactaga ggtggaagcc     300

aacaatgcct ttgaccagta tagagacctg tactttgagg gtggcgtatc gtctgtgtat     360

ctgtgggact tggatcatgg ctttgctggg gtcatcctca tcaagaaggc tggagacggc     420

tccaagaaga tcaaaggctg ctgggactcc atccatgtgg tggaggtgca ggagaagtcc     480

agcggacgga ccgctcacta caaactcacc tccaccgtca tgctgtggct ccagacgacc 540

aaggccgggt ctggaaccat gaacctgggt ggcagtctga caagacagat ggagaaagac 600

gagacagttg gagagtcttc cccacatatt gccaacatcg gccgcctggt ggaggatatg 660

gagaataaga ttcgctccac tctcaacgag atctactttg ggaagaccaa ggacatcgtc 720

aatggtttaa gatctattga ctctctgcct gataaccaaa agtaccggca gctccagaag 780

gagctgtctc aggtccttac ccagcgccag atcttcattg ac 822

<210> 61
<211> 274
<212> PRT
<213> Artificial Sequence

<220>
<223> Capping protein (Actin filament) muscle Z-line beta

<400> 61

Met Asn Asp Gln Gln Leu Asp Cys Ala Leu Asp Leu Met Arg Arg Leu
1               5                   10                  15

Pro Pro Gln Gln Ile Glu Lys Asn Leu Ser Asp Leu Ile Asp Leu Val
            20                  25                  30

Pro Ser Leu Cys Glu Asp Leu Leu Ser Ser Val Asp Gln Pro Leu Lys
        35                  40                  45

Ile Ala Arg Asp Lys Val Val Gly Lys Asp Tyr Leu Leu Cys Asp Tyr
    50                  55                  60

Asn Arg Asp Gly Asp Ser Tyr Arg Ser Pro Trp Ser Asn Lys Tyr Glu
65                  70                  75                  80

Pro Pro Ile Asp Asp Gly Ala Met Pro Ser Ala Arg Leu Arg Lys Leu
                85                  90                  95

Glu Val Glu Ala Asn Asn Ala Phe Asp Gln Tyr Arg Asp Leu Tyr Phe
            100                 105                 110

Glu Gly Gly Val Ser Ser Val Tyr Leu Trp Asp Leu Asp His Gly Phe
        115                 120                 125

Ala Gly Val Ile Leu Ile Lys Lys Ala Gly Asp Gly Ser Lys Lys Ile
    130                 135                 140

Lys Gly Cys Trp Asp Ser Ile His Val Val Glu Val Gln Glu Lys Ser
145                 150                 155                 160

Ser Gly Arg Thr Ala His Tyr Lys Leu Thr Ser Thr Val Met Leu Trp
                165                 170                 175

Leu Gln Thr Thr Lys Ala Gly Ser Gly Thr Met Asn Leu Gly Gly Ser
            180                 185                 190

Leu Thr Arg Gln Met Glu Lys Asp Glu Thr Val Gly Glu Ser Ser Pro
        195                 200                 205

His Ile Ala Asn Ile Gly Arg Leu Val Glu Asp Met Glu Asn Lys Ile
    210                 215                 220

Arg Ser Thr Leu Asn Glu Ile Tyr Phe Gly Lys Thr Lys Asp Ile Val
225                 230                 235                 240

Asn Gly Leu Arg Ser Ile Asp Ser Leu Pro Asp Asn Gln Lys Tyr Arg
                245                 250                 255

Gln Leu Gln Lys Glu Leu Ser Gln Val Leu Thr Gln Arg Gln Ile Phe
            260                 265                 270

Ile Asp

<210>  62
<211>  24
<212>  PRT
<213>  Artificial Sequence

<220>
<223>  Capping protein (Actin filament) muscle Z-line beta fragment

<400>  62

Asp Glu Thr Val Gly Glu Ser Ser Pro His Ile Ala Asn Ile Gly Arg
1               5                   10                  15

Leu Val Glu Asp Met Glu Asn Lys
            20

<210>  63
<211>  17
<212>  PRT
<213>  Artificial Sequence

<220>
<223>  Capping protein (Actin filament) muscle Z-line beta fragment

<400>  63

Asp Ile Val Asn Gly Leu Arg Ser Ile Asp Ser Leu Pro Asp Asn Gln
1               5                   10                  15

Lys

<210>  64
<211>  14
<212>  PRT
<213>  Artificial Sequence

<220>
<223>  Capping protein (Actin filament) muscle Z-line beta fragment

<400>  64

Glu Leu Ser Gln Val Leu Thr Gln Arg Gln Ile Phe Ile Asp
1               5                   10

<210>  65
<211>  12
<212>  PRT
<213>  Artificial Sequence

<220>
<223>  Capping protein (Actin filament) muscle Z-line beta fragment

<400>  65

Ile Arg Ser Thr Leu Asn Glu Ile Tyr Phe Gly Lys
1               5                   10

<210>  66
<211>  13
<212>  PRT
<213>  Artificial Sequence

<220>
<223>  Capping protein (Actin filament) muscle Z-line beta fragment

<400>  66

Leu Thr Ser Thr Val Met Leu Trp Leu Gln Thr Thr Lys
1               5                   10

<210>  67
<211>  10
<212>  PRT
<213>  Artificial Sequence

<220>
<223>  Capping protein (Actin filament) muscle Z-line beta fragment

<400>  67

Ser Ile Asp Ser Leu Pro Asp Asn Gln Lys
1               5                   10

<210>  68

<211>  17
<212>  PRT
<213>  Artificial Sequence

<220>
<223>  Capping protein (Actin filament) muscle Z-line beta fragment

<400>  68

Tyr Glu Pro Pro Ile Asp Asp Gly Ala Met Pro Ser Ala Arg Leu Arg
1               5                   10                  15

Lys

<210>  69
<211>  5799
<212>  DNA
<213>  Artificial Sequence

<220>
<223>  myosin heavy chain, fast skeletal muscle-like

<400>  69
atgagtacgg acgcggagat gcaagtctac ggcaaggctg ccatatacct tcgtaagtct      60

gagaaggaga ggatggaggc acaagccatg ccctttgatt caaagaacgc ctgctatgtg     120

acagacaagg tggagctgta ccttaagggt ttagtcactg ccagggccga cgggaagtgt     180

actgtgacag ctttagactt tcgtgaagga aaagagttca aagatgcaga catctatgag     240

atgaaccccc ctaagtacga caagattgag gacatggcca tgatgaccta cctgaatgaa     300

gcctctgtgt tgtataacct caaagagcgt tatgcagcat ggatgatcta tacctactct     360

gggctcttct gtgccacggt gaacccctac aagtggctcc cagtgtacga cgaagaggtt     420

gtcaacgcct acagagggaa gaagagggtg gaggctccac cacatatctt ctccgtctct     480

gacaacgcct ttcagttcat gatgattgat aaggagaacc agtccgtcct gattactgga     540

gaatccggtg caggaaagac tgtcaacacc aagcgtgtca tccagtactt tgccaccatt     600

gcagtgtctg gtggcaagaa ggaagcagac cccaacaaaa tgcaggggtc tcttgaggat     660

cagatcattg cagctaaccc tctgctagag tcttacggta atgccaagac agtgaggaac     720

gacaactcgt ctcgctttgg taaattcatc aggattcact tccaagctgg taaactggct     780

aaagctgaca ttgagaccta cctgctggag aagtccagag tgtccttcca actgcccgat     840

gagagaggct accacatctt cttccagatg atgacaggcc acaaacctga gctagttgaa     900

ttggcactcc tcaccaccaa cccctacgac ttccccatgt gcagccaggg tcagattgct     960

gtggccagca tcaacgacaa tgaagagctg gatgccacag atgaagccat tacaatcctg    1020

ggcttcacta atgaggagaa gcttggaata tacaagctga caggagctgt agtgcaccat    1080

ggcaacttga aattcaagca gaagcagcgt gaggagcagg ccgagccaga cggcacagag    1140

gtggctgata aaatcgccta cctgctgggc ctgaactcag ctgagatgtt gaaagctctg 1200

tgctacccca gagtgaaggt cggcaacgag tatgtgacca agggacagac tgtggctcag 1260

gttaataact cagtcagtgc tctggccaag tccatctatg agaggatgtt cttgtggatg 1320

gtcatccgta tcaatgagat gttggacacc aagaatccaa ggcagttcta tatcggtgtg 1380

cttgacattg ccgggtttga gatctttgat tacaacagca tggagcagct gtgcatcaac 1440

ttcaccaatg agaaactgca acagtttttc aaccacacca tgttcgtcct ggagcaagag 1500

gagtacaaga aggagggaat cgtctgggcc ttcattgact tcggcatgga tttggctgcc 1560

tgcattgagc ttattgagaa gccattgggc atcttctcca tccttgaaga ggagtgcatg 1620

ttccccaagt cttcagacac taccttcaag gacaagctgt acgcccagca tcttggtaaa 1680

acaaaggcgt ttgagaagcc caagcctgcc aaaggcaagg cagaggccca cttctccctg 1740

gtgcattacg ccggtactgt ggactacaac atcactggct ggctggagaa gaacaaggac 1800

cccttgaacg actcagtttg tcaactgtat gggaagtccg gagtcaaaat tttggctgcc 1860

ctgtatcccc ctccccctcc tgaggataaa gccaagaaag gaggcaagaa gaagggtggt 1920

tccatgcaga ctgtgtcctc ccagttcagg gagaacttac ataagctgat gaccaacttg 1980

aggagcactc atcctcactt tgtgcgctgc ctgatcccca acgagtcaaa gactccaggt 2040

ctgatggaga acttcctggt tatccaccag ctcaggtgta atggtgtact ggagggtatc 2100

aggatctgca gaaagggctt ccccagcaga atcatctatg ctgacttcaa gcaaaggtac 2160

aaagtactga atgccagcgt catccctgag ggccagttca tggacaacaa gaaggcttct 2220

gagaagctgc ttggatccat tgatgtgaat cacgaggatt acaagtttgg acacaccaag 2280

gtgttcttca aagccggtct gctgggtgtc ctggaggaga tgagagatga gaagctggcc 2340

tctctagtcg gcatgctcca ggctctcagc cgtggattcc tcatgaggag agagttcacc 2400

aagatgatgg agaggagaga atcaatttac tccatccagt acaacatccg ctcattcatg 2460

aatgtgaaaa cctggccatg gatgaagttg tacttcaaga tcaagcccct gctgcagagc 2520

gctgagactg agaaggagct ggccaacatg aaggagaact atgagaaaat gaagacagac 2580

ctggccaagg ctctgtctac aaagaagcaa atggaggaga agttggtgtc cctgacgcag 2640

gagaagaacg acctggcact ccaagtcgca tctgaaggag agagtctgaa cgatgctgag 2700

gaaaggtgcg aggggctcat caagagcaag atccagcagg aggccaaact caaagagacg 2760

accgagaggc tggaggatga ggaggagatc aatgctgagt tgactgccaa gaagaggaag 2820

ctggaggatg agtgctctga gctgaagaag gacattgatg atctggagct caccctggcc 2880

aaagtggaga aggagaagca cgccactgaa aacaaggtta aaaacctgac agaggagatg 2940

gcgtctatgg atgagagtgt tgccaagctg accaaggaga agaaagccct acaagaggcc 3000

caccagcaga cactggatga cctgcaggca gaggaggaca aagtcaacac tctgaccaag 3060

gccaagacca agctggaaca gcaagtggac gaccttgagg gttctctgga gcaagagaag 3120

aagctccgca tggaccttga gagatccaag agaaagctgg agggagatct gaaactggcc 3180

caggagtcca taatggacct ggagaatgac aagcagcaag ctgatgagaa aatcaagaag 3240

aaggagtttg agaccactca gctcctcagc aaggttgagg atgagcagtc tctgggagct 3300

cagctgcaga agaagatcaa ggaactccag gcccgtattg aggaactgga ggaggaaatt 3360

gaggctgagc gtgctgccag ggctaaggtt gagaagcaga gggctgatct ctccagggaa 3420

cttgaggaga tcagcgagag gctggaggag gccggaggcg ccactgctgc tcagattgag 3480

atgaacaaga agcgtgaggc tgagttccag aagctgcgtc gtgatcttga agagtccacc 3540

ctgcagcatg aggccacagc cgccgctctg cgcaagaagc aggccgacag tgtggctgag 3600

ctcggggagc agatcgacaa cctgcagcgc gtcaagcaga agctggagaa ggagaagagc 3660

gagtacaaga tggagattga tgacctctcc agcaacatgg aggccgttgc caaggctaag 3720

ggcaatctag agaagatgtg ccgtactctt gaggaccagc tgagcgagct caagactaag 3780

aatgatgaga atgttcgcca ggtcaacgac atcagcggac agagggccag actcctgaca 3840

gaaaatggtg agtttggtag gcagctggag gagaaggaag ccctggtgtc tcagctgacc 3900

agaggcaaac aggccttcac ccagcaggtg gaggagctga agagggcgat tgaggaggag 3960

gtcaaggcta aaaatgcact ggcccacgga gttcagtctg cccgccatga ctgtgacctc 4020

ctgagggagc agtttgagga ggagcaggag gccaaggcag agctgcaacg cggcatgtcc 4080

aaggccaata gtgaggtggc tcagtggagg actaagtatg aaactgatgc catccagcgc 4140

acagaggagc tggaggaggc caagaagaag ctggcccagc gtctgcagga tgccgaggag 4200

accattgagg cgaccaactc caagtgcgcc tccctggaga agaccaagca gagactacag 4260

ggagaggtgg aggacctcat gattgatgtt gagagagcca acgcattggc cgccaacctc 4320

gacaagaagc agaggaactt tgacaaggtt ctggcagagt ggaagcagaa gtatgaggag 4380

ggtcaggctg agctggaagg agctcagaag gaggctcgct ctatgagcac tgagctcttc 4440

aagatgaaga actcctacga ggaggctctg gatcatctgg agactctgaa gagagagaac 4500

aagaacctgc aacaggagat ctctgacctt actgagcaga ttggagagac tggcaagagc 4560

atccatgagc tggagaaggc caagaagacc gtggagacag agaagtctga gatccagacc 4620

gctctggagg aggctgaggg cacactggag cacgaggaat ccaagattct gcgtgtgcag 4680

ctggagctga accagatcaa gggtgaggtg gacaggaaga tcgctgagaa ggacgaggag 4740

atggagcaga tcaagaggaa cagccagagg atggttgact ccatgcagag caccctggac 4800

tctgaggtca ggagcaggaa tgatgccctg agggtgaaga agaagatgga gggagacctg 4860

aacgagatgg agatccagct gagccactcc aacaggcagg ccgctgaggc ccagaaacag 4920

ctgaggaatg tccagggaca gctcaaggat gcccaattgc accttgatga tgccgtccgt 4980

gtcgcagagg acatgaagga gcaggcagcc atggtggagc gcagaaacgg tctgatggtg 5040

gctgagatcg aggagctgag agttgctctg gagcagacag agagaggccg caaagtggct 5100

gagactgagc tggtagacgc cagcgagcgt gttggactgc tgcactccca gaacaccagc 5160

cttctgaaca ccaagaagaa gctggagact gacctggtgc aggtgcaggg agaggtggat 5220

gacatcgtcc aggaggccag gaatgcagaa gagaaggcca agaaggcaat cactgatgcg 5280

gcaatgatgg ctgaggagct gaagaaggag caggacacca gctctcacct ggagagaatg 5340

aagaagaacc tggagatcac agtcaaggac ctgcagcacc gcctggatga ggctgagaat 5400

ctggccatga agggaggcaa gaaacaactc cagaaactgg aatccagggt gcgtgagctt 5460

gagactgagg tggaggctga gcagagaaga ggtgtagacg cggtaaaggg agtccgcaag 5520

tatgagcgca gagtcaagga gctcacttac cagactgagg aggataagaa gaatgttaac 5580

agacttcagg acctggtaga taagctgcag atgaaagtga aggcctacaa gaggcacgct 5640

gaggaagcgg aggaagcagc aaaccagcac atgtctaagt tcaggaaggt tcagcatgag 5700

ctggaggagg ctgaggagcg tgctgacatc gctgagactc aggtcaacaa gctcagagcc 5760

aagacccgtg actctggaaa gggaaaagaa gttgctgaa 5799

<210> 70
<211> 1933
<212> PRT
<213> Artificial Sequence

<220>
<223> myosin heavy chain, fast skeletal muscle-like

<400> 70

Met Ser Thr Asp Ala Glu Met Gln Val Tyr Gly Lys Ala Ala Ile Tyr
1               5                   10                  15

Leu Arg Lys Ser Glu Lys Glu Arg Met Glu Ala Gln Ala Met Pro Phe
            20                  25                  30

Asp Ser Lys Asn Ala Cys Tyr Val Thr Asp Lys Val Glu Leu Tyr Leu
        35                  40                  45

Lys Gly Leu Val Thr Ala Arg Ala Asp Gly Lys Cys Thr Val Thr Ala
    50                  55                  60

Leu Asp Phe Arg Glu Gly Lys Glu Phe Lys Asp Ala Asp Ile Tyr Glu
65                  70                  75                  80

Met Asn Pro Pro Lys Tyr Asp Lys Ile Glu Asp Met Ala Met Met Thr

85                  90                  95

Tyr Leu Asn Glu Ala Ser Val Leu Tyr Asn Leu Lys Glu Arg Tyr Ala
            100                 105                 110

Ala Trp Met Ile Tyr Thr Tyr Ser Gly Leu Phe Cys Ala Thr Val Asn
        115                 120                 125

Pro Tyr Lys Trp Leu Pro Val Tyr Asp Glu Glu Val Val Asn Ala Tyr
    130                 135                 140

Arg Gly Lys Lys Arg Val Glu Ala Pro Pro His Ile Phe Ser Val Ser
145                 150                 155                 160

Asp Asn Ala Phe Gln Phe Met Met Ile Asp Lys Glu Asn Gln Ser Val
                165                 170                 175

Leu Ile Thr Gly Glu Ser Gly Ala Gly Lys Thr Val Asn Thr Lys Arg
            180                 185                 190

Val Ile Gln Tyr Phe Ala Thr Ile Ala Val Ser Gly Gly Lys Lys Glu
        195                 200                 205

Ala Asp Pro Asn Lys Met Gln Gly Ser Leu Glu Asp Gln Ile Ile Ala
    210                 215                 220

Ala Asn Pro Leu Leu Glu Ser Tyr Gly Asn Ala Lys Thr Val Arg Asn
225                 230                 235                 240

Asp Asn Ser Ser Arg Phe Gly Lys Phe Ile Arg Ile His Phe Gln Ala
                245                 250                 255

Gly Lys Leu Ala Lys Ala Asp Ile Glu Thr Tyr Leu Leu Glu Lys Ser
            260                 265                 270

Arg Val Ser Phe Gln Leu Pro Asp Glu Arg Gly Tyr His Ile Phe Phe
        275                 280                 285

Gln Met Met Thr Gly His Lys Pro Glu Leu Val Glu Leu Ala Leu Leu
    290                 295                 300

Thr Thr Asn Pro Tyr Asp Phe Pro Met Cys Ser Gln Gly Gln Ile Ala
305                 310                 315                 320

Val Ala Ser Ile Asn Asp Asn Glu Glu Leu Asp Ala Thr Asp Glu Ala
                325                 330                 335

Ile Thr Ile Leu Gly Phe Thr Asn Glu Glu Lys Leu Gly Ile Tyr Lys
            340                 345                 350

Leu Thr Gly Ala Val Val His His Gly Asn Leu Lys Phe Lys Gln Lys
        355                 360                 365

Gln Arg Glu Glu Gln Ala Glu Pro Asp Gly Thr Glu Val Ala Asp Lys
    370                 375                 380

Ile Ala Tyr Leu Leu Gly Leu Asn Ser Ala Glu Met Leu Lys Ala Leu
385                 390                 395                 400

Cys Tyr Pro Arg Val Lys Val Gly Asn Glu Tyr Val Thr Lys Gly Gln
                405                 410                 415

Thr Val Ala Gln Val Asn Asn Ser Val Ser Ala Leu Ala Lys Ser Ile
            420                 425                 430

Tyr Glu Arg Met Phe Leu Trp Met Val Ile Arg Ile Asn Glu Met Leu
        435                 440                 445

Asp Thr Lys Asn Pro Arg Gln Phe Tyr Ile Gly Val Leu Asp Ile Ala
    450                 455                 460

Gly Phe Glu Ile Phe Asp Tyr Asn Ser Met Glu Gln Leu Cys Ile Asn
465                 470                 475                 480

Phe Thr Asn Glu Lys Leu Gln Gln Phe Phe Asn His Thr Met Phe Val
                485                 490                 495

Leu Glu Gln Glu Glu Tyr Lys Lys Glu Gly Ile Val Trp Ala Phe Ile
            500                 505                 510

Asp Phe Gly Met Asp Leu Ala Ala Cys Ile Glu Leu Ile Glu Lys Pro
        515                 520                 525

Leu Gly Ile Phe Ser Ile Leu Glu Glu Glu Cys Met Phe Pro Lys Ser
    530                 535                 540

Ser Asp Thr Thr Phe Lys Asp Lys Leu Tyr Ala Gln His Leu Gly Lys
545                 550                 555                 560

Thr Lys Ala Phe Glu Lys Pro Lys Pro Ala Lys Gly Lys Ala Glu Ala
                565                 570                 575

His Phe Ser Leu Val His Tyr Ala Gly Thr Val Asp Tyr Asn Ile Thr
            580                 585                 590

Gly Trp Leu Glu Lys Asn Lys Asp Pro Leu Asn Asp Ser Val Cys Gln
        595                 600                 605

Leu Tyr Gly Lys Ser Gly Val Lys Ile Leu Ala Ala Leu Tyr Pro Pro
    610                 615                 620

Pro Pro Pro Glu Asp Lys Ala Lys Lys Gly Gly Lys Lys Lys Gly Gly
625                 630                 635                 640

Ser Met Gln Thr Val Ser Ser Gln Phe Arg Glu Asn Leu His Lys Leu
                645                 650                 655

Met Thr Asn Leu Arg Ser Thr His Pro His Phe Val Arg Cys Leu Ile
            660                 665                 670

Pro Asn Glu Ser Lys Thr Pro Gly Leu Met Glu Asn Phe Leu Val Ile
        675                 680                 685

His Gln Leu Arg Cys Asn Gly Val Leu Glu Gly Ile Arg Ile Cys Arg
    690                 695                 700

Lys Gly Phe Pro Ser Arg Ile Ile Tyr Ala Asp Phe Lys Gln Arg Tyr
705                 710                 715                 720

Lys Val Leu Asn Ala Ser Val Ile Pro Glu Gly Gln Phe Met Asp Asn
                725                 730                 735

Lys Lys Ala Ser Glu Lys Leu Leu Gly Ser Ile Asp Val Asn His Glu
            740                 745                 750

Asp Tyr Lys Phe Gly His Thr Lys Val Phe Phe Lys Ala Gly Leu Leu
        755                 760                 765

Gly Val Leu Glu Glu Met Arg Asp Glu Lys Leu Ala Ser Leu Val Gly
    770                 775                 780

Met Leu Gln Ala Leu Ser Arg Gly Phe Leu Met Arg Arg Glu Phe Thr
785                 790                 795                 800

Lys Met Met Glu Arg Arg Glu Ser Ile Tyr Ser Ile Gln Tyr Asn Ile
                805                 810                 815

Arg Ser Phe Met Asn Val Lys Thr Trp Pro Trp Met Lys Leu Tyr Phe
            820                 825                 830

Lys Ile Lys Pro Leu Leu Gln Ser Ala Glu Thr Glu Lys Glu Leu Ala
        835                 840                 845

Asn Met Lys Glu Asn Tyr Glu Lys Met Lys Thr Asp Leu Ala Lys Ala
    850                 855                 860

Leu Ser Thr Lys Lys Gln Met Glu Glu Lys Leu Val Ser Leu Thr Gln
865                 870                 875                 880

Glu Lys Asn Asp Leu Ala Leu Gln Val Ala Ser Glu Gly Glu Ser Leu
                885                 890                 895

Asn Asp Ala Glu Glu Arg Cys Glu Gly Leu Ile Lys Ser Lys Ile Gln
            900                 905                 910

Gln Glu Ala Lys Leu Lys Glu Thr Thr Glu Arg Leu Glu Asp Glu Glu
        915                 920                 925

Glu Ile Asn Ala Glu Leu Thr Ala Lys Lys Arg Lys Leu Glu Asp Glu
    930                 935                 940

Cys Ser Glu Leu Lys Lys Asp Ile Asp Asp Leu Glu Leu Thr Leu Ala
945                 950                 955                 960

Lys Val Glu Lys Glu Lys His Ala Thr Glu Asn Lys Val Lys Asn Leu
                965                 970                 975

Thr Glu Glu Met Ala Ser Met Asp Glu Ser Val Ala Lys Leu Thr Lys
            980                 985                 990

Glu Lys Lys Ala Leu Gln Glu Ala  His Gln Gln Thr Leu  Asp Asp Leu
        995                 1000                1005

Gln Ala  Glu Glu Asp Lys Val  Asn Thr Leu Thr Lys  Ala Lys Thr
    1010                1015                1020

Lys Leu  Glu Gln Gln Val Asp  Asp Leu Glu Gly Ser  Leu Glu Gln
    1025                1030                1035

Glu Lys  Lys Leu Arg Met Asp  Leu Glu Arg Ser Lys  Arg Lys Leu
    1040                1045                1050

Glu Gly  Asp Leu Lys Leu Ala  Gln Glu Ser Ile Met  Asp Leu Glu
    1055                1060                1065

Asn Asp  Lys Gln Gln Ala Asp  Glu Lys Ile Lys Lys  Lys Glu Phe
    1070                1075                1080

Glu Thr  Thr Gln Leu Leu Ser  Lys Val Glu Asp Glu  Gln Ser Leu

1085                1090                1095

Gly Ala Gln Leu Gln Lys Lys Ile Lys Glu Leu Gln Ala Arg Ile
    1100                1105                1110

Glu Glu Leu Glu Glu Glu Ile Glu Ala Glu Arg Ala Ala Arg Ala
    1115                1120                1125

Lys Val Glu Lys Gln Arg Ala Asp Leu Ser Arg Glu Leu Glu Glu
    1130                1135                1140

Ile Ser Glu Arg Leu Glu Glu Ala Gly Gly Ala Thr Ala Ala Gln
    1145                1150                1155

Ile Glu Met Asn Lys Lys Arg Glu Ala Glu Phe Gln Lys Leu Arg
    1160                1165                1170

Arg Asp Leu Glu Glu Ser Thr Leu Gln His Glu Ala Thr Ala Ala
    1175                1180                1185

Ala Leu Arg Lys Lys Gln Ala Asp Ser Val Ala Glu Leu Gly Glu
    1190                1195                1200

Gln Ile Asp Asn Leu Gln Arg Val Lys Gln Lys Leu Glu Lys Glu
    1205                1210                1215

Lys Ser Glu Tyr Lys Met Glu Ile Asp Asp Leu Ser Ser Asn Met
    1220                1225                1230

Glu Ala Val Ala Lys Ala Lys Gly Asn Leu Glu Lys Met Cys Arg
    1235                1240                1245

Thr Leu Glu Asp Gln Leu Ser Glu Leu Lys Thr Lys Asn Asp Glu
    1250                1255                1260

Asn Val Arg Gln Val Asn Asp Ile Ser Gly Gln Arg Ala Arg Leu
    1265                1270                1275

Leu Thr Glu Asn Gly Glu Phe Gly Arg Gln Leu Glu Glu Lys Glu
    1280                1285                1290

Ala Leu Val Ser Gln Leu Thr Arg Gly Lys Gln Ala Phe Thr Gln
    1295                1300                1305

Gln Val Glu Glu Leu Lys Arg Ala Ile Glu Glu Glu Val Lys Ala
    1310                1315                1320

Lys Asn Ala Leu Ala His Gly Val Gln Ser Ala Arg His Asp Cys
    1325                1330                1335

Asp Leu Leu Arg Glu Gln Phe Glu Glu Gln Glu Ala Lys Ala
    1340                1345                1350

Glu Leu Gln Arg Gly Met Ser Lys Ala Asn Ser Glu Val Ala Gln
        1355                1360                1365

Trp Arg Thr Lys Tyr Glu Thr Asp Ala Ile Gln Arg Thr Glu Glu
        1370                1375                1380

Leu Glu Glu Ala Lys Lys Lys Leu Ala Gln Arg Leu Gln Asp Ala
        1385                1390                1395

Glu Glu Thr Ile Glu Ala Thr Asn Ser Lys Cys Ala Ser Leu Glu
        1400                1405                1410

Lys Thr Lys Gln Arg Leu Gln Gly Glu Val Glu Asp Leu Met Ile
        1415                1420                1425

Asp Val Glu Arg Ala Asn Ala Leu Ala Ala Asn Leu Asp Lys Lys
        1430                1435                1440

Gln Arg Asn Phe Asp Lys Val Leu Ala Glu Trp Lys Gln Lys Tyr
        1445                1450                1455

Glu Glu Gly Gln Ala Glu Leu Glu Gly Ala Gln Lys Glu Ala Arg
        1460                1465                1470

Ser Met Ser Thr Glu Leu Phe Lys Met Lys Asn Ser Tyr Glu Glu
        1475                1480                1485

Ala Leu Asp His Leu Glu Thr Leu Lys Arg Glu Asn Lys Asn Leu
        1490                1495                1500

Gln Gln Glu Ile Ser Asp Leu Thr Glu Gln Ile Gly Glu Thr Gly
        1505                1510                1515

Lys Ser Ile His Glu Leu Glu Lys Ala Lys Lys Thr Val Glu Thr
        1520                1525                1530

Glu Lys Ser Glu Ile Gln Thr Ala Leu Glu Glu Ala Glu Gly Thr
        1535                1540                1545

Leu Glu His Glu Glu Ser Lys Ile Leu Arg Val Gln Leu Glu Leu
        1550                1555                1560

Asn Gln Ile Lys Gly Glu Val Asp Arg Lys Ile Ala Glu Lys Asp
        1565                1570                1575

Glu Glu Met Glu Gln Ile Lys Arg Asn Ser Gln Arg Met Val Asp
        1580                1585                1590

Ser Met Gln Ser Thr Leu Asp Ser Glu Val Arg Ser Arg Asn Asp
        1595                1600                1605

Ala Leu Arg Val Lys Lys Lys Met Glu Gly Asp Leu Asn Glu Met
        1610                1615                1620

Glu Ile Gln Leu Ser His Ser Asn Arg Gln Ala Ala Glu Ala Gln
        1625                1630                1635

Lys Gln Leu Arg Asn Val Gln Gly Gln Leu Lys Asp Ala Gln Leu
        1640                1645                1650

His Leu Asp Asp Ala Val Arg Val Ala Glu Asp Met Lys Glu Gln
        1655                1660                1665

Ala Ala Met Val Glu Arg Arg Asn Gly Leu Met Val Ala Glu Ile
        1670                1675                1680

Glu Glu Leu Arg Val Ala Leu Glu Gln Thr Glu Arg Gly Arg Lys
        1685                1690                1695

Val Ala Glu Thr Glu Leu Val Asp Ala Ser Glu Arg Val Gly Leu
        1700                1705                1710

Leu His Ser Gln Asn Thr Ser Leu Leu Asn Thr Lys Lys Lys Leu
        1715                1720                1725

Glu Thr Asp Leu Val Gln Val Gln Gly Glu Val Asp Asp Ile Val
        1730                1735                1740

Gln Glu Ala Arg Asn Ala Glu Glu Lys Ala Lys Lys Ala Ile Thr
        1745                1750                1755

Asp Ala Ala Met Met Ala Glu Glu Leu Lys Lys Glu Gln Asp Thr
        1760                1765                1770

Ser Ser His Leu Glu Arg Met Lys Lys Asn Leu Glu Ile Thr Val
        1775                1780                1785

Lys Asp Leu Gln His Arg Leu Asp Glu Ala Glu Asn Leu Ala Met
        1790                1795                1800

Lys Gly Gly Lys Lys Gln Leu Gln Lys Leu Glu Ser Arg Val Arg
    1805                1810                1815

Glu Leu Glu Thr Glu Val Glu Ala Glu Gln Arg Arg Gly Val Asp
    1820                1825                1830

Ala Val Lys Gly Val Arg Lys Tyr Glu Arg Arg Val Lys Glu Leu
    1835                1840                1845

Thr Tyr Gln Thr Glu Glu Asp Lys Lys Asn Val Asn Arg Leu Gln
    1850                1855                1860

Asp Leu Val Asp Lys Leu Gln Met Lys Val Lys Ala Tyr Lys Arg
    1865                1870                1875

His Ala Glu Glu Ala Glu Glu Ala Ala Asn Gln His Met Ser Lys
    1880                1885                1890

Phe Arg Lys Val Gln His Glu Leu Glu Glu Ala Glu Glu Arg Ala
    1895                1900                1905

Asp Ile Ala Glu Thr Gln Val Asn Lys Leu Arg Ala Lys Thr Arg
    1910                1915                1920

Asp Ser Gly Lys Gly Lys Glu Val Ala Glu
    1925                1930

<210>  71
<211>  7
<212>  PRT
<213>  Artificial Sequence

<220>
<223>  myosin heavy chain, fast skeletal muscle-like fragment

<400>  71

Ala Ala Ile Tyr Leu Arg Lys
1               5

<210>  72
<211>  10
<212>  PRT
<213>  Artificial Sequence

<220>
<223>  myosin heavy chain, fast skeletal muscle-like fragment

<400>  72

Ala Asp Ile Glu Thr Tyr Leu Leu Glu Lys

1               5                   10


<210>  73
<211>  19
<212>  PRT
<213>  Artificial Sequence

<220>
<223>  myosin heavy chain, fast skeletal muscle-like fragment

<400>  73

Ala Leu Gln Glu Ala His Gln Gln Thr Leu Asp Asp Leu Gln Ala Glu
1               5                   10                  15


Glu Asp Lys




<210>  74
<211>  11
<212>  PRT
<213>  Artificial Sequence

<220>
<223>  myosin heavy chain, fast skeletal muscle-like fragment

<400>  74

Asp Ile Asp Asp Leu Glu Leu Thr Leu Ala Lys
1               5                   10


<210>  75
<211>  11
<212>  PRT
<213>  Artificial Sequence

<220>
<223>  myosin heavy chain, fast skeletal muscle-like fragment

<400>  75

Glu Ala Leu Val Ser Gln Leu Thr Arg Gly Lys
1               5                   10


<210>  76
<211>  10
<212>  PRT
<213>  Artificial Sequence

<220>
<223>  myosin heavy chain, fast skeletal muscle-like fragment

<400>  76

Glu Phe Glu Thr Thr Gln Leu Leu Ser Lys
1               5                   10

<210>  77
<211>  10
<212>  PRT
<213>  Artificial Sequence

<220>
<223>  myosin heavy chain, fast skeletal muscle-like fragment

<400>  77

Glu Leu Thr Tyr Gln Thr Glu Glu Asp Lys
1               5                   10


<210>  78
<211>  15
<212>  PRT
<213>  Artificial Sequence

<220>
<223>  myosin heavy chain, fast skeletal muscle-like fragment

<400>  78

Glu Asn Gln Ser Val Leu Ile Thr Gly Glu Ser Gly Ala Gly Lys
1               5                   10                  15


<210>  79
<211>  19
<212>  PRT
<213>  Artificial Sequence

<220>
<223>  myosin heavy chain, fast skeletal muscle-like fragment

<400>  79

Glu Thr Thr Glu Arg Leu Glu Asp Glu Glu Glu Ile Asn Ala Glu Leu
1               5                   10                  15


Thr Ala Lys


<210>  80
<211>  10
<212>  PRT
<213>  Artificial Sequence

<220>
<223>  myosin heavy chain, fast skeletal muscle-like fragment

<400>  80

Phe Ile Arg Ile His Phe Gln Ala Gly Lys
1               5                   10


<210>  81
<211>  12
<212>  PRT

<213>  Artificial Sequence

<220>
<223>  myosin heavy chain, fast skeletal muscle-like fragment

<400>  81

Gly Phe Pro Ser Arg Ile Ile Tyr Ala Asp Phe Lys
1               5                   10


<210>  82
<211>  10
<212>  PRT
<213>  Artificial Sequence

<220>
<223>  myosin heavy chain, fast skeletal muscle-like fragment

<400>  82

Gly Leu Val Thr Ala Arg Ala Asp Gly Lys
1               5                   10


<210>  83
<211>  16
<212>  PRT
<213>  Artificial Sequence

<220>
<223>  myosin heavy chain, fast skeletal muscle-like fragment

<400>  83

Gly Gln Thr Val Ala Gln Val Asn Asn Ser Val Ser Ala Leu Ala Lys
1               5                   10                  15


<210>  84
<211>  12
<212>  PRT
<213>  Artificial Sequence

<220>
<223>  myosin heavy chain, fast skeletal muscle-like fragment

<400>  84

Ile Lys Pro Leu Leu Gln Ser Ala Glu Thr Glu Lys
1               5                   10


<210>  85
<211>  14
<212>  PRT
<213>  Artificial Sequence

<220>
<223>  myosin heavy chain, fast skeletal muscle-like fragment

<400>  85

Ile Leu Ala Ala Leu Tyr Pro Pro Pro Pro Pro Glu Asp Lys
1               5                   10

<210>  86
<211>  12
<212>  PRT
<213>  Artificial Sequence

<220>
<223>  myosin heavy chain, fast skeletal muscle-like fragment

<400>  86

Ile Leu Arg Val Gln Leu Glu Leu Asn Gln Ile Lys
1               5                   10

<210>  87
<211>  18
<212>  PRT
<213>  Artificial Sequence

<220>
<223>  myosin heavy chain, fast skeletal muscle-like fragment

<400>  87

Leu Ala Gln Arg Leu Gln Asp Ala Glu Glu Thr Ile Glu Ala Thr Asn
1               5                   10                  15

Ser Lys

<210>  88
<211>  16
<212>  PRT
<213>  Artificial Sequence

<220>
<223>  myosin heavy chain, fast skeletal muscle-like fragment

<400>  88

Leu Glu Gln Gln Val Asp Asp Leu Glu Gly Ser Leu Glu Gln Glu Lys
1               5                   10                  15

<210>  89
<211>  24
<212>  PRT
<213>  Artificial Sequence

<220>
<223>  myosin heavy chain, fast skeletal muscle-like fragment

<400>  89

Leu Glu Ser Arg Val Arg Glu Leu Glu Thr Glu Val Glu Ala Glu Gln
1               5                   10                  15

Arg Arg Gly Val Asp Ala Val Lys
            20

<210>  90
<211>  13
<212>  PRT
<213>  Artificial Sequence

<220>
<223>  myosin heavy chain, fast skeletal muscle-like fragment

<400>  90

Leu Leu Gly Ser Ile Asp Val Asn His Glu Asp Tyr Lys
1               5                   10

<210>  91
<211>  21
<212>  PRT
<213>  Artificial Sequence

<220>
<223>  myosin heavy chain, fast skeletal muscle-like fragment

<400>  91

Leu Arg Arg Asp Leu Glu Glu Ser Thr Leu Gln His Glu Ala Thr Ala
1               5                   10                  15

Ala Ala Leu Arg Lys
            20

<210>  92
<211>  12
<212>  PRT
<213>  Artificial Sequence

<220>
<223>  myosin heavy chain, fast skeletal muscle-like fragment

<400>  92

Leu Thr Gly Ala Val Val His His Gly Asn Leu Lys
1               5                   10

<210>  93
<211>  32
<212>  PRT
<213>  Artificial Sequence

<220>
<223>  myosin heavy chain, fast skeletal muscle-like fragment

<400>  93

Asn Asp Glu Asn Val Arg Gln Val Asn Asp Ile Ser Gly Gln Arg Ala
1               5                   10                  15

Arg Leu Leu Thr Glu Asn Gly Glu Phe Gly Arg Gln Leu Glu Glu Lys
            20                  25                  30

<210>  94
<211>  7
<212>  PRT
<213>  Artificial Sequence

<220>
<223>  myosin heavy chain, fast skeletal muscle-like fragment

<400>  94

Asn Leu Glu Ile Thr Val Lys
1               5

<210>  95
<211>  18
<212>  PRT
<213>  Artificial Sequence

<220>
<223>  myosin heavy chain, fast skeletal muscle-like fragment

<400>  95

Asn Leu Gln Gln Glu Ile Ser Asp Leu Thr Glu Gln Ile Gly Glu Thr
1               5                   10                  15

Gly Lys

<210>  96
<211>  14
<212>  PRT
<213>  Artificial Sequence

<220>
<223>  myosin heavy chain, fast skeletal muscle-like fragment

<400>  96

Asn Ser Tyr Glu Glu Ala Leu Asp His Leu Glu Thr Leu Lys
1               5                   10

<210>  97
<211>  19
<212>  PRT
<213>  Artificial Sequence

<220>
<223>  myosin heavy chain, fast skeletal muscle-like fragment

<400>  97

Gln Ala Asp Ser Val Ala Glu Leu Gly Glu Gln Ile Asp Asn Leu Gln

1               5                   10                  15

Arg Val Lys

<210>  98
<211>  11
<212>  PRT
<213>  Artificial Sequence

<220>
<223>  myosin heavy chain, fast skeletal muscle-like fragment

<400>  98

Gln Ala Phe Thr Gln Gln Val Glu Glu Leu Lys
1               5                   10

<210>  99
<211>  16
<212>  PRT
<213>  Artificial Sequence

<220>
<223>  myosin heavy chain, fast skeletal muscle-like fragment

<400>  99

Gln Arg Glu Glu Gln Ala Glu Pro Asp Gly Thr Glu Val Ala Asp Lys
1               5                   10                  15

<210>  100
<211>  8
<212>  PRT
<213>  Artificial Sequence

<220>
<223>  myosin heavy chain, fast skeletal muscle-like fragment

<400>  100

Arg Ala Ile Glu Glu Glu Val Lys
1               5

<210>  101
<211>  20
<212>  PRT
<213>  Artificial Sequence

<220>
<223>  myosin heavy chain, fast skeletal muscle-like fragment

<400>  101

Ser Glu Ile Gln Thr Ala Leu Glu Glu Ala Glu Gly Thr Leu Glu His
1               5                   10                  15

Glu Glu Ser Lys
            20

<210>  102
<211>  12
<212>  PRT
<213>  Artificial Sequence

<220>
<223>  myosin heavy chain, fast skeletal muscle-like fragment

<400>  102

Thr Val Arg Asn Asp Asn Ser Ser Arg Phe Gly Lys
1               5                   10

<210>  103
<211>  27
<212>  PRT
<213>  Artificial Sequence

<220>
<223>  myosin heavy chain, fast skeletal muscle-like fragment

<400>  103

Val Ala Glu Thr Glu Leu Val Asp Ala Ser Glu Arg Val Gly Leu Leu
1               5                   10                  15

His Ser Gln Asn Thr Ser Leu Leu Asn Thr Lys
            20                  25

<210>  104
<211>  13
<212>  PRT
<213>  Artificial Sequence

<220>
<223>  myosin heavy chain, fast skeletal muscle-like fragment

<400>  104

Val Glu Asp Glu Gln Ser Leu Gly Ala Gln Leu Gln Lys
1               5                   10

<210>  105
<211>  8
<212>  PRT
<213>  Artificial Sequence

<220>
<223>  myosin heavy chain, fast skeletal muscle-like fragment

<400>  105

Val Gly Asn Glu Tyr Val Thr Lys
1               5

<210>  106
<211>  21
<212>  PRT
<213>  Artificial Sequence

<220>
<223>  myosin heavy chain, fast skeletal muscle-like fragment

<400>  106

Val Gln His Glu Leu Glu Glu Ala Glu Glu Arg Ala Asp Ile Ala Glu
1               5                   10                  15

Thr Gln Val Asn Lys
            20

<210>  107
<211>  13
<212>  PRT
<213>  Artificial Sequence

<220>
<223>  myosin heavy chain, fast skeletal muscle-like fragment

<400>  107

Tyr Glu Glu Gly Gln Ala Glu Leu Glu Gly Ala Gln Lys
1               5                   10

<210>  108
<211>  16
<212>  PRT
<213>  Artificial Sequence

<220>
<223>  myosin heavy chain, fast skeletal muscle-like fragment

<400>  108

Tyr Glu Thr Asp Ala Ile Gln Arg Thr Glu Glu Leu Glu Glu Ala Lys
1               5                   10                  15

<210>  109
<211>  2532
<212>  DNA
<213>  Artificial Sequence

<220>
<223>  glycogen phosphorylase, muscle form-like

<400>  109
atgtcaaaac cattgtcaga ccacgataaa aggaagcaga tctccgtgag aggtcttgct      60

ggtgtggaaa atgtcgcaga actgaaggtc gctttcaaca ggcatctcca ttttacactg     120

gtcaaggaca gaaatgtggc aaacaaacgg gattactact ttgctctcgc caacaccgtg     180

cgtgaccact tggtgggcag gtggatcaga acccagcagt actactatga gaaagatccc     240

aaacgtgtgt actacatctc cctggagttt tatatgggtc gcaccctgca gaacactatg 300

gtgaacctgg ccctggagaa cgcttgtgat gaggccatat accagctggg tctggacatg 360

gaggagctgg aggacatgga agaggatgct ggcctgggaa acggtggtct tggccgtctt 420

gccgcatgct tcctggactc aatggcttct ctaggccttg ctgcctatgg ctatggtatc 480

cgctatgagt ttggcatctt caatcagaag atcgtcaatg gctggcaggt tgaggaggcc 540

gatgattggt tgcgttacgg caacccctgg gagaaggccc gccctgagta catgcgcccc 600

gtcaagttct atggcaggac cgagcacacc ccagatggtg tgaaatgggt tgacactcag 660

gtagtgttgg ctctgccata tgacacccct attcccggct acagaaacaa cattgtcaac 720

accatgagac tgtggtctgc taaggcccca tgcgacttca acctgaaaga cttcaacgtt 780

ggtggctaca ttcaggctgt gttggacaga aacttgtgtg agaacatttc ccgcgtgctg 840

taccccaatg ataatttctt tgagggcaag gagctgcgtc tgaagcagga gtactttgtg 900

gtggccgcca ctctgcagga cgtcgtccgt cgtttcaagg cctctaagtt tggctccaga 960

gagattgtcc gcacagactt cgccgagcta ccaaacaaag ttgccatcca gctgaatgac 1020

actcaccctg ccatggctat tcctgagctg atgagggtcc tggttgatga ggagaagctg 1080

ggttgggaca aggcctggga cgtgtgtgtc cgtacctgtg cctacacaaa ccacaccgtg 1140

ctgcctgagg ccctggagcg ctggcccatt gacctgttcc atcacctgct gccacgtcac 1200

ctggagatca tctacgagat caaccgtcgc ttcatggagt atgttgcctc gaagttccct 1260

ggagacaacg accgtctgcg tcgcatgtcc ctgattgagg agggaggatg caagaaagtc 1320

aacatggctc atctgtgtat cgttggttcc catgctgtca acggcgtggc ccgcatccac 1380

tctgagatcc tcatcgccac tctgttcaag gacttctatg agttggaccc acacaagttt 1440

cagaacaaga ccaatggcat caccccccgt cgctggctgg ttatgtgcaa ccccggcttg 1500

gcggaggtca tcgcagagaa aattggagag gagtttatcc gtgaccttga ccagcttaag 1560

cgactgttga agttcgttaa tgatgatgct ttcatccgtg acatcgccaa agtcaagcag 1620

gagaacaagc tgaagttcgc tgtgcacctg gaagagcact acaaggtcaa gatcaacccc 1680

cagtccatgt ttgacttcca agtcaaaaga atccacgagt acaaaagaca gctgctcaac 1740

tgtctgcaca tgatcaccta ctacaaccgt atcaagaagg agcccaacaa gcactggacc 1800

ccaagaacca tcatggtcgg aggaaaggct gcaccaggct accacacagc caagatgatc 1860

atccgtctca tcacagctat cggtgaggtt gtcaaccacg accccgttgt cggcgaccgc 1920

ctcaaagtta tcttcctgga gaactacaga gtcaccctgg ctgagaaagc catcccctct 1980

gccgacctgt ctgagcagat ctctacagct ggcaccgagg cctctggcac tggtaacatg 2040

aagttcatgc tgaacggtgc tcttaccatt ggcaccatgg atggagccaa tgtggagatg 2100

gccgaggagg ccggagagga aaacttcttc atcttcggta tgagagtgga ggatgtggac 2160

gcaatggacg ccggcaaagg ataccacgcc tctgagtact acaaccgtat tcccgagctg 2220

aagcaggcca tggaccagat tgctggcggc ttcttcagtc ccaagcagcc tgacctcttc 2280

aaggaacttg tggacctgct gatgcaccat gacaggttca aggtgtttgc tgactacgaa 2340

gcctacatca aatgccagga caaggtcaac caactgtaca agaatcccaa ggaatggacc 2400

aagatggtga tccataacat tgcgggctgt ggtaaattct ccagcgaccg caccattgcc 2460

cagtacgccc gagagatctg gggcatggag cccagcctgg agaagatccc tgcccccgat 2520

gagaaactca aa 2532

<210> 110
<211> 844
<212> PRT
<213> Artificial Sequence

<220>
<223> glycogen phosphorylase, muscle form-like

<400> 110

Met Ser Lys Pro Leu Ser Asp His Asp Lys Arg Lys Gln Ile Ser Val
1               5                   10                  15

Arg Gly Leu Ala Gly Val Glu Asn Val Ala Glu Leu Lys Val Ala Phe
            20                  25                  30

Asn Arg His Leu His Phe Thr Leu Val Lys Asp Arg Asn Val Ala Asn
        35                  40                  45

Lys Arg Asp Tyr Tyr Phe Ala Leu Ala Asn Thr Val Arg Asp His Leu
    50                  55                  60

Val Gly Arg Trp Ile Arg Thr Gln Gln Tyr Tyr Tyr Glu Lys Asp Pro
65                  70                  75                  80

Lys Arg Val Tyr Tyr Ile Ser Leu Glu Phe Tyr Met Gly Arg Thr Leu
                85                  90                  95

Gln Asn Thr Met Val Asn Leu Ala Leu Glu Asn Ala Cys Asp Glu Ala
            100                 105                 110

Ile Tyr Gln Leu Gly Leu Asp Met Glu Glu Leu Glu Asp Met Glu Glu
        115                 120                 125

Asp Ala Gly Leu Gly Asn Gly Gly Leu Gly Arg Leu Ala Ala Cys Phe
    130                 135                 140

Leu Asp Ser Met Ala Ser Leu Gly Leu Ala Ala Tyr Gly Tyr Gly Ile
145                 150                 155                 160

Arg Tyr Glu Phe Gly Ile Phe Asn Gln Lys Ile Val Asn Gly Trp Gln
                165                 170                 175

Val Glu Glu Ala Asp Asp Trp Leu Arg Tyr Gly Asn Pro Trp Glu Lys
            180                 185                 190

Ala Arg Pro Glu Tyr Met Arg Pro Val Lys Phe Tyr Gly Arg Thr Glu
        195                 200                 205

His Thr Pro Asp Gly Val Lys Trp Val Asp Thr Gln Val Val Leu Ala
    210                 215                 220

Leu Pro Tyr Asp Thr Pro Ile Pro Gly Tyr Arg Asn Asn Ile Val Asn
225                 230                 235                 240

Thr Met Arg Leu Trp Ser Ala Lys Ala Pro Cys Asp Phe Asn Leu Lys
                245                 250                 255

Asp Phe Asn Val Gly Gly Tyr Ile Gln Ala Val Leu Asp Arg Asn Leu
            260                 265                 270

Cys Glu Asn Ile Ser Arg Val Leu Tyr Pro Asn Asp Asn Phe Phe Glu
        275                 280                 285

Gly Lys Glu Leu Arg Leu Lys Gln Glu Tyr Phe Val Val Ala Ala Thr
    290                 295                 300

Leu Gln Asp Val Val Arg Arg Phe Lys Ala Ser Lys Phe Gly Ser Arg
305                 310                 315                 320

Glu Ile Val Arg Thr Asp Phe Ala Glu Leu Pro Asn Lys Val Ala Ile
                325                 330                 335

Gln Leu Asn Asp Thr His Pro Ala Met Ala Ile Pro Glu Leu Met Arg
            340                 345                 350

Val Leu Val Asp Glu Glu Lys Leu Gly Trp Asp Lys Ala Trp Asp Val
        355                 360                 365

Cys Val Arg Thr Cys Ala Tyr Thr Asn His Thr Val Leu Pro Glu Ala
    370                 375                 380

Leu Glu Arg Trp Pro Ile Asp Leu Phe His His Leu Leu Pro Arg His
385                 390                 395                 400

Leu Glu Ile Ile Tyr Glu Ile Asn Arg Arg Phe Met Glu Tyr Val Ala
                405                 410                 415

Ser Lys Phe Pro Gly Asp Asn Asp Arg Leu Arg Arg Met Ser Leu Ile
            420                 425                 430

Glu Glu Gly Gly Cys Lys Lys Val Asn Met Ala His Leu Cys Ile Val
        435                 440                 445

Gly Ser His Ala Val Asn Gly Val Ala Arg Ile His Ser Glu Ile Leu
    450                 455                 460

Ile Ala Thr Leu Phe Lys Asp Phe Tyr Glu Leu Asp Pro His Lys Phe
465                 470                 475                 480

Gln Asn Lys Thr Asn Gly Ile Thr Pro Arg Arg Trp Leu Val Met Cys
                485                 490                 495

Asn Pro Gly Leu Ala Glu Val Ile Ala Glu Lys Ile Gly Glu Glu Phe
            500                 505                 510

Ile Arg Asp Leu Asp Gln Leu Lys Arg Leu Leu Lys Phe Val Asn Asp
        515                 520                 525

Asp Ala Phe Ile Arg Asp Ile Ala Lys Val Lys Gln Glu Asn Lys Leu
    530                 535                 540

Lys Phe Ala Val His Leu Glu Glu His Tyr Lys Val Lys Ile Asn Pro
545                 550                 555                 560

Gln Ser Met Phe Asp Phe Gln Val Lys Arg Ile His Glu Tyr Lys Arg
                565                 570                 575

Gln Leu Leu Asn Cys Leu His Met Ile Thr Tyr Tyr Asn Arg Ile Lys
            580                 585                 590

Lys Glu Pro Asn Lys His Trp Thr Pro Arg Thr Ile Met Val Gly Gly
        595                 600                 605

Lys Ala Ala Pro Gly Tyr His Thr Ala Lys Met Ile Ile Arg Leu Ile
    610                 615                 620

Thr Ala Ile Gly Glu Val Val Asn His Asp Pro Val Val Gly Asp Arg
625                 630                 635                 640

Leu Lys Val Ile Phe Leu Glu Asn Tyr Arg Val Thr Leu Ala Glu Lys
                645                 650                 655

Ala Ile Pro Ser Ala Asp Leu Ser Glu Gln Ile Ser Thr Ala Gly Thr
            660                 665                 670

Glu Ala Ser Gly Thr Gly Asn Met Lys Phe Met Leu Asn Gly Ala Leu
        675                 680                 685

Thr Ile Gly Thr Met Asp Gly Ala Asn Val Glu Met Ala Glu Glu Ala
    690                 695                 700

Gly Glu Glu Asn Phe Phe Ile Phe Gly Met Arg Val Glu Asp Val Asp
705                 710                 715                 720

Ala Met Asp Ala Gly Lys Gly Tyr His Ala Ser Glu Tyr Tyr Asn Arg
                725                 730                 735

Ile Pro Glu Leu Lys Gln Ala Met Asp Gln Ile Ala Gly Gly Phe Phe
            740                 745                 750

Ser Pro Lys Gln Pro Asp Leu Phe Lys Glu Leu Val Asp Leu Leu Met
        755                 760                 765

His His Asp Arg Phe Lys Val Phe Ala Asp Tyr Glu Ala Tyr Ile Lys
    770                 775                 780

Cys Gln Asp Lys Val Asn Gln Leu Tyr Lys Asn Pro Lys Glu Trp Thr
785                 790                 795                 800

Lys Met Val Ile His Asn Ile Ala Gly Cys Gly Lys Phe Ser Ser Asp
                805                 810                 815

Arg Thr Ile Ala Gln Tyr Ala Arg Glu Ile Trp Gly Met Glu Pro Ser
            820                 825                 830

Leu Glu Lys Ile Pro Ala Pro Asp Glu Lys Leu Lys
        835                 840

<210>  111
<211>  9
<212>  PRT
<213>  Artificial Sequence

<220>
<223>  glycogen phosphorylase, muscle form-like fragment

<400>  111

Asp Phe Tyr Glu Leu Asp Pro His Lys
1               5

<210>  112
<211>  10
<212>  PRT
<213>  Artificial Sequence

<220>
<223>  glycogen phosphorylase, muscle form-like fragment

<400>  112

Phe Ala Val His Leu Glu Glu His Tyr Lys
1               5                   10


<210>  113
<211>  13
<212>  PRT
<213>  Artificial Sequence

<220>
<223>  glycogen phosphorylase, muscle form-like fragment

<400>  113

Phe Tyr Gly Arg Thr Glu His Thr Pro Asp Gly Val Lys
1               5                   10


<210>  114
<211>  15
<212>  PRT
<213>  Artificial Sequence

<220>
<223>  glycogen phosphorylase, muscle form-like fragment

<400>  114

Gly Tyr His Ala Ser Glu Tyr Tyr Asn Arg Ile Pro Glu Leu Lys
1               5                   10                  15


<210>  115
<211>  13
<212>  PRT
<213>  Artificial Sequence

<220>
<223>  glycogen phosphorylase, muscle form-like fragment

<400>  115

Ile Gly Glu Glu Phe Ile Arg Asp Leu Asp Gln Leu Lys
1               5                   10


<210>  116
<211>  14
<212>  PRT
<213>  Artificial Sequence

<220>

<223>  glycogen phosphorylase, muscle form-like fragment

<400>  116

Gln Ala Met Asp Gln Ile Ala Gly Gly Phe Phe Ser Pro Lys
1               5                   10


<210>  117
<211>  17
<212>  PRT
<213>  Artificial Sequence

<220>
<223>  glycogen phosphorylase, muscle form-like fragment

<400>  117

Gln Ile Ser Val Arg Gly Leu Ala Gly Val Glu Asn Val Ala Glu Leu
1               5                   10                  15


Lys


<210>  118
<211>  10
<212>  PRT
<213>  Artificial Sequence

<220>
<223>  glycogen phosphorylase, muscle form-like fragment

<400>  118

Val Phe Ala Asp Tyr Glu Ala Tyr Ile Lys
1               5                   10


<210>  119
<211>  14
<212>  PRT
<213>  Artificial Sequence

<220>
<223>  glycogen phosphorylase, muscle form-like fragment

<400>  119

Val Ile Phe Leu Glu Asn Tyr Arg Val Thr Leu Ala Glu Lys
1               5                   10


<210>  120
<211>  3399
<212>  DNA
<213>  Artificial Sequence

<220>
<223>  myosin-binding protein C, fast-type-like

<400>  120

atgcctgaag ccgctgatgc agtgaagccg gagggcgagg agggagaagc accagcagaa 60

gccgaagaaa atactgcaga agatgatgag ccactgcctg aaggagagga gggtgcccat 120

cagtcacagg agattacagg actgtttctt cagcaccctg agactacggt tgccataaca 180

ggtacggaca ttgtgtttac tgccaaagtg gactctacta cactatcaag gaaacccacc 240

attaaatggc tgaaggggaa gtggctggac ctgggcagca aagcaggaaa acacatgcag 300

ttcaaagaga cttttgacag agccacaaag atctacacct gggacatgaa gataatcaag 360

gtggtccctg gtgacgctgg ggcctacagg tgtgaggtca cctccaaaga caagtgtgac 420

agcagcgctt tcgacatctc cgtggaggct gtgcaaattg aggaggaaca tgattctttg 480

gccgcattca aaagaacgga tgctggagag gatgaaggca gtttggattt cagtgctttg 540

ctgaaagcta ccaagaagaa gaagaagcca gtcgtagatg aggagaaggc agacgtgtgg 600

gaaatcctga agaacgctca acccagcgag tatgagaaga ttgcttttga gtacggcatt 660

acagacctga ggggtctgct caagcgactg aagaagatga agactgttga gcccaagcac 720

agcgacgctt tcctaaagag aatggagtct gcctactctg tggataaggg caagaaaatc 780

gtactgcagg tggaagtggt tgacccaaat gcccaggtca aatggttgaa gaacggtcag 840

gagataaaat catcagccaa gtacatcata gaatcagttg gcaacatcag gacgctcacc 900

atcaacaagt gtagcctggc tgacgacgct gcgtacgagt gtgtgattgg ggaagagaag 960

tccttcacag aggtgtttgt caaagagccc ccggtcacca tcaccaagct gctggatgat 1020

taccacgtgg tggtgggaga gagagtggag tttgaggtgg aggtgtctgt ggagggagca 1080

cacgttaact ggatgtttga ggaccaagaa ctctccaggg acacccataa gtaccgcttt 1140

aagaaggacg ggttgaagca catgctcatc atccaagagg ctacgctgga tgacattgga 1200

atgtactggt gcttcaccaa cggcgggcga accaaaggag agctagaagt tgaagagaaa 1260

gaactggagg tgttgcagaa catcgctgac ctgacggtga aggccgagga ccaggccatg 1320

ttcaagtgtg aggtgtctga tgagaaggta acagggaaat ggctcaaaga tggcgtggag 1380

gtgctgccca gcagtcgcat caagctgacc cacatcggaa gaatccatcg gcttaccata 1440

gacgacgtca agcccgagga cgctggagac tataccttta tcccagacgg atatgccctc 1500

tcactgtccg ccaaactcaa cttcctggaa attaagatcg actatgtccc ccgccaagag 1560

ccacccaaga tccatctaga caccaccggc aacatggttt cccagaacac catcattgtg 1620

gtggctggca acaagctgcg cctggacgtg gagatctctg gagagcctcc tcccactgtc 1680

gtctgggcaa agggagacac ggcaatcaca gctgtggagg ggcgtgtgag gactgagagc 1740

cggaaggacc tgagctgctt cgtcatagag ggggcagaga gagaggatga gggcaactac 1800

accatcattg tcaccaaccc tgccggagag gacaaggctc atctgtttgt caagattgtt 1860

gatgtgcctg actgccctga gaatgtaaag tgtacctcgg tgggagaaga ctgtgccacc 1920

atggtctggg acccacccaa gttcgacgga ggcgcaccag tcacaggcta tctcatggag 1980

aggaagaaga agggctccac caggtggacg aagctcaact ttgacgtgta cgagggggtg 2040

acgtacgaag ccaagaggat gattgagggc gtgctctacg agatgagagt gtacgccgtc 2100

aatggcattg gcatgtccca gcccagcctc aattccaaac ccttcatgcc catcgctgca 2160

accagtgagc ctctgggcct caaggtgcat gatgtcacag acaccacatg taccctgaag 2220

tggctggccc ctgagaagat cggtgccgga ggcctggacg gatacgttat tgagtactgc 2280

aaggagggag acacggagtg ggtggtggcg aacactgagc tggtggagag acagaattac 2340

acagtccgca acctccccac cgcggagaag atcaacttca gagtggtggc cgtgaacatt 2400

gctggacgca gcccccctac cggcctgagc cagcccgtca ctatccgcga gatcgtggaa 2460

ctgcccaaga tccgcctccc tcgctacttg agacagaagt acattagaag agtgggcgac 2520

aaaatcaacc tgaccatccc cttccagggt aagccacgtc ccaaagtgca atggtacaag 2580

gatggggaag agcttgacac tcgagttgcc agcatacgca attccgaagt ggactctatc 2640

ctgttcatcc gctcggctga gagatcccac tctgggaagt acgagttggt cctgcagatt 2700

gagaacatgg aggccagggc tattctggaa atcaggatcg tagaggcacc tgggcctccc 2760

gaggtagtga aggtcacaga cgtttggggc ttcaacgctg cgctggagtg gaagccaccg 2820

aaggacgacg gcaactgcga gatcaccggt tacacaatac agaaggcgga caagaagaca 2880

aatgaatggt tcactatcta cgagcacaac aggaggacaa actgcacagc ctcagacctg 2940

atcatgggaa atgagtacat gttccgtgtc ttcagcgaga acctcgttgg aaagagtgag 3000

gatttctgcc tcagcaagga cacagccatc atacctaaga taggattgga gtacaaccca 3060

cctccattca aggagaagga tatgcaaagc gcccccaagt tcatacagcc cctgcttgac 3120

aggtctgtgg tggcaggcta cagtaccacc atcagctctg ctgtcaagac ttgtcctaaa 3180

cctaagatca ggtggttgag gaataagatt cccctggacg agaaccctag gttcctgatg 3240

cagaacaacc agggggtttt gaccctgaac atccgcaagc ccagtcagta tgacgggggc 3300

aagttcacct gcaaggctat caactctttg ggggaggacg tcgtggagtg caccctactc 3360

gtacgagctc tcaaggacaa ggaagatgga gacgagaaa 3399

<210> 121
<211> 1133
<212> PRT
<213> Artificial Sequence

<220>
<223> myosin-binding protein C, fast-type-like

<400> 121
   Met Pro Glu Ala Ala Asp Ala Val Lys Pro Glu Gly Glu Glu Gly Glu
   1                   5                        10                       15
   Ala Pro Ala Glu Ala Glu Glu Asn Thr Ala Glu Asp Asp Glu Pro Leu
               20                       25                       30
   Pro Glu Gly Glu Glu Gly Ala His Gln Ser Gln Glu Ile Thr Gly Leu
           35                       40                       45
   Phe Leu Gln His Pro Glu Thr Thr Val Ala Ile Thr Gly Thr Asp Ile
       50                       55                       60
   Val Phe Thr Ala Lys Val Asp Ser Thr Thr Leu Ser Arg Lys Pro Thr
   65                   70                       75                       80
   Ile Lys Trp Leu Lys Gly Lys Trp Leu Asp Leu Gly Ser Lys Ala Gly
                   85                       90                       95
   Lys His Met Gln Phe Lys Glu Thr Phe Asp Arg Ala Thr Lys Ile Tyr
               100                      105                      110
   Thr Trp Asp Met Lys Ile Ile Lys Val Val Pro Gly Asp Ala Gly Ala
           115                      120                      125
   Tyr Arg Cys Glu Val Thr Ser Lys Asp Lys Cys Asp Ser Ser Ala Phe
       130                      135                      140
   Asp Ile Ser Val Glu Ala Val Gln Ile Glu Glu Glu His Asp Ser Leu
   145                 150                      155                      160
   Ala Ala Phe Lys Arg Thr Asp Ala Gly Glu Asp Glu Gly Ser Leu Asp
                   165                      170                      175
   Phe Ser Ala Leu Leu Lys Ala Thr Lys Lys Lys Lys Lys Pro Val Val
               180                      185                      190
   Asp Glu Glu Lys Ala Asp Val Trp Glu Ile Leu Lys Asn Ala Gln Pro
           195                      200                      205
   Ser Glu Tyr Glu Lys Ile Ala Phe Glu Tyr Gly Ile Thr Asp Leu Arg
       210                      215                      220
   Gly Leu Leu Lys Arg Leu Lys Lys Met Lys Thr Val Glu Pro Lys His
   225                 230                      235                      240
   Ser Asp Ala Phe Leu Lys Arg Met Glu Ser Ala Tyr Ser Val Asp Lys
                   245                      250                      255
   Gly Lys Lys Ile Val Leu Gln Val Glu Val Val Asp Pro Asn Ala Gln
               260                 265                 270
   Val Lys Trp Leu Lys Asn Gly Gln Glu Ile Lys Ser Ser Ala Lys Tyr
           275                 280                 285
   Ile Ile Glu Ser Val Gly Asn Ile Arg Thr Leu Thr Ile Asn Lys Cys
       290                 295                 300
   Ser Leu Ala Asp Asp Ala Ala Tyr Glu Cys Val Ile Gly Glu Glu Lys
   305                 310                 315                 320
   Ser Phe Thr Glu Val Phe Val Lys Glu Pro Pro Val Thr Ile Thr Lys
                   325                 330                 335
   Leu Leu Asp Asp Tyr His Val Val Val Gly Glu Arg Val Glu Phe Glu
               340                 345                 350
   Val Glu Val Ser Val Glu Gly Ala His Val Asn Trp Met Phe Glu Asp
           355                 360                 365
   Gln Glu Leu Ser Arg Asp Thr His Lys Tyr Arg Phe Lys Lys Asp Gly
       370                 375                 380
   Leu Lys His Met Leu Ile Ile Gln Glu Ala Thr Leu Asp Asp Ile Gly
   385                 390                 395                 400
   Met Tyr Trp Cys Phe Thr Asn Gly Gly Arg Thr Lys Gly Glu Leu Glu
                   405                 410                 415
   Val Glu Glu Lys Glu Leu Glu Val Leu Gln Asn Ile Ala Asp Leu Thr
               420                 425                 430
   Val Lys Ala Glu Asp Gln Ala Met Phe Lys Cys Glu Val Ser Asp Glu
           435                 440                 445
   Lys Val Thr Gly Lys Trp Leu Lys Asp Gly Val Glu Val Leu Pro Ser
       450                 455                 460
   Ser Arg Ile Lys Leu Thr His Ile Gly Arg Ile His Arg Leu Thr Ile
   465                 470                 475                 480
   Asp Asp Val Lys Pro Glu Asp Ala Gly Asp Tyr Thr Phe Ile Pro Asp
                   485                 490                 495
   Gly Tyr Ala Leu Ser Leu Ser Ala Lys Leu Asn Phe Leu Glu Ile Lys
               500                 505                 510
   Ile Asp Tyr Val Pro Arg Gln Glu Pro Pro Lys Ile His Leu Asp Thr
   515 520 525
   Thr Gly Asn Met Val Ser Gln Asn Thr Ile Ile Val Val Ala Gly Asn
   530 535 540
   Lys Leu Arg Leu Asp Val Glu Ile Ser Gly Glu Pro Pro Pro Thr Val
   545 550 555 560
   Val Trp Ala Lys Gly Asp Thr Ala Ile Thr Ala Val Glu Gly Arg Val
   565 570 575
   Arg Thr Glu Ser Arg Lys Asp Leu Ser Cys Phe Val Ile Glu Gly Ala
   580 585 590
   Glu Arg Glu Asp Glu Gly Asn Tyr Thr Ile Ile Val Thr Asn Pro Ala
   595 600 605
   Gly Glu Asp Lys Ala His Leu Phe Val Lys Ile Val Asp Val Pro Asp
   610 615 620
   Cys Pro Glu Asn Val Lys Cys Thr Ser Val Gly Glu Asp Cys Ala Thr
   625 630 635 640
   Met Val Trp Asp Pro Pro Lys Phe Asp Gly Gly Ala Pro Val Thr Gly
   645 650 655
   Tyr Leu Met Glu Arg Lys Lys Lys Gly Ser Thr Arg Trp Thr Lys Leu
   660 665 670
   Asn Phe Asp Val Tyr Glu Gly Val Thr Tyr Glu Ala Lys Arg Met Ile
   675 680 685
   Glu Gly Val Leu Tyr Glu Met Arg Val Tyr Ala Val Asn Gly Ile Gly
   690 695 700
   Met Ser Gln Pro Ser Leu Asn Ser Lys Pro Phe Met Pro Ile Ala Ala
   705 710 715 720
   Thr Ser Glu Pro Leu Gly Leu Lys Val His Asp Val Thr Asp Thr Thr
   725 730 735
   Cys Thr Leu Lys Trp Leu Ala Pro Glu Lys Ile Gly Ala Gly Gly Leu
   740 745 750
   Asp Gly Tyr Val Ile Glu Tyr Cys Lys Glu Gly Asp Thr Glu Trp Val
   755 760 765
   Val Ala Asn Thr Glu Leu Val Glu Arg Gln Asn Tyr Thr Val Arg Asn
       770                 775                 780
   Leu Pro Thr Ala Glu Lys Ile Asn Phe Arg Val Val Ala Val Asn Ile
   785                 790                 795                 800
   Ala Gly Arg Ser Pro Pro Thr Gly Leu Ser Gln Pro Val Thr Ile Arg
                   805                 810                 815
   Glu Ile Val Glu Leu Pro Lys Ile Arg Leu Pro Arg Tyr Leu Arg Gln
               820                 825                 830
   Lys Tyr Ile Arg Arg Val Gly Asp Lys Ile Asn Leu Thr Ile Pro Phe
           835                 840                 845
   Gln Gly Lys Pro Arg Pro Lys Val Gln Trp Tyr Lys Asp Gly Glu Glu
       850                 855                 860
   Leu Asp Thr Arg Val Ala Ser Ile Arg Asn Ser Glu Val Asp Ser Ile
   865                 870                 875                 880
   Leu Phe Ile Arg Ser Ala Glu Arg Ser His Ser Gly Lys Tyr Glu Leu
                   885                 890                 895
   Val Leu Gln Ile Glu Asn Met Glu Ala Arg Ala Ile Leu Glu Ile Arg
               900                 905                 910
   Ile Val Glu Ala Pro Gly Pro Pro Glu Val Val Lys Val Thr Asp Val
           915                 920                 925
   Trp Gly Phe Asn Ala Ala Leu Glu Trp Lys Pro Pro Lys Asp Asp Gly
       930                 935                 940
   Asn Cys Glu Ile Thr Gly Tyr Thr Ile Gln Lys Ala Asp Lys Lys Thr
   945                 950                 955                 960
   Asn Glu Trp Phe Thr Ile Tyr Glu His Asn Arg Arg Thr Asn Cys Thr
                   965                 970                 975
   Ala Ser Asp Leu Ile Met Gly Asn Glu Tyr Met Phe Arg Val Phe Ser
               980                 985                 990
   Glu Asn Leu Val Gly Lys Ser Glu  Asp Phe Cys Leu Ser  Lys Asp Thr
           995                 1000                1005
   Ala Ile  Ile Pro Lys Ile Gly  Leu Glu Tyr Asn Pro  Pro Pro Phe
   1010                1015                1020
   Lys Glu  Lys Asp Met Gln Ser  Ala Pro Lys Phe Ile  Gln Pro Leu
       1025                1030                1035
   Leu Asp  Arg Ser Val Val Ala  Gly Tyr Ser Thr Thr  Ile Ser Ser
       1040                1045                1050
   Ala Val  Lys Thr Cys Pro Lys  Pro Lys Ile Arg Trp  Leu Arg Asn
       1055                1060                1065
   Lys Ile  Pro Leu Asp Glu Asn  Pro Arg Phe Leu Met  Gln Asn Asn
       1070                1075                1080
   Gln Gly  Val Leu Thr Leu Asn  Ile Arg Lys Pro Ser  Gln Tyr Asp
       1085                1090                1095
   Gly Gly  Lys Phe Thr Cys Lys  Ala Ile Asn Ser Leu  Gly Glu Asp
       1100                1105                1110
   Val Val  Glu Cys Thr Leu Leu  Val Arg Ala Leu Lys  Asp Lys Glu
       1115                1120                1125
   Asp Gly  Asp Glu Lys
       1130

<210>  122
<211>  8
<212>  PRT
<213>  Artificial Sequence

<220>
<223>  myosin-binding protein C, fast-type-like fragment

<400>  122

Ala Asp Val Trp Glu Ile Leu Lys
1               5

<210>  123
<211>  14
<212>  PRT
<213>  Artificial Sequence

<220>
<223>  myosin-binding protein C, fast-type-like fragment

<400>  123

Glu Leu Glu Val Leu Gln Asn Ile Ala Asp Leu Thr Val Lys
1               5                   10

<210>  124
<211>  8
<212>  PRT
<213>  Artificial Sequence

<220>
<223>  myosin-binding protein C, fast-type-like fragment

<400>  124

Glu Pro Pro Val Thr Ile Thr Lys
1               5


<210>  125
<211>  8
<212>  PRT
<213>  Artificial Sequence

<220>
<223>  myosin-binding protein C, fast-type-like fragment

<400>  125

Gly Glu Leu Glu Val Glu Glu Lys
1               5


<210>  126
<211>  7
<212>  PRT
<213>  Artificial Sequence

<220>
<223>  myosin-binding protein C, fast-type-like fragment

<400>  126

His Ser Asp Ala Phe Leu Lys
1               5


<210>  127
<211>  15
<212>  PRT
<213>  Artificial Sequence

<220>
<223>  myosin-binding protein C, fast-type-like fragment

<400>  127

Ile Ala Phe Glu Tyr Gly Ile Thr Asp Leu Arg Gly Leu Leu Lys
1               5                   10                  15


<210>  128
<211>  11
<212>  PRT
<213>  Artificial Sequence

<220>
<223>  myosin-binding protein C, fast-type-like fragment

<400>  128

Ile Gly Leu Glu Tyr Asn Pro Pro Pro Phe Lys
1               5                   10


<210>  129
<211>  14
<212>  PRT
<213>  Artificial Sequence

<220>
<223>  myosin-binding protein C, fast-type-like fragment

<400>  129

Ile Asn Leu Thr Ile Pro Phe Gln Gly Lys Pro Arg Pro Lys
1               5                   10


<210>  130
<211>  15
<212>  PRT
<213>  Artificial Sequence

<220>
<223>  myosin-binding protein C, fast-type-like fragment

<400>  130

Ile Val Leu Gln Val Glu Val Val Asp Pro Asn Ala Gln Val Lys
1               5                   10                  15


<210>  131
<211>  14
<212>  PRT
<213>  Artificial Sequence

<220>
<223>  myosin-binding protein C, fast-type-like fragment

<400>  131

Leu Asn Phe Asp Val Tyr Glu Gly Val Thr Tyr Glu Ala Lys
1               5                   10


<210>  132
<211>  7
<212>  PRT
<213>  Artificial Sequence

<220>
<223>  myosin-binding protein C, fast-type-like fragment

<400>  132

Leu Asn Phe Leu Glu Ile Lys
1               5

<210>  133
<211>  9
<212>  PRT
<213>  Artificial Sequence

<220>
<223>  myosin-binding protein C, fast-type-like fragment

<400>  133

Asn Ala Gln Pro Ser Glu Tyr Glu Lys
1               5


<210>  134
<211>  18
<212>  PRT
<213>  Artificial Sequence

<220>
<223>  myosin-binding protein C, fast-type-like fragment

<400>  134

Arg Thr Asp Ala Gly Glu Asp Glu Gly Ser Leu Asp Phe Ser Ala Leu
1               5                   10                  15


Leu Lys



<210>  135
<211>  8
<212>  PRT
<213>  Artificial Sequence

<220>
<223>  myosin-binding protein C, fast-type-like fragment

<400>  135

Ser Phe Thr Glu Val Phe Val Lys
1               5


<210>  136
<211>  13
<212>  PRT
<213>  Artificial Sequence

<220>
<223>  myosin-binding protein C, fast-type-like fragment

<400>  136

Val Asp Ser Thr Thr Leu Ser Arg Lys Pro Thr Ile Lys
1               5                   10


<210>  137
<211>  1551
<212>  DNA

<213>  Artificial Sequence

<220>
<223>  ATP synthase subunit beta, mitochondrial

<400>  137
atgttaggag ctgtgggacg ctgctgcacc ggggctctcc aggcacttaa gcctggggtc      60

cagcccctga aggcccttgt aggatcgcca tctgtccttg gacgcagaga ctactctgca     120

cctgctgctg ctgccgcctt cgcccatggc aggatcgtag cggtcatcgg tgccgtcgtc     180

gacgtccagt tcgatgaggg cctcccaccc atcctcaatg ccctggaagt tgctgggcgt     240

gagtccaggc tagttctgga ggtggcacag catcttggtg agaacactgt gcgtaccatt     300

gccatggatg gtactgaagg tcttgtccgt ggacagaagg ttgtggacac tggcgatccc     360

atcagaatcc cagtgggtcc cgagacccta ggcagaatca tgaatgtcat tggagagccc     420

attgatgaga gggggccaat ctccaccaag cagactgccg ccatccacgc tgaggcccca     480

gagttcactg acatgagtgt ggagcaggag atcctggtaa ctggcatcaa ggtggtagat     540

ctgctggctc cctatgccaa gggaggcaaa atcggtctgt tcggtggtgc tggtgtgggc     600

aagactgtgt tgatcatgga gctgatcaac aatgtggcca aggcccatgg tggttactct     660

gtgtttgccg gagtgggaga gcgtacccgc gagggaaatg acttgtacca cgagatgatt     720

gagtcgggtg tcatcaacct gaaggatgac acctccaagg tggcgctggt gtacggacaa     780

atgaacgagc ccccaggcgc ccgtgcccgt gtggctctga ctggtctgac cgtggcagag     840

tatttccgtg accaggaggg tcaggatgtg ctgctcttca tcgataacat cttccgtttc     900

acccaggctg gctccgaggt gtctgccctg ctgggtcgta tcccctccgc tgtgggttac     960

cagcccaccc tggccactga catgggtacc atgcaggaga gaatcaccac caccaagaag    1020

ggttccatca cctctgtgca ggccatctat gtgccggctg acgatttgac tgatcccgcc    1080

cctgccacca ccttcgctca cttggacgcc accactgtgc tgtcccgtgc aatcgctgag    1140

ctgggtatct accctgctgt ggatcccctg gattccacct cccgtatcat ggaccccaac    1200

attgtcggcg ctgagcacta tgatgttgct cgtggcgtgc agaagatcct ccaggactac    1260

aagtccctgc aggatatcat tgccatcctg ggtatggatg agttgtctga ggaggacaag    1320

ctgattgtct ctcgcgcccg caagatccag cgtttcctgt cccagccctt ccaggtggcg    1380

gaggtattca ccggtcatgc tggcaagctg gtgccactca aggagaccat cagtggcttc    1440

cagagcattc taaatggtga gtacgatgcc ctgcccgagc aggctttcta catggttgga    1500

cccatcgagg aggtggttga gaaggctgca cagatggcca aggatctcgc a             1551

<210>  138
<211>  517
<212>  PRT
<213>  Artificial Sequence

<220>
<223>  ATP synthase subunit beta, mitochondrial

<400>  138

Met Leu Gly Ala Val Gly Arg Cys Cys Thr Gly Ala Leu Gln Ala Leu
1               5                   10                  15

Lys Pro Gly Val Gln Pro Leu Lys Ala Leu Val Gly Ser Pro Ser Val
            20                  25                  30

Leu Gly Arg Arg Asp Tyr Ser Ala Pro Ala Ala Ala Ala Ala Phe Ala
        35                  40                  45

His Gly Arg Ile Val Ala Val Ile Gly Ala Val Val Asp Val Gln Phe
    50                  55                  60

Asp Glu Gly Leu Pro Pro Ile Leu Asn Ala Leu Glu Val Ala Gly Arg
65                  70                  75                  80

Glu Ser Arg Leu Val Leu Glu Val Ala Gln His Leu Gly Glu Asn Thr
                85                  90                  95

Val Arg Thr Ile Ala Met Asp Gly Thr Glu Gly Leu Val Arg Gly Gln
            100                 105                 110

Lys Val Val Asp Thr Gly Asp Pro Ile Arg Ile Pro Val Gly Pro Glu
        115                 120                 125

Thr Leu Gly Arg Ile Met Asn Val Ile Gly Glu Pro Ile Asp Glu Arg
    130                 135                 140

Gly Pro Ile Ser Thr Lys Gln Thr Ala Ala Ile His Ala Glu Ala Pro
145                 150                 155                 160

Glu Phe Thr Asp Met Ser Val Glu Gln Glu Ile Leu Val Thr Gly Ile
                165                 170                 175

Lys Val Val Asp Leu Leu Ala Pro Tyr Ala Lys Gly Gly Lys Ile Gly
            180                 185                 190

Leu Phe Gly Gly Ala Gly Val Gly Lys Thr Val Leu Ile Met Glu Leu
        195                 200                 205

Ile Asn Asn Val Ala Lys Ala His Gly Gly Tyr Ser Val Phe Ala Gly
    210                 215                 220

Val Gly Glu Arg Thr Arg Glu Gly Asn Asp Leu Tyr His Glu Met Ile

225                 230                 235                 240

Glu Ser Gly Val Ile Asn Leu Lys Asp Asp Thr Ser Lys Val Ala Leu
                245                 250                 255

Val Tyr Gly Gln Met Asn Glu Pro Pro Gly Ala Arg Ala Arg Val Ala
            260                 265                 270

Leu Thr Gly Leu Thr Val Ala Glu Tyr Phe Arg Asp Gln Glu Gly Gln
        275                 280                 285

Asp Val Leu Leu Phe Ile Asp Asn Ile Phe Arg Phe Thr Gln Ala Gly
    290                 295                 300

Ser Glu Val Ser Ala Leu Leu Gly Arg Ile Pro Ser Ala Val Gly Tyr
305                 310                 315                 320

Gln Pro Thr Leu Ala Thr Asp Met Gly Thr Met Gln Glu Arg Ile Thr
                325                 330                 335

Thr Thr Lys Lys Gly Ser Ile Thr Ser Val Gln Ala Ile Tyr Val Pro
            340                 345                 350

Ala Asp Asp Leu Thr Asp Pro Ala Pro Ala Thr Thr Phe Ala His Leu
        355                 360                 365

Asp Ala Thr Thr Val Leu Ser Arg Ala Ile Ala Glu Leu Gly Ile Tyr
    370                 375                 380

Pro Ala Val Asp Pro Leu Asp Ser Thr Ser Arg Ile Met Asp Pro Asn
385                 390                 395                 400

Ile Val Gly Ala Glu His Tyr Asp Val Ala Arg Gly Val Gln Lys Ile
                405                 410                 415

Leu Gln Asp Tyr Lys Ser Leu Gln Asp Ile Ile Ala Ile Leu Gly Met
            420                 425                 430

Asp Glu Leu Ser Glu Glu Asp Lys Leu Ile Val Ser Arg Ala Arg Lys
        435                 440                 445

Ile Gln Arg Phe Leu Ser Gln Pro Phe Gln Val Ala Glu Val Phe Thr
    450                 455                 460

Gly His Ala Gly Lys Leu Val Pro Leu Lys Glu Thr Ile Ser Gly Phe
465                 470                 475                 480

Gln Ser Ile Leu Asn Gly Glu Tyr Asp Ala Leu Pro Glu Gln Ala Phe
                485                 490                 495

Tyr Met Val Gly Pro Ile Glu Glu Val Val Glu Lys Ala Ala Gln Met
            500                 505                 510

Ala Lys Asp Leu Ala
        515

<210>  139
<211>  11
<212>  PRT
<213>  Artificial Sequence

<220>
<223>  ATP synthase subunit beta, mitochondrial fragment

<400>  139

Ile Gly Leu Phe Gly Gly Ala Gly Val Gly Lys
1               5                   10

<210>  140
<211>  21
<212>  PRT
<213>  Artificial Sequence

<220>
<223>  ATP synthase subunit beta, mitochondrial fragment

<400>  140

Ile Gln Arg Phe Leu Ser Gln Pro Phe Gln Val Ala Glu Val Phe Thr
1               5                   10                  15

Gly His Ala Gly Lys
            20

<210>  141
<211>  13
<212>  PRT
<213>  Artificial Sequence

<220>
<223>  ATP synthase subunit beta, mitochondrial fragment

<400>  141

Thr Val Leu Ile Met Glu Leu Ile Asn Asn Val Ala Lys
1               5                   10

<210>  142
<211>  10
<212>  PRT
<213>  Artificial Sequence

<220>
<223>  ATP synthase subunit beta, mitochondrial fragment

<400>  142

Val Val Asp Leu Leu Ala Pro Tyr Ala Lys
1               5                   10


<210>  143
<211>  996
<212>  DNA
<213>  Artificial Sequence

<220>
<223>  L-lactate dehydrogenase A chain-like

<400>  143
atgactacca aggagaagct gatcacccat gtgttggctg gtgagcctgt tggctcccgg      60

agcaaggtga cagttgttgg cgtcggcatg gttggcatgg cctccgcagt cagcgtcctg     120

ctcaaggacc tgtgcgatga gctgtgcctg attgacgtga tggaagataa actgaagggt     180

gaggtcatgg acctgcagca tggcagcctc ttctgcaaga ctcacaagat tgtgggcgac     240

aaggactaca gtacgactgc ccactccaag gtggtggtgg tcacagccgg tgctcgtcag     300

caagagggtg agagccgtct gaacctggtg cagcgtaacg tcaacatctt caaattcata     360

attccccaga tcgtcaagta cagccccaac gccatcctgc tggtcgtctc caatcctgtt     420

gacatcctaa cctacgtggc ttggaagctg agtggtttcc cccgtcaccg cgtcatcggt     480

tccggcacca acctggactc tggtcgtttc cgccacctga tgggcgagaa gctacacctt     540

cacccatcca gctgtcacgg ctggatcatt ggagaacacg gagactccag cgtgcccgta     600

tggagcggtg tgaatgttgc cggtgtttcc ctgaagggcc tgaacccaga catgggcaca     660

gacgcagaca aggaggactg gaagcacgtc cacaagatgg tggtcgacgg tgcctatgag     720

gtcatcaagc tgaagggtta cacctcctgg gctatcggca tgtccgtcgc tgacctggtt     780

gagagcatcc tgaagaacct ccacaaagtc caccctgtgt ccaccctggt caagggaatg     840

cacggtgtga aggaggaggt gtttctcagc gtgccctgcg tgctgggaaa cagcggtctg     900

accgacgtca tccacatgac tctgaagccc gaggaggaga agcagctgag caacagtgcc     960

gagaccctat ggggcgtaca gaaagagctc accttg                               996


<210>  144
<211>  332
<212>  PRT
<213>  Artificial Sequence

<220>
<223>  L-lactate dehydrogenase A chain-like

<400>  144

Met Thr Thr Lys Glu Lys Leu Ile Thr His Val Leu Ala Gly Glu Pro
1               5                   10                  15

Val Gly Ser Arg Ser Lys Val Thr Val Val Gly Val Gly Met Val Gly
            20                  25                  30

Met Ala Ser Ala Val Ser Val Leu Leu Lys Asp Leu Cys Asp Glu Leu
        35                  40                  45

Cys Leu Ile Asp Val Met Glu Asp Lys Leu Lys Gly Glu Val Met Asp
    50                  55                  60

Leu Gln His Gly Ser Leu Phe Cys Lys Thr His Lys Ile Val Gly Asp
65                  70                  75                  80

Lys Asp Tyr Ser Thr Thr Ala His Ser Lys Val Val Val Val Thr Ala
                85                  90                  95

Gly Ala Arg Gln Gln Glu Gly Glu Ser Arg Leu Asn Leu Val Gln Arg
            100                 105                 110

Asn Val Asn Ile Phe Lys Phe Ile Ile Pro Gln Ile Val Lys Tyr Ser
        115                 120                 125

Pro Asn Ala Ile Leu Leu Val Val Ser Asn Pro Val Asp Ile Leu Thr
    130                 135                 140

Tyr Val Ala Trp Lys Leu Ser Gly Phe Pro Arg His Arg Val Ile Gly
145                 150                 155                 160

Ser Gly Thr Asn Leu Asp Ser Gly Arg Phe Arg His Leu Met Gly Glu
                165                 170                 175

Lys Leu His Leu His Pro Ser Ser Cys His Gly Trp Ile Ile Gly Glu
            180                 185                 190

His Gly Asp Ser Ser Val Pro Val Trp Ser Gly Val Asn Val Ala Gly
        195                 200                 205

Val Ser Leu Lys Gly Leu Asn Pro Asp Met Gly Thr Asp Ala Asp Lys
    210                 215                 220

Glu Asp Trp Lys His Val His Lys Met Val Val Asp Gly Ala Tyr Glu
225                 230                 235                 240

Val Ile Lys Leu Lys Gly Tyr Thr Ser Trp Ala Ile Gly Met Ser Val
                245                 250                 255

Ala Asp Leu Val Glu Ser Ile Leu Lys Asn Leu His Lys Val His Pro
            260                 265                 270

Val Ser Thr Leu Val Lys Gly Met His Gly Val Lys Glu Glu Val Phe
        275                 280                 285

Leu Ser Val Pro Cys Val Leu Gly Asn Ser Gly Leu Thr Asp Val Ile
    290                 295                 300

His Met Thr Leu Lys Pro Glu Glu Glu Lys Gln Leu Ser Asn Ser Ala
305                 310                 315                 320

Glu Thr Leu Trp Gly Val Gln Lys Glu Leu Thr Leu
                325                 330

<210>  145
<211>  8
<212>  PRT
<213>  Artificial Sequence

<220>
<223>  L-lactate dehydrogenase A chain-like fragment

<400>  145

Phe Ile Ile Pro Gln Ile Val Lys
1               5

<210>  146
<211>  16
<212>  PRT
<213>  Artificial Sequence

<220>
<223>  L-lactate dehydrogenase A chain-like fragment

<400>  146

Leu Ile Thr His Val Leu Ala Gly Glu Pro Val Gly Ser Arg Ser Lys
1               5                   10                  15

<210>  147
<211>  14
<212>  PRT
<213>  Artificial Sequence

<220>
<223>  L-lactate dehydrogenase A chain-like fragment

<400>  147

Thr His Val Leu Ala Gly Glu Pro Val Gly Ser Arg Ser Lys
1               5                   10

<210>  148
<211>  9
<212>  PRT
<213>  Artificial Sequence

<220>
<223>  L-lactate dehydrogenase A chain-like fragment

<400>  148

Val His Pro Val Ser Thr Leu Val Lys
1               5


<210>  149
<211>  28
<212>  PRT
<213>  Artificial Sequence

<220>
<223>  L-lactate dehydrogenase A chain-like fragment

<400>  149

Val Val Val Val Thr Ala Gly Ala Arg Gln Gln Glu Gly Glu Ser Arg
1               5                   10                  15


Leu Asn Leu Val Gln Arg Asn Val Asn Ile Phe Lys
            20                  25

## Description

### TECHNICAL FIELD

The present invention relates to a novel antigen of an allergy to fish. The present invention also relates to a kit, a composition, and a method for diagnosing allergy to fish. The present invention also relates to a pharmaceutical composition comprising the antigen and fish or fish eggs in which the antigen is eliminated or reduced, a processed product of the fish or the fish eggs or fish which lay the fish eggs or have hatched from the eggs. The present invention further relates to a tester for determining the presence or absence of a fish antigen in an object of interest.

### BACKGROUND ART

In blood and tissues of allergic patients, IgE antibodies specific to particular antigens are produced. Physiological consequences caused by interaction between such IgE antibodies and such particular antigens elicit allergic reactions.

In the process of production of conventional allergy testing agents, antigen reagents are commonly prepared simply by grinding a candidate allergenic food, food material or the like (Patent Literature 1). Therefore, numerous proteins were contained in a reagent and there the content of each protein was very little. For this reason, the only case where conventional allergy tests have permitted detection of a positive allergic reaction is when in a conventional antigen reagent containing many proteins, a particular antigen protein which causes the allergic reaction (allergen component) is present in an amount exceeding a threshold that allows determination of a positive reaction for binding to an IgE antibody. However, no determination of a positive reaction was possible and diagnosis efficiency was not sufficiently high when using a conventional allergy testing agent in patients possessing an IgE antibody binding to an allergen component present in small amounts in an allergen such as food, food material or the like.

The severity and symptoms of an allergic reaction do not necessarily correlate with the content of an allergen component. Even when a patient's IgE antibody reacts with an allergen component present in trace amounts in a candidate allergic food and food material, the allergic reaction may develop allergic symptoms or may affect the severity of those symptoms.

An attempt to increase the diagnostic efficiency is being made by examining IgE antibodies to each protein composing food and food material to distinguish sensitization that directly contributes to a diagnosis from sensitization based on cross-antigenicity by a pan-allergen or the like. The allergens set forth in Table below are currently known as fish allergens (Non Patent Literature 1).

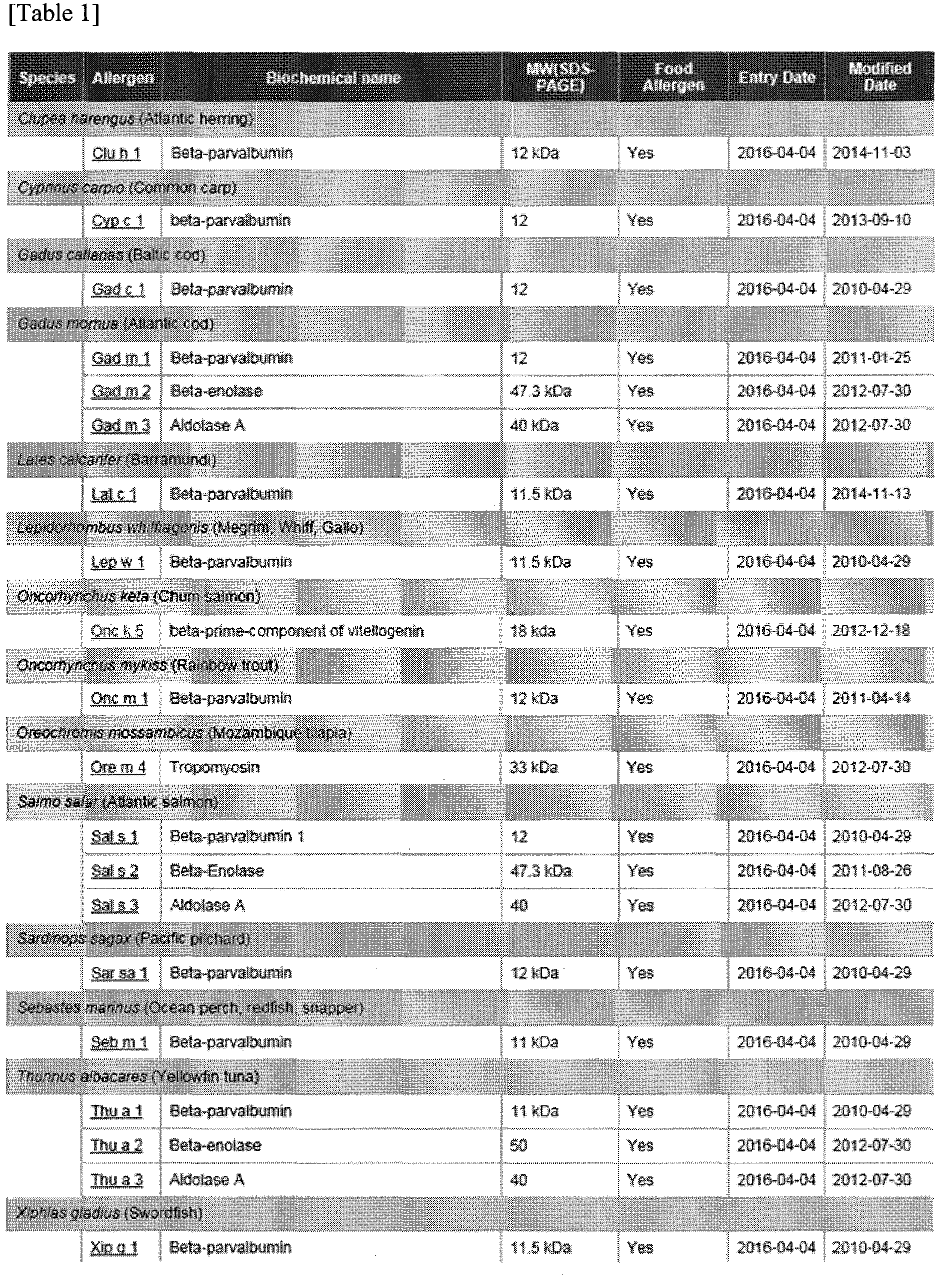

However, while it is necessary to exhaustively identify allergen components in candidate allergic foods and food materials in order to enhance the reliability of allergy tests, the patient detection rate by the measurement of such allergen components is far from sufficient. Identification of novel allergens in fish is very important not only for increasing the precision of diagnosis, but also for determining targets of therapeutic agents and low allergenic foods and low allergenic food materials.

Meanwhile, in the field of protein separation and purification, various efforts have conventionally been made to develop methods for separating and purifying a protein or nucleic acid of interest from cell extracts or the like. Such methods may well be exemplified by dialysis based on salt concentration, and centrifugal separation.

Other efforts have been made to develop many purification methods based on electric charges of protein or nucleic acid residues or on the difference in molecular weight. Electric charge-based purification methods can be exemplified by column chromatography using ion exchange resins, and isoelectric focusing. Purifications based on molecular weight difference can be exemplified by centrifugal separation, molecular-sieve column chromatography, and SDS-PAGE (sodium dodecyl sulfate-polyacrylamide gel electrophoresis).

In recent years, a method for separating and purifying many different proteins from a small amount of sample has been used, which is more specifically a two-dimensional electrophoresis consisting of isoelectric focusing in the first dimension, followed by SDS-PAGE in the second dimension. The present applicant has conventionally developed some 2D electrophoresis methods with high separation ability (Patent Literature 2-5).

### CITATION LIST

### PATENT LITERATURE

PTL1: Japanese Patent Application Publication No. JP 2002-286716
PTL2: Japanese Patent Application Publication No. JP 2011-33544
PTL3: Japanese Patent Application Publication No. JP 2011-33546
PTL4: Japanese Patent Application Publication No. JP 2011-33547
PTL5: Japanese Patent Application Publication No. JP 2011-33548

### NON-PATENT LITERATURE

NPTL1: Allergen Nomenclature, WHO/IUIS Allergen Nomenclature Sub-Committee, [searched on June 9, 2016], intemet<URL: http://www.allergen.org/search.php?allergenname=&allergensource=&TaxSource=Animalia +Chordata&TaxOrder=&foodallerg=1&bioname=>

### SUMMARY OF INVENTION

### TECHNICAL PROBLEM

The present invention provides novel antigens of an allergy to fish. The present invention also provides methods and kits for diagnosing allergy to fish. The present invention also provides pharmaceutical compositions comprising such an antigen and fish or fish eggs in which such an antigen is eliminated or reduced, a processed product of the fish or the fish eggs or fish which lay the fish eggs or have hatched from the eggs.

### SOLUTION TO PROBLEM

In order to solve the aforementioned problems, the present inventors had made intensive studies to identify causative antigens of an allergy to fish. As a result, the inventors succeeded in identifying novel antigens to which an IgE antibody in the serum of a fish-allergic patient specifically binds. The present invention has been completed based on this finding.

Thus, in one embodiment, the present invention can be as defined below.
[1] A kit for diagnosing a fish allergy, the kit comprising, as an antigen, at least one protein defined in any one of the following (1) to (9):
   (1)
      (1A) Alpha-actinin-3 or a variant thereof, which is a protein defined below in any of (1A-a) to (1A-e) which is an antigen for the fish allergy:
         (1A-a) a protein comprising an amino acid sequence with deletion, substitution, insertion or addition of one or several amino acids in SEQ ID NO: 2;
         (1A-b) a protein comprising an amino acid sequence having at least 70% identity to the amino acid sequence of SEQ ID NO: 2;
         (1A-c) a protein comprising an amino acid sequence encoded by a nucleotide sequence with deletion, substitution, insertion or addition of one or several nucleotides in SEQ ID NO: 1;
         (1A-d) a protein comprising an amino acid sequence encoded by a nucleotide sequence having at least 70% identity to the nucleotide sequence of SEQ ID NO: 1; or
         (1A-e) a protein comprising an amino acid sequence encoded by a nucleic acid that hybridizes under stringent conditions with a nucleic acid having a nucleotide sequence complementary to the nucleotide sequence of SEQ ID NO: 1; or
      (1B) a protein comprising an amino acid sequence of SEQ ID NO: 2 or 3;
   (2)
      (2A) EEF1A2 binding protein-like or a variant thereof, which is a protein defined below in any of (2A-a) to (2A-e) which is an antigen for the fish allergy:
         (2A-a) a protein comprising an amino acid sequence with deletion, substitution, insertion or addition of one or several amino acids in SEQ ID NO: 5;
         (2A-b) a protein comprising an amino acid sequence having at least 70% identity to the amino acid sequence of SEQ ID NO: 5;
         (2A-c) a protein comprising an amino acid sequence encoded by a nucleotide sequence with deletion, substitution, insertion or addition of one or several nucleotides in SEQ ID NO: 4;
         (2A-d) a protein comprising an amino acid sequence encoded by a nucleotide sequence having at least 70% identity to the nucleotide sequence of SEQ ID NO: 4; or
         (2A-e) a protein comprising an amino acid sequence encoded by a nucleic acid that hybridizes under stringent conditions with a nucleic acid having a nucleotide sequence complementary to the nucleotide sequence of SEQ ID NO: 4; or
      (2B) a protein comprising at least one amino acid sequence selected from the group consisting of SEQ ID NOs: 5-8;
   (3)
      (3A) Alpha-1,4-glucan phosphorylase or a variant thereof, which is a protein defined below in any of (3A-a) to (3A-e) which is an antigen for the fish allergy:
         (3A-a) a protein comprising an amino acid sequence with deletion, substitution, insertion or addition of one or several amino acids in SEQ ID NO: 10;
         (3A-b) a protein comprising an amino acid sequence having at least 70% identity to the amino acid sequence of SEQ ID NO: 10;
         (3A-c) a protein comprising an amino acid sequence encoded by a nucleotide sequence with deletion, substitution, insertion or addition of one or several nucleotides in SEQ ID NO: 9;
         (3A-d) a protein comprising an amino acid sequence encoded by a nucleotide sequence having at least 70% identity to the nucleotide sequence of SEQ ID NO: 9; or
         (3A-e) a protein comprising an amino acid sequence encoded by a nucleic acid that hybridizes under stringent conditions with a nucleic acid having a nucleotide sequence complementary to the nucleotide sequence of SEQ ID NO: 9; or
      (3B) a protein comprising at least one amino acid sequence selected from the group consisting of SEQ ID NOs: 10-16;
   (4)
      (4A) Elongation factor 2 or a variant thereof, which is a protein defined below in any of (4A-a) to (4A-e) which is an antigen for the fish allergy:
         (4A-a) a protein comprising an amino acid sequence with deletion, substitution, insertion or addition of one or several amino acids in SEQ ID NO: 19;
         (4A-b) a protein comprising an amino acid sequence having at least 70% identity to the amino acid sequence of SEQ ID NO: 19;
         (4A-c) a protein comprising an amino acid sequence encoded by a nucleotide sequence with deletion, substitution, insertion or addition of one or several nucleotides in SEQ ID NO: 18;
         (4A-d) a protein comprising an amino acid sequence encoded by a nucleotide sequence having at least 70% identity to the nucleotide sequence of SEQ ID NO: 18; or
         (4A-e) a protein comprising an amino acid sequence encoded by a nucleic acid that hybridizes under stringent conditions with a nucleic acid having a nucleotide sequence complementary to the nucleotide sequence of SEQ ID NO: 18; or
      (4B) a protein comprising at least one amino acid sequence selected from the group consisting of SEQ ID NOs: 19-24;
   (5)
      (5A) Heat shock cognate 70 kDa protein or a variant thereof, which is a protein defined below in any of (5A-a) to (5A-e) which is an antigen for the fish allergy:
         (5A-a) a protein comprising an amino acid sequence with deletion, substitution, insertion or addition of one or several amino acids in SEQ ID NO: 26;
         (5A-b) a protein comprising an amino acid sequence having at least 70% identity to the amino acid sequence of SEQ ID NO: 26;
         (5A-c) a protein comprising an amino acid sequence encoded by a nucleotide sequence with deletion, substitution, insertion or addition of one or several nucleotides in SEQ ID NO: 25;
         (5A-d) a protein comprising an amino acid sequence encoded by a nucleotide sequence having at least 70% identity to the nucleotide sequence of SEQ ID NO: 25; or
         (5A-e) a protein comprising an amino acid sequence encoded by a nucleic acid that hybridizes under stringent conditions with a nucleic acid having a nucleotide sequence complementary to the nucleotide sequence of SEQ ID NO: 25; or
      (5B) a protein comprising at least one amino acid sequence selected from the group consisting of SEQ ID NOs: 26-31;
   (6)
      (6A) Serotransferrin or a variant thereof, which is a protein defined below in any of (6A-a) to (6A-e) which is an antigen for the fish allergy:
         (6A-a) a protein comprising an amino acid sequence with deletion, substitution, insertion or addition of one or several amino acids in SEQ ID NO: 33;
         (6A-b) a protein comprising an amino acid sequence having at least 70% identity to the amino acid sequence of SEQ ID NO: 33;
         (6A-c) a protein comprising an amino acid sequence encoded by a nucleotide sequence with deletion, substitution, insertion or addition of one or several nucleotides in SEQ ID NO: 32;
         (6A-d) a protein comprising an amino acid sequence encoded by a nucleotide sequence having at least 70% identity to the nucleotide sequence of SEQ ID NO: 32; or
         (6A-e) a protein comprising an amino acid sequence encoded by a nucleic acid that hybridizes under stringent conditions with a nucleic acid having a nucleotide sequence complementary to the nucleotide sequence of SEQ ID NO: 32; or
      (6B) a protein comprising at least one amino acid sequence selected from the group consisting of SEQ ID NOs: 33-41;
   (7)
      (7A) Myosin binding protein H-like or a variant thereof, which is a protein defined below in any of (7A-a) to (7A-e) which is an antigen for the fish allergy:
         (7A-a) a protein comprising an amino acid sequence with deletion, substitution, insertion or addition of one or several amino acids in SEQ ID NO: 43;
         (7A-b) a protein comprising an amino acid sequence having at least 70% identity to the amino acid sequence of SEQ ID NO: 43;
         (7A-c) a protein comprising an amino acid sequence encoded by a nucleotide sequence with deletion, substitution, insertion or addition of one or several nucleotides in SEQ ID NO: 42;
         (7A-d) a protein comprising an amino acid sequence encoded by a nucleotide sequence having at least 70% identity to the nucleotide sequence of SEQ ID NO: 42; or
         (7A-e) a protein comprising an amino acid sequence encoded by a nucleic acid that hybridizes under stringent conditions with a nucleic acid having a nucleotide sequence complementary to the nucleotide sequence of SEQ ID NO: 42; or
      (7B) a protein comprising at least one amino acid sequence selected from the group consisting of SEQ ID NOs: 43-54,
   (8)
      (8A) Desmin (Fragment) or a variant thereof, which is a protein defined below in any of (8A-a) to (8A-e) which is an antigen for the fish allergy:
         (8A-a) a protein comprising an amino acid sequence with deletion, substitution, insertion or addition of one or several amino acids in SEQ ID NO: 56;
         (8A-b) a protein comprising an amino acid sequence having at least 70% identity to the amino acid sequence of SEQ ID NO: 56;
         (8A-c) a protein comprising an amino acid sequence encoded by a nucleotide sequence with deletion, substitution, insertion or addition of one or several nucleotides in SEQ ID NO: 55;
         (8A-d) a protein comprising an amino acid sequence encoded by a nucleotide sequence having at least 70% identity to the nucleotide sequence of SEQ ID NO: 55; or
         (8A-e) a protein comprising an amino acid sequence encoded by a nucleic acid that hybridizes under stringent conditions with a nucleic acid having a nucleotide sequence complementary to the nucleotide sequence of SEQ ID NO: 55; or
      (8B) a protein comprising at least one amino acid sequence selected from the group consisting of SEQ ID NOs: 56-59;
   (9)
      (9A) Capping protein (Actin filament) muscle Z-line beta or a variant thereof, which is a protein defined below in any of (9A-a) to (9A-e) which is an antigen for the fish allergy:
         (9A-a) a protein comprising an amino acid sequence with deletion, substitution, insertion or addition of one or several amino acids in SEQ ID NO: 61;
         (9A-b) a protein comprising an amino acid sequence having at least 70% identity to the amino acid sequence of SEQ ID NO: 61;
         (9A-c) a protein comprising an amino acid sequence encoded by a nucleotide sequence with deletion, substitution, insertion or addition of one or several nucleotides in SEQ ID NO: 60;
         (9A-d) a protein comprising an amino acid sequence encoded by a nucleotide sequence having at least 70% identity to the nucleotide sequence of SEQ ID NO: 60; or
         (9A-e) a protein comprising an amino acid sequence encoded by a nucleic acid that hybridizes under stringent conditions with a nucleic acid having a nucleotide sequence complementary to the nucleotide sequence of SEQ ID NO: 60; or
      (9B) a protein comprising at least one amino acid sequence selected from the group consisting of SEQ ID NOs: 61-68.
[2] A composition for diagnosing a fish allergy, the composition comprising, as an antigen, at least one of proteins as defined above in any of (1) to (9) of [1].
[3] A method for providing an indicator for diagnosing a fish allergy in a subject, the method comprising the steps of:
   (i) contacting a sample obtained from the subject with an antigen, wherein the sample is a solution comprising an IgE antibody;
   (ii) detecting binding between an IgE antibody present in the sample from the subject and the antigen; and
   (iii) when the binding between the IgE antibody in the subject and the antigen is detected, an indicator of the fact that the subject has a fish allergy is provided;
   wherein the antigen is at least one of proteins as defined above in any of (1) to (9) of [1].
[4] A pharmaceutical composition comprising at least one of proteins as defined above in any of (1) to (9) of [1].
[5] The pharmaceutical composition as set forth in [4] for the treatment of a fish allergy.
[6] A fish or a processed product of fish, in which an antigen is eliminated or reduced, wherein the antigen is at least one of proteins as defined above in any of (1) to (9) of [1].
[7] A tester for determining the presence or absence of a fish antigen in an object of interest, the tester comprising an antibody that binds to at least one of proteins as defined above in any of (1) to (9) of [1].
[8] A tester for determining the presence or absence of an antigen causing an allergy to fish in an object of interest, the tester comprising a primer having a nucleotide sequence complementary to at least one part of one of the nucleotide sequences set forth in SEQ ID NO: 1, 4, 9, 18, 25, 32, 42, 55, or 60.
[9] A kit for diagnosing a fish allergy, the kit comprising, as an antigen, at least one protein defined in any one of the following (10) to (14):
   (10)
      (10A) Myosin heavy chain, fast skeletal muscle-like or a variant thereof, which is a protein defined below in any of (10A-a) to (10A-e) which is an antigen for the fish allergy:
         (10A-a) a protein comprising an amino acid sequence with deletion, substitution, insertion or addition of one or several amino acids in SEQ ID NO: 70;
         (10A-b) a protein comprising an amino acid sequence having at least 70% identity to the amino acid sequence of SEQ ID NO: 70;
         (10A-c) a protein comprising an amino acid sequence encoded by a nucleotide sequence with deletion, substitution, insertion or addition of one or several nucleotides in SEQ ID NO: 69;
         (10A-d) a protein comprising an amino acid sequence encoded by a nucleotide sequence having at least 70% identity to the nucleotide sequence of SEQ ID NO: 69; or
         (10A-e) a protein comprising an amino acid sequence encoded by a nucleic acid that hybridizes under stringent conditions with a nucleic acid having a nucleotide sequence complementary to the nucleotide sequence of SEQ ID NO: 69; or
      (10B) a protein comprising at least one amino acid sequence selected from the group consisting of SEQ ID NOs: 70-108;
   (11)
      (11A) Glycogen phosphorylase, muscle form-like or a variant thereof, which is a protein defined below in any of (11A-a) to (11A-e) which is an antigen for the fish allergy:
         (11A-a) a protein comprising an amino acid sequence with deletion, substitution, insertion or addition of one or several amino acids in SEQ ID NO: 110;
         (11A-b) a protein comprising an amino acid sequence having at least 70% identity to the amino acid sequence of SEQ ID NO: 110;
         (11A-c) a protein comprising an amino acid sequence encoded by a nucleotide sequence with deletion, substitution, insertion or addition of one or several nucleotides in SEQ ID NO: 109;
         (11A-d) a protein comprising an amino acid sequence encoded by a nucleotide sequence having at least 70% identity to the nucleotide sequence of SEQ ID NO: 109; or
         (11A-e) a protein comprising an amino acid sequence encoded by a nucleic acid that hybridizes under stringent conditions with a nucleic acid having a nucleotide sequence complementary to the nucleotide sequence of SEQ ID NO: 109; or
      (11B) a protein comprising at least one amino acid sequence selected from the group consisting of SEQ ID NOs: 110-119;
   (12)
      (12A) Myosin-binding protein C, fast-type-like or a variant thereof, which is a protein defined below in any of (12A-a) to (12A-e) which is an antigen for the fish allergy:
         (12A-a) a protein comprising an amino acid sequence with deletion, substitution, insertion or addition of one or several amino acids in SEQ ID NO: 121;
         (12A-b) a protein comprising an amino acid sequence having at least 70% identity to the amino acid sequence of SEQ ID NO: 121;
         (12A-c) a protein comprising an amino acid sequence encoded by a nucleotide sequence with deletion, substitution, insertion or addition of one or several nucleotides in SEQ ID NO: 120;
         (12A-d) a protein comprising an amino acid sequence encoded by a nucleotide sequence having at least 70% identity to the nucleotide sequence of SEQ ID NO: 120; or
         (12A-e) a protein comprising an amino acid sequence encoded by a nucleic acid that hybridizes under stringent conditions with a nucleic acid having a nucleotide sequence complementary to the nucleotide sequence of SEQ ID NO: 120; or
      (12B) a protein comprising at least one amino acid sequence selected from the group consisting of SEQ ID NOs: 121-136;
   (13)
      (13A) ATP synthase subunit beta, mitochondrial or a variant thereof, which is a protein defined below in any of (13A-a) to (13A-e) which is an antigen for the fish allergy:
         (13A-a) a protein comprising an amino acid sequence with deletion, substitution, insertion or addition of one or several amino acids in SEQ ID NO: 138;
         (13A-b) a protein comprising an amino acid sequence having at least 70% identity to the amino acid sequence of SEQ ID NO: 138;
         (13A-c) a protein comprising an amino acid sequence encoded by a nucleotide sequence with deletion, substitution, insertion or addition of one or several nucleotides in SEQ ID NO: 137;
         (13A-d) a protein comprising an amino acid sequence encoded by a nucleotide sequence having at least 70% identity to the nucleotide sequence of SEQ ID NO: 137; or
         (13A-e) a protein comprising an amino acid sequence encoded by a nucleic acid that hybridizes under stringent conditions with a nucleic acid having a nucleotide sequence complementary to the nucleotide sequence of SEQ ID NO: 137; or
      (13B) a protein comprising at least one amino acid sequence selected from the group consisting of SEQ ID NOs: 138-142;
   (14)
      (14A) L-lactate dehydrogenase A chain-like or a variant thereof, which is a protein defined below in any of (14A-a) to (14A-e) which is an antigen for the fish allergy:
         (14A-a) a protein comprising an amino acid sequence with deletion, substitution, insertion or addition of one or several amino acids in SEQ ID NO: 144;
         (14A-b) a protein comprising an amino acid sequence having at least 70% identity to the amino acid sequence of SEQ ID NO: 144;
         (14A-c) a protein comprising an amino acid sequence encoded by a nucleotide sequence with deletion, substitution, insertion or addition of one or several nucleotides in SEQ ID NO: 143;
         (14A-d) a protein comprising an amino acid sequence encoded by a nucleotide sequence having at least 70% identity to the nucleotide sequence of SEQ ID NO: 143; or
         (14A-e) a protein comprising an amino acid sequence encoded by a nucleic acid that hybridizes under stringent conditions with a nucleic acid having a nucleotide sequence complementary to the nucleotide sequence of SEQ ID NO: 143; or
      (14B) a protein comprising at least one amino acid sequence selected from the group consisting of SEQ ID NOs: 144-149.
[10] A composition for diagnosing a fish allergy, the composition comprising, as an antigen, at least one of proteins as defined above in any of (10) to (14) of [9].
[11] A method for providing an indicator for diagnosing a fish allergy in a subject, the method comprising the steps of:
   (i) contacting a sample obtained from the subject with an antigen, wherein the sample is a solution comprising an IgE antibody;
   (ii) detecting binding between an IgE antibody present in the sample from the subject and the antigen; and
   (iii) when the binding between the IgE antibody in the subject and the antigen is detected, an indicator of the fact that the subject has a fish allergy is provided;
   wherein the antigen is at least one of proteins as defined above in any of (10) to (14) of [9].
[12] A pharmaceutical composition comprising at least one of proteins as defined above in any of (10) to (14) of [9].
[13] The pharmaceutical composition as set forth in [12] for the treatment of a fish allergy.
[14] A fish or a processed product of fish, in which an antigen is eliminated or reduced, wherein the antigen is at least one of proteins as defined above in any of (10) to (14) of [9].
[15] A tester for determining the presence or absence of a fish antigen in an object of interest, the tester comprising an antibody that binds to at least one of proteins as defined above in any of (10) to (14) of [9].
[16] A tester for determining the presence or absence of an antigen causing an allergy to fish in an object of interest, the tester comprising a primer having a nucleotide sequence complementary to at least one part of one of the nucleotide sequences set forth in SEQ ID NO: 69, 109, 120, 137, or 143.
[17] A fish-derived antigen which is at least one of proteins as defined above in any of (1) to (9) of [1] and causes an allergy to fish.
[18] A fish-derived antigen which is at least one of proteins as defined above in any of (10) to (14) of [9] and causes an allergy to fish.

### ADVANTAGEOUS EFFECTS OF INVENTION

The present invention can provide novel antigens of an allergy to fish. Since the novel antigens (allergen components) that trigger a fish allergy were identified in the present invention, it is possible to provide highly sensitive methods and kits for diagnosing an allergy to fish, pharmaceutical compositions comprising such an antigen, fish or fish eggs in which the antigen is eliminated or reduced, a processed product of the fish or the fish eggs or fish which lay the fish eggs or have hatched from the eggs, and testers for determining the presence or absence of a fish antigen in an object of interest.

### BRIEF DESCRIPTION OF DRAWINGS

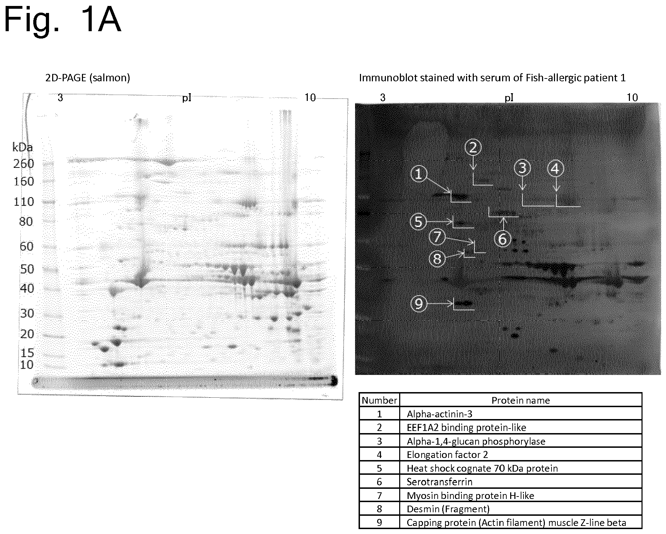

Fig. 1A is a photograph of a gel showing a protein electrophoretic pattern in two-dimensional electrophoresis of proteins contained in an extract of flesh of salmon (left panel) and a photograph of an immunoblot obtained from the two-dimensional electrophoretic pattern by using a serum of Fish-allergic patient 1 (right panel). The bands at the left of the photograph of the gel are bands of molecular weight markers and the numbers on the left side of the photograph of the gel are molecular weights of respective molecular weight markers (KDa). The numbers on the upper side of the photograph indicate isoelectric points.
Fig. 1B is a photograph of an immunoblot obtained from the two-dimensional electrophoretic pattern of the proteins contained in the extract of the flesh of salmon by using a serum of Fish-allergic patient 2 (left panel) and a photograph of an immunoblot of the proteins stained with serum of Fish-allergic patient 3 (right panel). The numbers on the upper side of each photograph indicate isoelectric points.
Fig. 2 is photographs of immunoblots obtained from two-dimensional electrophoretic patterns of proteins contained in extracts of the flesh of 10 species of fish stained with serum of Fish-allergic patient 1.
Fig. 3 is photographs of immunoblots obtained from two-dimensional electrophoretic patterns of proteins contained in extracts of the flesh of 10 species of fish stained with serum of a subject who does not exhibit fish allergic symptoms (Non-fish-allergic subject).
Fig. 4 is photographs of immunoblots obtained from two-dimensional electrophoretic patterns of proteins contained in extracts of the fish of 6 species of fish by using a serum of Fish-allergic patient 4.

### DESCRIPTION OF EMBODIMENTS

The present invention will be described in detail below, but the present invention is not limited to them.

Unless otherwise defined herein, all scientific and technical terms used in relation to the present invention shall have meanings commonly understood by those skilled in the art.

As referred to herein, the "allergy" refers to the state in which, when a certain antigen reenters a living organism sensitized to said antigen, the living organism shows a hypersensitive reaction detrimental to the living organism. In blood and tissues of individuals with many food-allergic diseases, IgE antibodies specific to antigens are produced. IgE antibodies bind to mast cells or basophils. When an antigen specific to such an IgE antibody reenters the body of a patient with an allergic disease, said antigen combines with the IgE antibody bound to mast cells or basophils, and the IgE antibody crosslinks said antigen on the cell surface, resulting in physiological effects of IgE antibody-antigen interaction. Examples of such physiological effects include release of histamine, serotonin, heparin, eosinophil chemotactic factors, leucotrienes, or the like. These released substances provoke an allergic reaction resulting from the combination of an IgE antibody with particular antigens. Such allergic reactions caused by particular antigens occur through the aforementioned pathway.

As used herein, the term fish means teleost and elasmobranch among fishes, preferably teleost, more preferably those belonging to Salmoniformes, Perciformes, Anguilliformes, Gadiformes, and Pleuronectiformes, further preferably those belonging to Salmonidae, Carangidae, Congridae, Sparidae, Scombridae, Gadidae, Anguillidae, and Paralichthyidae, and even preferably salmon, horse mackerel, sea eel, black porgy, mackerel, sea bream, cod, young yellowtail, eel, and flatfish. The fish may be edible.

As used herein, the term fish eggs means eggs of fish. The fish eggs may be edible.

As referred to herein, the "allergy to fish" refers to the state in which an individual has an allergic reaction caused by proteins, etc. present in fish which act as an antigen. The allergy to fish can produce an allergic reaction upon contact with, or consumption of, an antigen present in fish. In general, allergic reactions caused by consumption of foods are particularly referred to as "food allergies". The allergy to fish may be a food allergy.

As referred to herein, the "antigen" refers to a substance that provokes an allergic reaction, and is also referred to as an "allergen component". The antigen is preferably a protein.

As referred to herein, the "protein" refers to a molecule having a structure in which naturally occurring amino acids are joined together by peptide bond. The number of amino acids present in a protein is not particularly limited, but proteins having about 2 to 50 amino acids joined together by peptide bond are in some cases called "peptides". In the case where amino acids can form different enantiomers, the amino acids are understood to form an L-enantiomer, unless otherwise indicated. The amino acid sequences of proteins or peptides as used herein are represented by one-letter symbols of amino acids in accordance with standard usage and the notational convention commonly used in the art. The leftward direction represents the amino-terminal direction, and the rightward direction represents the carboxy-terminal direction.

### Identification of antigens

Proteins contained in fish were analyzed by the aforementioned technique to identify antigens of an allergy to fish. To be specific, proteins were extracted from the flesh of fish and subjected to two-dimensional electrophoresis under the conditions described below.

The electrophoresis in the first dimension was isoelectric focusing, which was performed using isoelectric focusing gels with a gel-strip length of 5 to 10 cm and a gel pH range of 3 to 10. The pH gradient of the gels in the direction of electrophoresis was as follows: when the total gel-strip length is taken as 1, the gel-strip length up to pH 5 is taken as "a", the gel-strip length from pH 5 to 7 is taken as "b", and the gel-strip length above pH 7 is taken as "c", "a" is in the range of 0.15 to 0.3, "b" is in the range of 0.4 to 0.7, and "c" is in the range of 0.15 to 0.3. More specifically, the isoelectric focusing was performed using the IPG gels, Immobiline Drystrip (pH3-10NL), produced by GE Healthcare Bio-Sciences Corporation (hereinafter abbreviated as "GE"). The electrophoresis system used was IPGphor produced by GE. The maximum current of the electrophoresis system was limited to 75 µA per gel strip. The voltage program adopted to perform the first-dimensional isoelectric focusing was as follows: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

The electrophoresis in the second dimension was SDS-PAGE, which was performed using polyacrylamide gels whose gel concentration at the distal end in the direction of electrophoresis was set to 3 to 6% and whose gel concentration at the proximal end was set to a higher value than that at the distal end. More specifically, the SDS-PAGE was performed using NuPAGE 4-12% Bris-Tris Gels (IPG well, Mini, 1 mm) produced by Life Technologies. The electrophoresis system used was XCell SureLock Mini-Cell produced by Life Technologies. The electrophoresis was run at a constant voltage of 200 V for about 45 minutes using an electrophoresis buffer composed of 50 mM MOPS, 50 mM Tris base, 0.1% (w/v) SDS and 1mM EDTA.

As a result, the following spots in a two-dimensional electrophoresis gel run under the conditions described above for fish proteins have been revealed to exhibit specific binding to IgE antibodies from patients having an allergy to fish and being diagnosed as having immediate-type allergy.
Spot 1: Molecular weight 80 to 160 kDa, pI 3.0 to 7.0
Spot 2: Molecular weight 110 to 260 kDa, pI 4.0 to 8.0
Spot 3: Molecular weight 80 to 160 kDa, pI 4.0 to 10.0
Spot 4: Molecular weight 80 to 160 kDa, pI 5.0 to 11.0
Spot 5: Molecular weight 50 to 110 kDa, pI 3.0 to 7.0
Spot 6: Molecular weight 60 to 110 kDa, pI 4.0 to 8.0
Spot 7: Molecular weight 40 to 80 kDa, pI 4.0 to 8.0
Spot 8: Molecular weight 40 to 80 kDa, pI 3.0 to 7.0
Spot 9: Molecular weight 20 to 50 kDa, pI 3.0 to 7.0
Spot 10: Molecular weight 160 to 300 kDa, pI 3.0 to 7.0
Spot 11: Molecular weight 80 to 160 kDa, pI 4.0 to 8.0
Spot 12: Molecular weight 100 to 160 kDa, pI 4.0 to 7.0
Spot 13: Molecular weight 30 to 70 kDa, pI 3.0 to 7.0
Spot 14: Molecular weight 20 to 50 kDa, pI 5.0 to 9.0

### Antigen

### (1) Antigen in spot 1

As the result of sequence identification of the antigen in spot 1 by mass spectroscopy, the following amino acid sequences of SEQ ID NO: 3 were detected.

Also, the mass spectroscopic data (SEQ ID NO: 3) obtained for spot 1 on a mass spectrometer was analyzed by comparing the data against the NCB1 protein data, and as a result, the antigen in question was identified as Alpha-actinin-3 (amino acid sequence: SEQ ID NO: 2, encoding nucleotide sequence: SEQ ID NO: 1). SEQ ID NO: 3 corresponds to amino acid 572 to 585 in SEQ ID NO: 2.

Accordingly, in the present invention, the antigen in spot 1 can be any of (1A-a) to (1A-e) and (1B) as defined below:
(1A-a) a protein comprising an amino acid sequence with deletion, substitution, insertion or addition of one or several amino acids in SEQ ID NO: 2;
(1A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 2;
(1A-c) a protein comprising an amino acid sequence encoded by a nucleotide sequence with deletion, substitution, insertion or addition of one or several nucleotides in SEQ ID NO: 1;
(1A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 1;
(1A-e) a protein comprising an amino acid sequence encoded by a nucleic acid that hybridizes under stringent conditions with a nucleic acid having a nucleotide sequence complementary to the nucleotide sequence of SEQ ID NO: 1;
(1B) a protein comprising at least one amino acid sequence of SEQ ID NO: 2 or 3, preferably a protein comprising all sequences of the amino acid sequences. As referred to above, the amino acid sequence of SEQ ID NO: 2 or 3 may be an amino acid sequence derived therefrom by deletion, substitution, insertion or addition of one or several amino acids.

The proteins of (1A-a) to (1A-e) and (1B) as defined above also include those proteins whose amino acid residues are modified by phosphorylation, sugar chain modification, aminoacylation, ring-opening, deamination or the like.

The proteins of (1A-a) to (1A-e) and (1B) as defined above can be proteins that are found in a protein spot with a molecular weight of around 80 to 160 kDa, preferably around 90 to 120 kDa and an isoelectric point of 3.0 to 7.0, preferably an isoelectric point of 3.0 to 6.0 on gels used in the two-dimensional electrophoresis performed under the conditions described above in the subsection titled "Identification of antigens".

Preferably, a protein that is an antigen in spot 1 is an antigen of an allergy to fish.

### (2) Antigen in spot 2

As the result of sequence identification of the antigen in spot 2 by mass spectroscopy, the following amino acid sequences of SEQ ID NOs: 6-8 were detected.

Also, the mass spectroscopic data obtained for spot 2 (SEQ ID NOs: 6-8) on a mass spectrometer was analyzed by comparing the data against the UniProt protein data, and as a result, the antigen in question was identified as EEF1A2 binding protein-like (amino acid sequence: SEQ ID NO: 5, encoding nucleotide sequence: SEQ ID NO: 4). SEQ ID NO: 6 corresponds to amino acid 143-159 in SEQ ID NO: 5, SEQ ID NO: 7 corresponds to amino acid 395-404 in SEQ ID NO: 5, and SEQ ID NO: 8 corresponds to amino acid 427-447 in SEQ ID NO: 5.

Accordingly, in the present invention, the antigen in spot 2 can be any of (2A-a) to (2A-e) and (2B) as defined below:
(2A-a) a protein comprising an amino acid sequence with deletion, substitution, insertion or addition of one or several amino acids in SEQ ID NO: 5;
(2A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 5;
(2A-c) a protein comprising an amino acid sequence encoded by a nucleotide sequence with deletion, substitution, insertion or addition of one or several nucleotides in SEQ ID NO: 4;
(2A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 4;
(2A-e) a protein comprising an amino acid sequence encoded by a nucleic acid that hybridizes under stringent conditions with a nucleic acid having a nucleotide sequence complementary to the nucleotide sequence of SEQ ID NO: 4;
(2B) a protein comprising at least one amino acid sequence selected from the group consisting of SEQ ID NOs: 5-8, preferably a protein comprising at least 2, 3, or all sequences of the amino acid sequences. As referred to above, the amino acid sequence of any of SEQ ID NOs: 5-8 may be an amino acid sequence derived therefrom by deletion, substitution, insertion or addition of one or several amino acids.

The proteins of (2A-a) to (2A-e) and (2B) as defined above also include those proteins whose amino acid residues are modified by phosphorylation, sugar chain modification, aminoacylation, ring-opening, deamination or the like.

The proteins of (2A-a) to (2A-e) and (2B) as defined above can be proteins that are found in a protein spot with a molecular weight of around 110 to 260 kDa, preferably around 120 to 160 kDa and an isoelectric point of 4.0 to 8.0, preferably an isoelectric point of 4.0 to 7.0 on gels used in the two-dimensional electrophoresis performed under the conditions described above in the subsection titled "Identification of antigens".

Preferably, a protein that is an antigen in spot 2 is an antigen of an allergy to fish.

### (3) Antigen in spot 3

As the result of sequence identification of the antigen in spot 3 by mass spectroscopy, the following amino acid sequences of SEQ ID NOs: 11-16 were detected.

Also, the mass spectroscopic data (SEQ ID NOs: 11-17) obtained for spot 3 on a mass spectrometer was analyzed by comparing the data against the Uniprot protein data, and as a result, the antigen in question was identified as Alpha-1, 4-glucan phosphorylase (amino acid sequence: SEQ ID NO: 10, encoding nucleotide sequence: SEQ ID NO: 9). SEQ ID NO: 11 corresponds to amino acid 836-844 in SEQ ID NO: 10, SEQ ID NO: 12 corresponds to amino acid 13-29 in SEQ ID NO: 10, SEQ ID NO: 13 corresponds to amino acid 657-681 in SEQ ID NO: 10, SEQ ID NO: 14 corresponds to amino acid 471-479 in SEQ ID NO: 10, SEQ ID NO: 15 corresponds to amino acid 546-555 in SEQ ID NO: 10, and SEQ ID NO: 16 corresponds to amino acid 727-741 in SEQ ID NO: 10.

Accordingly, in the present invention, the antigen in spot 3 can be any of (3A-a) to (3A-e) and (3B) as defined below:
(3A-a) a protein comprising an amino acid sequence with deletion, substitution, insertion or addition of one or several amino acids in SEQ ID NO: 10;
(3A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 10;
(3A-c) a protein comprising an amino acid sequence encoded by a nucleotide sequence with deletion, substitution, insertion or addition of one or several nucleotides in SEQ ID NO: 9;
(3A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 9.
(3A-e) a protein comprising an amino acid sequence encoded by a nucleic acid that hybridizes under stringent conditions with a nucleic acid having a nucleotide sequence complementary to the nucleotide sequence of SEQ ID NO: 9;
(3B) a protein comprising at least one amino acid sequence selected from the group consisting of SEQ ID NOs: 10-16, preferably a protein comprising at least 2, 3, 4, 5, 6 or all sequences of the amino acid sequences. More preferably, a protein comprising at least one amino acid sequence selected from the group consisting of SEQ ID NOs: 10, 12, 13, 15, and 16, preferably a protein comprising at least 2, 3, 4, or all sequences of the amino acid sequences. As referred to above, the amino acid sequence of any of SEQ ID NOs:
   10-16 may be an amino acid sequence derived therefrom by deletion, substitution, insertion or addition of one or several amino acids.

The proteins of (3A-a) to (3A-e) and (3B) as defined above also include those proteins whose amino acid residues are modified by phosphorylation, sugar chain modification, aminoacylation, ring-opening, deamination or the like.

The proteins of (3A-a) to (3A-e) and (3B) as defined above can be proteins that are found in a protein spot with a molecular weight of around 80 to 160 kDa, preferably around 90 to 120 kDa and an isoelectric point of 4.0 to 10.0, preferably an isoelectric point of 5.0 to 8.0 on gels used in the two-dimensional electrophoresis performed under the conditions described above in the subsection titled "Identification of antigens".

Preferably, a protein that is an antigen in spot 3 is an antigen of an allergy to fish.

### (4) Antigen in spot 4

As the result of sequence identification of the antigen in spot 4 by mass spectroscopy, the following amino acid sequences of SEQ ID NOs: 20-24 were detected.

Also, the mass spectroscopic data (SEQ ID NOs: 20-24) obtained for spot 4 on a mass spectrometer was analyzed by comparing the data against the Uniprot protein data, and as a result, the antigen in question was identified as Elongation factor 2 (amino acid sequence: SEQ ID NO: 19, encoding nucleotide sequence: SEQ ID NO: 18). SEQ ID NO: 20 corresponds to amino acid 831-840 in SEQ ID NO: 19, SEQ ID NO: 21 corresponds to amino acid 560-571 in SEQ ID NO: 19, SEQ ID NO: 22 corresponds to amino acid 560-572 in SEQ ID NO: 19, SEQ ID NO: 23 corresponds to amino acid 677-688 in SEQ ID NO: 19, and SEQ ID NO: 24 corresponds to amino acid 620-629 in SEQ ID NO: 19.

Accordingly, in the present invention, the antigen in spot 4 can be any of (4-a) to (4-e) and (4B) as defined below:
(4A-a) a protein comprising an amino acid sequence with deletion, substitution, insertion or addition of one or several amino acids in SEQ ID NO: 19;
(4A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 19;
(4A-c) a protein comprising an amino acid sequence encoded by a nucleotide sequence with deletion, substitution, insertion or addition of one or several nucleotides in SEQ ID NO: 18;
(4A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 18;
(4A-e) a protein comprising an amino acid sequence encoded by a nucleic acid that hybridizes under stringent conditions with a nucleic acid having a nucleotide sequence complementary to the nucleotide sequence of SEQ ID NO: 18;
(4B) a protein comprising at least one amino acid sequence selected from the group consisting of SEQ ID NOs: 19-24, preferably a protein comprising at least 2, 3, 4, 5, 6 or all sequences of the amino acid sequences. As referred to above, the amino acid sequence of any of SEQ ID NOs: 19-24 may be an amino acid sequence derived therefrom by deletion, substitution, insertion or addition of one or several amino acids.

The proteins of (4A-a) to (4A-e) and (4B) as defined above also include those proteins whose amino acid residues are modified by phosphorylation, sugar chain modification, aminoacylation, ring-opening, deamination or the like.

The proteins of (4A-a) to (4A-e) and (4B) as defined above can be proteins that are found in a protein spot with a molecular weight of around 80 to 160 kDa, preferably around 90 to 120 kDa and an isoelectric point of 5.0 to 11.0, preferably an isoelectric point of 6.0 to 8.0 on gels used in the two-dimensional electrophoresis performed under the conditions described above in the subsection titled "Identification of antigens".

Preferably, a protein that is an antigen in spot 4 is an antigen of an allergy to fish.

### (5) Antigen in spot 5

As the result of sequence identification of the antigen in spot 5 by mass spectroscopy, the following amino acid sequences of SEQ ID NOs: 27-31 were detected.

Also, the mass spectroscopic data (SEQ ID NOs: 27-31) obtained for spot 5 on a mass spectrometer was analyzed by comparing the data against the Uniprot protein data, and as a result, the antigen in question was identified as Heat shock cognate 70 kDa protein (amino acid sequence: SEQ ID NO: 26, encoding nucleotide sequence: SEQ ID NO: 25). SEQ ID NO: 27 corresponds to amino acid 113-126 in SEQ ID NO: 26, SEQ ID NO: 28 corresponds to amino acid 513-526 in SEQ ID NO: 26, SEQ ID NO: 29 corresponds to amino acid 89-102 in SEQ ID NO: 26, SEQ ID NO: 30 corresponds to amino acid 584-597 in SEQ ID NO: 26, and SEQ ID NO: 31 corresponds to amino acid 138-159 in SEQ ID NO: 26.

Accordingly, in the present invention, the antigen in spot 5 can be any of (5A-a) to (5A-e) and (5B) as defined below:
(5A-a) a protein comprising an amino acid sequence with deletion, substitution, insertion or addition of one or several amino acids in SEQ ID NO: 26;
(5A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 26;
(5A-c) a protein comprising an amino acid sequence encoded by a nucleotide sequence with deletion, substitution, insertion or addition of one or several nucleotides in SEQ ID NO: 25;
(5A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 25;
(5A-e) a protein comprising an amino acid sequence encoded by a nucleic acid that hybridizes under stringent conditions with a nucleic acid having a nucleotide sequence complementary to the nucleotide sequence of SEQ ID NO: 25;
(5B) a protein comprising at least one amino acid sequence selected from the group consisting of SEQ ID NOs: 26-31, preferably a protein comprising at least 2, 3, 4, 5 or all sequences of the amino acid sequences. As referred to above, the amino acid sequence of any of SEQ ID NOs: 26-31 may be an amino acid sequence derived therefrom by deletion, substitution, insertion or addition of one or several amino acids.

The proteins of (5A-a) to (5A-e) and (5B) as defined above also include those proteins whose amino acid residues are modified by phosphorylation, sugar chain modification, aminoacylation, ring-opening, deamination or the like.

The proteins of (5A-a) to (5A-e) and (5B) as defined above can be proteins that are found in a protein spot with a molecular weight of around 50 to 110 kDa, preferably around 60 to 90 kDa and an isoelectric point of 3.0 to 7.0, preferably an isoelectric point of 3.0 to 6.0 on gels used in the two-dimensional electrophoresis performed under the conditions described above in the subsection titled "Identification of antigens".

Preferably, a protein that is an antigen is an antigen of an allergy to fish.

### (6) Antigen in spot 6

As the result of sequence identification of the antigen in spot 6 by mass spectroscopy, the following amino acid sequences of SEQ ID NOs: 34-41 were detected.

Also, the mass spectroscopic data (SEQ ID NOs: 34-41) obtained for spot 6 on a mass spectrometer was analyzed by comparing the data against the Uniprot protein data, and as a result, the antigen in question was identified as Serotransferrin (amino acid sequence: SEQ ID NO: 33, encoding nucleotide sequence: SEQ ID NO: 32). SEQ ID NO: 34 corresponds to amino acid 330-341 in SEQ ID NO: 33, SEQ ID NO: 35 corresponds to amino acid 332-341 in SEQ ID NO: 33, SEQ ID NO: 36 corresponds to amino acid 264-274 in SEQ ID NO: 33, SEQ ID NO: 37 corresponds to amino acid 113-124 in SEQ ID NO: 33, SEQ ID NO: 38 corresponds to amino acid 112-124 in SEQ ID NO: 33, SEQ ID NO: 39 corresponds to amino acid 112-125 in SEQ ID NO: 33, SEQ ID NO: 40 corresponds to amino acid 275-292 in SEQ ID NO: 33, and SEQ ID NO: 41 corresponds to amino acid 436-446 in SEQ ID NO: 33.

Accordingly, in the present invention, the antigen in spot 6 can be any of (6A-a) to (6A-e) and (6B) as defined below:
(6A-a) a protein comprising an amino acid sequence with deletion, substitution, insertion or addition of one or several amino acids in SEQ ID NO: 33;
(6A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 33;
(6A-c) a protein comprising an amino acid sequence encoded by a nucleotide sequence with deletion, substitution, insertion or addition of one or several nucleotides in SEQ ID NO: 32;
(6A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 32;
(6A-e) a protein comprising an amino acid sequence encoded by a nucleic acid that hybridizes under stringent conditions with a nucleic acid having a nucleotide sequence complementary to the nucleotide sequence of SEQ ID NO: 32;
(6B) a protein comprising at least one amino acid sequence selected from the group consisting of SEQ ID NOs: 33-41, preferably a protein comprising at least 2, 3, 4, 5, 6, 7, 8 or all sequences of the amino acid sequences. As referred to above, the amino acid sequence of any of SEQ ID NOs: 33-41 may be an amino acid sequence derived therefrom by deletion, substitution, insertion or addition of one or several amino acids.

The proteins of (6A-a) to (6A-e) and (6B) as defined above also include those proteins whose amino acid residues are modified by phosphorylation, sugar chain modification, aminoacylation, ring-opening, deamination or the like.

The proteins of (6A-a) to (6A-e) and (6B) as defined above can be proteins that are found in a protein spot with a molecular weight of around 60 to 110 kDa, preferably around 70 to 100 kDa and an isoelectric point of 4.0 to 8.0, preferably an isoelectric point of 5.0 to 7.0 on gels used in the two-dimensional electrophoresis performed under the conditions described above in the subsection titled "Identification of antigens".

Preferably, a protein that is an antigen in spot 6 is an antigen of an allergy to fish.

### (7) Antigen in spot 7

As the result of sequence identification of the antigen in spot 7 by mass spectroscopy, the following amino acid sequences of SEQ ID NOs: 44-54 were detected.

Also, the mass spectroscopic data (SEQ ID NOs: 44-54) obtained for spot 7 on a mass spectrometer was analyzed by comparing the data against the Uniprot protein data, and as a result, the antigen in question was identified as Myosin binding protein H-like (amino acid sequence: SEQ ID NO: 43, encoding nucleotide sequence: SEQ ID NO: 42). SEQ ID NO: 44 corresponds to amino acid 275-292 in SEQ ID NO: 43, SEQ ID NO: 45 corresponds to amino acid 49-66 in SEQ ID NO: 43, SEQ ID NO: 46 corresponds to amino acid 67-77 in SEQ ID NO: 43, SEQ ID NO: 47 corresponds to amino acid 310-323 in SEQ ID NO: 43, SEQ ID NO: 48 corresponds to amino acid 382-401 in SEQ ID NO: 43, SEQ ID NO: 49 corresponds to amino acid 402-418 in SEQ ID NO: 43, SEQ ID NO: 50 corresponds to amino acid 210-219 in SEQ ID NO: 43, SEQ ID NO: 51 corresponds to amino acid 210-228 in SEQ ID NO: 43, SEQ ID NO: 52 corresponds to amino acid 152-183 in SEQ ID NO: 43, SEQ ID NO: 53 corresponds to amino acid 100-112 in SEQ ID NO: 43, and SEQ ID NO: 54 corresponds to amino acid 113-129 in SEQ ID NO: 43.

Accordingly, in the present invention, the antigen in spot 7 can be any of (7A-a) to (7A-e) and (7B) as defined below:
(7A-a) a protein comprising an amino acid sequence with deletion, substitution, insertion or addition of one or several amino acids in SEQ ID NO: 43;
(7A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 43;
(7A-c) a protein comprising an amino acid sequence encoded by a nucleotide sequence with deletion, substitution, insertion or addition of one or several nucleotides in SEQ ID NO: 42;
(7A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 42;
(7A-e) a protein comprising an amino acid sequence encoded by a nucleic acid that hybridizes under stringent conditions with a nucleic acid having a nucleotide sequence complementary to the nucleotide sequence of SEQ ID NO: 42;
(7B) a protein comprising at least one amino acid sequence selected from the group consisting of SEQ ID NOs: 43-54, preferably a protein comprising at least 2, 3, 4, 5, 6, 7, 8, 9, 10, 11 or all sequences of the amino acid sequences; As referred to above, the amino acid sequence of any of SEQ ID NOs: 43-54 may be an amino acid sequence derived therefrom by deletion, substitution, insertion or addition of one or several amino acids.

The proteins of (7A-a) to (7A-e) and (7B) as defined above also include those proteins whose amino acid residues are modified by phosphorylation, sugar chain modification, aminoacylation, ring-opening, deamination or the like.

The proteins of (7A-a) to (7A-e) and (7B) as defined above can be proteins that are found in a protein spot with a molecular weight of around 40 to 80 kDa, preferably around 50 to 60 kDa and an isoelectric point of 4.0 to 8.0, preferably an isoelectric point of 4.0 to 7.0 on gels used in the two-dimensional electrophoresis performed under the conditions described above in the subsection titled "Identification of antigens".

Preferably, a protein that is an antigen in spot 7 is an antigen of an allergy to fish.

### (8) Antigen in spot 8

As the result of sequence identification of the antigen in spot 8 by mass spectroscopy, the following amino acid sequences of SEQ ID NOs: 57-59 were detected.

Also, the mass spectroscopic data (SEQ ID NOs: 57-59) obtained for spot 8 on a mass spectrometer was analyzed by comparing the data against the Uniprot protein data, and as a result, the antigen in question was identified as Desmin (Fragment) (amino acid sequence: SEQ ID NO: 56, encoding nucleotide sequence: SEQ ID NO: 55). SEQ ID NO: 57 corresponds to amino acid 364-380 in SEQ ID NO: 56, SEQ ID NO: 58 corresponds to amino acid 74-94 in SEQ ID NO: 56, and SEQ ID NO: 59 corresponds to amino acid 273-282 in SEQ ID NO: 56.

Accordingly, in the present invention, the antigen in spot 8 can be any of (8A-a) to (8A-e) and (8B) as defined below:
(8A-a) a protein comprising an amino acid sequence with deletion, substitution, insertion or addition of one or several amino acids in SEQ ID NO: 56;
(8A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 56;
(8A-c) a protein comprising an amino acid sequence encoded by a nucleotide sequence with deletion, substitution, insertion or addition of one or several nucleotides in SEQ ID NO: 55;
(8A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 55;
(8A-e) a protein comprising an amino acid sequence encoded by a nucleic acid that hybridizes under stringent conditions with a nucleic acid having a nucleotide sequence complementary to the nucleotide sequence of SEQ ID NO: 55;
(8B) a protein comprising at least one amino acid sequence selected from the group consisting of SEQ ID NOs: 56-59, preferably a protein comprising at least 2, 3, or all sequences of the amino acid sequences. As referred to above, the amino acid sequence of any of SEQ ID NOs: 56-59 may be an amino acid sequence derived therefrom by deletion, substitution, insertion or addition of one or several amino acids.

The proteins of (8A-a) to (8A-e) and (8B) as defined above also include those proteins whose amino acid residues are modified by phosphorylation, sugar chain modification, aminoacylation, ring-opening, deamination or the like.

The proteins of (8A-a) to (8A-e) and (8B) as defined above can be proteins that are found in a protein spot with a molecular weight of around 40 to 80 kDa, preferably around 50 to 60 kDa and an isoelectric point of 3.0 to 7.0, preferably an isoelectric point of 4.0 to 6.0 on gels used in the two-dimensional electrophoresis performed under the conditions described above in the subsection titled "Identification of antigens".

Preferably, a protein that is an antigen in spot 8 is an antigen of an allergy to fish.

### (9) Antigen in spot 9

As the result of sequence identification of the antigen in spot 9 by mass spectroscopy, the following amino acid sequences of SEQ ID NOs: 62-68 were detected.

Also, the mass spectroscopic data (SEQ ID NOs: 62-68) obtained for spot 9 on a mass spectrometer was analyzed by comparing the data against the UniProt protein data, and as a result, the antigen in question was identified as Capping protein (Actin filament) muscle Z-line beta (amino acid sequence: SEQ ID NO: 61, encoding nucleotide sequence: SEQ ID NO: 60). SEQ ID NO: 62 corresponds to amino acid 200-223 in SEQ ID NO: 61, SEQ ID NO: 63 corresponds to amino acid 238-254 in SEQ ID NO: 61, SEQ ID NO: 64 corresponds to amino acid 261-274 in SEQ ID NO: 61, SEQ ID NO: 65 corresponds to amino acid 224-235 in SEQ ID NO: 61, SEQ ID NO: 66 corresponds to amino acid 169-181 in SEQ ID NO: 61, SEQ ID NO: 67 corresponds to amino acid 245-254 in SEQ ID NO: 61, and SEQ ID NO: 68 corresponds to amino acid 79-95 in SEQ ID NO: 61.

Accordingly, the antigen in spot 9 in the present invention can be any of the proteins as defined below in (9A-a) to (9A-e), and (9B):
(9A-a) a protein comprising an amino acid sequence with deletion, substitution, insertion or addition of one or several amino acids in SEQ ID NO: 61;
(9A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 61;
(9A-c) a protein comprising an amino acid sequence encoded by a nucleotide sequence with deletion, substitution, insertion or addition of one or several nucleotides in SEQ ID NO: 60;
(9A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 60;
(9A-e) a protein comprising an amino acid sequence encoded by a nucleic acid that hybridizes under stringent conditions with a nucleic acid having a nucleotide sequence complementary to the nucleotide sequence of SEQ ID NO: 60;
(9B) a protein comprising at least one amino acid sequence selected from the group consisting of SEQ ID NOs: 61-68, preferably a protein comprising at least 2, 3, 4, 5, 6, 7 or all sequences of the amino acid sequences. As referred to above, the amino acid sequence of any of SEQ ID NOs: 61-68 may be an amino acid sequence derived therefrom by deletion, substitution, insertion or addition of one or several amino acids;

The proteins as defined above in (9A-a) to (9A-e), and (9B) also include those proteins whose amino acid residues are modified by phosphorylation, sugar chain modification, aminoacylation, ring-opening, deamination or the like.

The proteins as defined above in (9A-a) to (9A-e), and (9B) can be proteins that are found in a protein spot with a molecular weight of around 20 to 50 kDa, preferably around 30 to 40 kDa, and an isoelectric point of 3.0 to 7.0, preferably an isoelectric point of 4.0 to 6.0 on gels used in the two-dimensional electrophoresis performed under the conditions described above in the subsection titled "Identification of antigens".

Preferably, a protein that is an antigen in spot 9 is an antigen of an allergy to fish.

### (10) Antigen in spot 10

As the result of sequence identification of the antigen in spot 10 by mass spectroscopy, the following amino acid sequences of SEQ ID NOs: 71-108 were detected.

Also, the mass spectroscopic data (SEQ ID NOs: 71-108) obtained for spot 10 on a mass spectrometer was analyzed by comparing the data against the NCBI protein data, and as a result, the antigen in question was identified as myosin heavy chain, fast skeletal muscle-like (amino acid sequence: SEQ ID NO: 70, encoding nucleotide sequence: SEQ ID NO: 69). SEQ ID NO: 71 corresponds to amino acid 13-19 in SEQ ID NO:70, SEQ ID NO: 72 corresponds to amino acid 262-271 in SEQ ID NO: 70, SEQ ID NO: 73 corresponds to amino acid 996-1014 in SEQ ID NO: 70, SEQ ID NO: 74 corresponds to amino acid 951-961 in SEQ ID NO: 70, SEQ ID NO: 75 corresponds to amino acid 1293-1303 in SEQ ID NO: 70, SEQ ID NO: 76 corresponds to amino acid 1082-1091 in SEQ ID NO: 70, SEQ ID NO: 77 corresponds to amino acid 1847-1856 in SEQ ID NO:70, SEQ ID NO: 78 corresponds to amino acid 172-186 in SEQ ID NO: 70, SEQ ID NO: 79 corresponds to amino acid 919-937 in SEQ ID NO: 70, SEQ ID NO: 80 corresponds to amino acid 249-258 in SEQ ID NO: 70, SEQ ID NO: 81 corresponds to amino acid 706-717 in SEQ ID NO: 70, SEQ ID NO: 82 corresponds to amino acid 50-59 in SEQ ID NO: 70, SEQ ID NO: 83 corresponds to amino acid 415-430 in SEQ ID NO:70, SEQ ID NO: 84 corresponds to amino acid 834-845 in SEQ ID NO: 70, SEQ ID NO: 85 corresponds to amino acid 617-630 in SEQ ID NO: 70, SEQ ID NO: 86 corresponds to amino acid 1556-1567 in SEQ ID NO: 70, SEQ ID NO: 87 corresponds to amino acid 1391-1408 in SEQ ID NO: 70, SEQ ID NO: 88 corresponds to amino acid 1025-1040 in SEQ ID NO: 70, SEQ ID NO: 89 corresponds to amino acid 1813-1836 in SEQ ID NO:70, SEQ ID NO: 90 corresponds to amino acid 743-755 in SEQ ID NO: 70, SEQ ID NO: 91 corresponds to amino acid 1172-1192 in SEQ ID NO: 70, SEQ ID NO: 92 corresponds to amino acid 353-364 in SEQ ID NO: 70, SEQ ID NO: 93 corresponds to amino acid 1261-1292 in SEQ ID NO: 70, SEQ ID NO: 94 corresponds to amino acid 1783-1789 in SEQ ID NO: 70, SEQ ID NO: 95 corresponds to amino acid 1502-1519 in SEQ ID NO: 70, SEQ ID NO: 96 corresponds to amino acid 1484-1497 in SEQ ID NO:70, SEQ ID NO: 97 corresponds to amino acid 1194-1212 in SEQ ID NO: 70, SEQ ID NO: 98 corresponds to amino acid 1304-1314 in SEQ ID NO: 70, SEQ ID NO: 99 corresponds to amino acid 369-384 in SEQ ID NO: 70, SEQ ID NO: 100 corresponds to amino acid 1315-1322 in SEQ ID NO: 70, SEQ ID NO: 101 corresponds to amino acid 1536-1555 in SEQ ID NO: 70, SEQ ID NO: 102 corresponds to amino acid 237-248 in SEQ ID NO:70, SEQ ID NO: 103 corresponds to amino acid 1699-1725 in SEQ ID NO: 70, SEQ ID NO: 104 corresponds to amino acid 1092-1104 in SEQ ID NO: 70, SEQ ID NO: 105 corresponds to amino acid 407-414 in SEQ ID NO: 70, SEQ ID NO: 106 corresponds to amino acid 1897-1917 in SEQ ID NO: 70, SEQ ID NO:107 corresponds to amino acid 1458-1470 in SEQ ID NO: 70, and SEQ ID NO: 108 corresponds to amino acid 1373-1388 in SEQ ID NO: 70.

Accordingly, the antigen in spot 10 in the present invention can be any of the proteins as defined below in (10A-a) to (10A-e), and (10B):
(10A-a) a protein comprising an amino acid sequence with deletion, substitution, insertion or addition of one or several amino acids in SEQ ID NO: 70;
(10A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 70;
(10A-c) a protein comprising an amino acid sequence encoded by a nucleotide sequence with deletion, substitution, insertion or addition of one or several nucleotides in SEQ ID NO: 69;
(10A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 69;
(10A-e) a protein comprising an amino acid sequence encoded by a nucleic acid that hybridizes under stringent conditions with a nucleic acid having a nucleotide sequence complementary to the nucleotide sequence of SEQ ID NO: 69;
(10B) a protein comprising at least one amino acid sequence selected from the group consisting of SEQ ID NOs: 70-108, preferably a protein comprising at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 or all sequences of the amino acid sequences. As referred to above, the amino acid sequence of any of SEQ ID NOs: 70-108 may be an amino acid sequence derived therefrom by deletion, substitution, insertion or addition of one or several amino acids;

The proteins as defined above in (10A-a) to (10A-e), and (10B) also include those proteins whose amino acid residues are modified by phosphorylation, sugar chain modification, aminoacylation, ring-opening, deamination or the like.

The proteins as defined above in (10A-a) to (10A-e), and (10B) can be proteins that are found in a protein spot with a molecular weight of around 160 to 300 kDa, preferably around 160 to 260 kDa, more preferably around 200 to 230 kDa, and an isoelectric point of 3.0 to 7.0, preferably an isoelectric point of 4.0 to 6.0, more preferably an isoelectric point of 4.5 to 5.5 on gels used in the two-dimensional electrophoresis performed under the conditions described above in the subsection titled "Identification of antigens".

Preferably, a protein that is an antigen in spot 10 is an antigen of an allergy to fish.

### (11) Antigen in spot 11

As the result of sequence identification of the antigen in spot 11 by mass spectroscopy, the following amino acid sequences of SEQ ID NOs: 111-119 were detected.

Also, the mass spectroscopic data (SEQ ID NOs: 111-119) obtained for spot 11 on a mass spectrometer was analyzed by comparing the data against the NCBI protein data, and as a result, the antigen in question was identified as glycogen phosphorylase, muscle form-like (amino acid sequence: SEQ ID NO: 110, encoding nucleotide sequence: SEQ ID NO: 109). SEQ ID NO: 111 corresponds to amino acid 471-479 in SEQ ID NO: 110, SEQ ID NO: 112 corresponds to amino acid 547-555 in SEQ ID NO: 110, SEQ ID NO: 113 corresponds to amino acid 203-215 in SEQ ID NO:110, SEQ ID NO: 114 corresponds to amino acid 727-741 in SEQ ID NO: 110, SEQ ID NO: 115 corresponds to amino acid 508-520 in SEQ ID NO: 110, SEQ ID NO: 116 corresponds to amino acid 742-755 in SEQ ID NO: 110, SEQ ID NO: 117 corresponds to amino acid 13-29 in SEQ ID NO: 110, SEQ ID NO:118 corresponds to amino acid 775-784 in SEQ ID NO: 110, and SEQ ID NO: 119 corresponds to amino acid 643-656 in SEQ ID NO: 110.

Accordingly, the antigens in spot 11 in the present invention can be any of the proteins as defined below in (11A-a) to (11A-e) and (11B):
(1 1A-a) a protein comprising an amino acid sequence with deletion, substitution, insertion or addition of one or several amino acids in SEQ ID NO: 110;
(11A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 110;
(11A-c) a protein comprising an amino acid sequence encoded by a nucleotide sequence with deletion, substitution, insertion or addition of one or several nucleotides in SEQ ID NO: 109;
(11A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 109;
(11A-e) a protein comprising an amino acid sequence encoded by a nucleic acid that hybridizes under stringent conditions with a nucleic acid having a nucleotide sequence complementary to the nucleotide sequence of SEQ ID NO: 109;
(11B) a protein comprising at least one amino acid sequence selected from the group consisting of SEQ ID NOs: 110-119, preferably a protein comprising at least 2, 3, 4, 5, 6, 7, 8, 9 or all sequences of the amino acid sequences. As referred to above, the amino acid sequence of any of SEQ ID NOs: 110-119 may be an amino acid sequence derived therefrom by deletion, substitution, insertion or addition of one or several amino acids.

The proteins of (11A-a) to (11A-e) and (11B) as defined above also include those proteins whose amino acid residues are modified by phosphorylation, sugar chain modification, aminoacylation, ring-opening, deamination or the like.

The proteins of (11A-a) to (11A-e) and (11B) as defined above can be proteins that are found in a protein spot with a molecular weight of around 80 to 160 kDa, preferably around 80 to 110 kDa, more preferably around 90 to 110 kDa and an isoelectric point of 4.0 to 8.0, preferably an isoelectric point of 5.0 to 7.5, more preferably an isoelectric point of 6.5 to 7.0 on gels used in the two-dimensional electrophoresis performed under the conditions described above in the subsection titled "Identification.of antigens".

Preferably, a protein that is an antigen in spot 11 is an antigen of an allergy to fish.

### (12) Antigen in spot 12

As the result of sequence identification of the antigen in spot 12 by mass spectroscopy, the following amino acid sequences of SEQ ID NOs: 122-136 were detected.

Also, the mass spectroscopic data (SEQ ID NOs: 122-136) obtained for spot 12 on a mass spectrometer was analyzed by comparing the data against the NCBI protein data, and as a result, the antigen in question was identified as myosin-binding protein C, fast-type-like (amino acid sequence: SEQ ID NO: 121, encoding nucleotide sequence: SEQ ID NO: 120). SEQ ID NO: 122 corresponds to amino acid 197-204 in SEQ ID NO: 121, SEQ ID NO: 123 corresponds to amino acid 421-434 in SEQ ID NO: 121, SEQ ID NO: 124 corresponds to amino acid 329-336 in SEQ ID NO:121, SEQ ID NO: 125 corresponds to amino acid 413-420 in SEQ ID NO: 121, SEQ ID NO:126 corresponds to amino acid 240-246 in SEQ ID NO: 121, SEQ ID NO: 127 corresponds to amino acid 214-228 in SEQ ID NO: 121, SEQ ID NO: 128 corresponds to amino acid 1014-1024 in SEQ ID NO: 121, SEQ ID NO: 129 corresponds to amino acid 842-855 in SEQ ID NO: 121, SEQ ID NO: 130 corresponds to amino acid 260-274 in SEQ ID NO:121, SEQ ID NO: 131 corresponds to amino acid 672-685 in SEQ ID NO: 121, SEQ ID NO: 132 corresponds to amino acid 506-512 in SEQ ID NO: 121, SEQ ID NO: 133 corresponds to amino acid 205-213 in SEQ ID NO: 121, SEQ ID NO: 134 corresponds to amino acid 165-182 in SEQ ID NO: 121, SEQ ID NO:135 corresponds to amino acid 321-328 in SEQ ID NO: 121, and SEQ ID NO: 136 corresponds to amino acid 70-82 in SEQ ID NO: 121.

Accordingly, the antigens in spot 12 in the present invention can be any of the proteins as defined below in (12A-a) to (12A-e) and (12B):
(12A-a) a protein comprising an amino acid sequence with deletion, substitution, insertion or addition of one or several amino acids in SEQ ID NO: 121;
(12A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 121;
(12A-c) a protein comprising an amino acid sequence encoded by a nucleotide sequence with deletion, substitution, insertion or addition of one or several nucleotides in SEQ ID NO: 119;
(12A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 119;
(12A-e) a protein comprising an amino acid sequence encoded by a nucleic acid that hybridizes under stringent conditions with a nucleic acid having a nucleotide sequence complementary to the nucleotide sequence of SEQ ID NO: 119;
(12B) a protein comprising at least one amino acid sequence selected from the group consisting of SEQ ID NOs: 121-136, preferably a protein comprising at least 2, 3, 4, 5, 6, 7, 8, 9, 10, 11, 12, 13, 14, 15 or all sequences of the amino acid sequences. As referred to above, the amino acid sequence of any of SEQ ID NOs: 121-136 may be an amino acid sequence derived therefrom by deletion, substitution, insertion or addition of one or several amino acids.

The proteins of (12A-a) to (12A-e) and (12B) as defined above also include those proteins whose amino acid residues are modified by phosphorylation, sugar chain modification, aminoacylation, ring-opening, deamination or the like.

The proteins of (12A-a) to (12A-e) and (12B) as defined above can be proteins that are found in a protein spot with a molecular weight of around 100 to 160 kDa, preferably around 110 to 150 kDa, more preferably around 120 to 140 kDa and an isoelectric point of 4.0 to 7.0, preferably an isoelectric point of 4.0 to 6.0, more preferably an isoelectric point of 5.0 to 6.0 on gels used in the two-dimensional electrophoresis performed under the conditions described above in the subsection titled "Identification of antigens".

Preferably, a protein that is an antigen in spot 12 is an antigen of an allergy to fish.

### (13) Antigen in spot 13

As the result of sequence identification of the antigen in spot 13 by mass spectroscopy, the following amino acid sequences of SEQ ID NOs: 139-142 were detected.

Also, the mass spectroscopic data (SEQ ID NOs: 139-142) obtained for spot 13 on a mass spectrometer was analyzed by comparing the data against the NCBI protein data, and as a result, the antigen in question was identified as ATP synthase subunit beta, mitochondrial (amino acid sequence: SEQ ID NO: 138, encoding nucleotide sequence: SEQ ID NO: 137). SEQ ID NO: 139 corresponds to amino acid 191-201 in SEQ ID NO: 138, SEQ ID NO: 140 corresponds to amino acid 449-469 in SEQ ID NO: 138, SEQ ID NO:141 corresponds to amino acid 202-214 in SEQ ID NO: 138, and SEQ ID NO: 142 corresponds to amino acid 178-187 in SEQ ID NO: 138.

Accordingly, the antigen in spot 13 in the present invention can be any of the proteins as defined below in (13A-a) to (13A-e), and (13B):
(13A-a) a protein comprising an amino acid sequence with deletion, substitution, insertion or addition of one or several amino acids in SEQ ID NO: 138;
(13A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 138;
(13A-c) a protein comprising an amino acid sequence encoded by a nucleotide sequence with deletion, substitution, insertion or addition of one or several nucleotides in SEQ ID NO: 137;
(13A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO:137;
(13A-e) a protein comprising an amino acid sequence encoded by a nucleic acid that hybridizes under stringent conditions with a nucleic acid having a nucleotide sequence complementary to the nucleotide sequence of SEQ ID NO: 137;
(13B) a protein comprising at least one amino acid sequence selected from the group consisting of SEQ ID NOs: 138-142, preferably a protein comprising at least 2, 3, 4 or all sequences of the amino acid sequences. As referred to above, the amino acid sequence of any of SEQ ID NOs: 138-142 may be an amino acid sequence derived therefrom by deletion, substitution, insertion or addition of one or several amino acids;

The proteins as defined above in (13A-a) to (13A-e), and (13B) also include those proteins whose amino acid residues are modified by phosphorylation, sugar chain modification, aminoacylation, ring-opening, deamination or the like.

The proteins as defined above in (13A-a) to (13A-e), and (13B) can be proteins that are found in a protein spot with a molecular weight of around 30 to 70 kDa, preferably around 40 to 60 kDa, more preferably around 45 to 55 kDa and an isoelectric point of 3.0 to 7.0, preferably an isoelectric point of 3.0 to 6.0, more preferably an isoelectric point of 4.0 to 5.5 on gels used in the two-dimensional electrophoresis performed under the conditions described above in the subsection titled "Identification of antigens".

Preferably, a protein that is an antigen in spot 13 is an antigen of an allergy to fish.

### (14) Antigen in spot 14

As the result of sequence identification of the antigen in spot 14 by mass spectroscopy, the following amino acid sequences of SEQ ID NOs: 145-149 were detected.

Also, the mass spectroscopic data (SEQ ID NOs: 145-149) obtained for spot 14 on a mass spectrometer was analyzed by comparing the data against the NCBI protein data, and as a result, the antigen in question was identified as L-lactate dehydrogenase A chain-like (amino acid sequence: SEQ ID NO: 144, encoding nucleotide sequence: SEQ ID NO: 143). SEQ ID NO: 145 corresponds to amino acid 119-126 in SEQ ID NO: 144, SEQ ID NO: 146 corresponds to amino acid 7-22 in SEQ ID NO: 144, SEQ ID NO: 147 corresponds to amino acid 9-22 in SEQ ID NO: 144, SEQ ID NO: 148 corresponds to amino acid 270-278 in SEQ ID NO: 144, and SEQ ID NO: 149 corresponds to amino acid 91-118 in SEQ ID NO: 144.

Accordingly, the antigens in spot 14 in the present invention can be any of the proteins as defined below in (14A-a) to (14A-e) and (14B):
(14A-a) a protein comprising an amino acid sequence with deletion, substitution, insertion or addition of one or several amino acids in SEQ ID NO: 144;
(14A-b) a protein comprising an amino acid sequence having at least 70%, preferably at least 75%, at least 80%, at least 85%, at least 90%, at least 95%, at least 97%, at least 98%, or at least 99% identity to the amino acid sequence of SEQ ID NO: 144;
(14A-c) a protein comprising an amino acid sequence encoded by a nucleotide sequence with deletion, substitution, insertion or addition of one or several nucleotides in SEQ ID NO: 143;
(14A-d) a protein comprising an amino acid sequence encoded by a nucleotide sequence having at least 70%, preferably at least 75%, at least 80%, at least 85%, at least 90%, at least 95%, at least 97%, at least 98%, or at least 99% identity to the nucleotide sequence of SEQ ID NO: 143;
(14A-e) a protein comprising an amino acid sequence encoded by a nucleic acid that hybridizes under stringent conditions with a nucleic acid having a nucleotide sequence complementary to the nucleotide sequence of SEQ ID NO: 143;
(14B) a protein comprising at least one amino acid sequence selected from the group consisting of SEQ ID NOs: 144-149, preferably a protein comprising at least 2, 3, 4, 5 or all sequences of the amino acid sequences. As referred to above, the amino acid sequence of any of SEQ ID NOs: 144-149 may be an amino acid sequence derived therefrom by deletion, substitution, insertion or addition of one or several amino acids.

The proteins of (14A-a) to (14A-e) and (14B) as defined above also include those proteins whose amino acid residues are modified by phosphorylation, sugar chain modification, aminoacylation, ring-opening, deamination or the like.

The proteins of (14A-a) to (14A-e) and (14B) as defined above can be proteins that are found in a protein spot with a molecular weight of around 20 to 50 kDa, preferably around 30 to 50 kDa, more preferably around 30 to 40 kDa and an isoelectric point of 5.0 to 9.0, preferably an isoelectric point of 6.0 to 8.0, more preferably an isoelectric point of 6.5 to 7.5 on gels used in the two-dimensional electrophoresis performed under the conditions described above in the subsection titled "Identification of antigens".

Preferably, a protein that is an antigen in spot 14 is an antigen of an allergy to fish.

By stating herein "deletion, substitution, insertion or addition of one or several amino acids" in relation to amino acid sequence, it is meant that in an amino acid sequence of interest, one or several amino acids (e.g., 30%, preferably 25%, 20%, 15%, 10%, 5%, 3%, 2%, or 1% of amino acids with respect to the total length of the amino acid sequence) are deleted, one or several amino acids are substituted by any other amino acids, any other amino acids are inserted, and/or any other amino acids are added.

Among the aforementioned modifications, substitution is preferably conservative substitution. The "conservative substitution" refers to the substitution of a certain amino acid residue by a different amino acid residue having similar physicochemical characteristics, and can be any type of substitution as long as it does not substantially change the characteristics of the structure of the original sequence for example, any type of substitution is acceptable as long as any substituted amino acids do not disrupt the helical structure of the original sequence or other secondary structures that characterize the original sequence. The following gives examples of separate groups of amino acid residues that are conservatively substitutable with each other, but substitutable amino acid residues are not limited to the examples given below.
Group A: leucine, isoleucine, valine, alanine, methionine
Group B: aspartic acid, glutamic acid
Group C: asparagine, glutamine
Group D: lysine, arginine
Group E: serine, threonine
Group F: phenylalanine, tyrosine

In the case of non-conservative substitution, one member belonging to one of the aforementioned groups can be replaced with a member belong to any other group. For example, in order to eliminate the possibility of unwanted sugar-chain modification, amino acids of group B, D or E as listed above may be substituted by those of any other group. Also, cysteines may be deleted or substituted by any other amino acids to prevent them from being folded into a protein in its tertiary structure. Also, in order to maintain the balance between hydrophilicity and hydrophobicity or to increase hydrophilicity for the purpose of facilitating synthesis, any amino acids may be substituted in consideration of the hydropathy scales of amino acids, which are a measure of the hydrophilic and hydrophobic properties of amino acids (J. Kyte and R. Doolittle, J. Mol. Biol., Vol.157, p.105-132, 1982).

As referred to herein, the percent identity between two amino acid sequences can be determined by visual inspection and mathematical calculation. Alternatively, the percent identity can be determined using a computer program. Examples of such computer programs include BLAST and ClustalW. In particular, various conditions (parameters) for identity searches with the BLAST program are described in Altschul, et al. (Nucl. Acids. Res., 25, p.3389-3402, 1997), and are publicly available on the websites of the National Center for Biotechnology Information (NCBI) and DNA Data Bank of Japan (DDBJ) (Altschul, et al., BLAST Manual, Altschul, et al., NCB/NLM/NIH Bethesda, MD 20894). Also, the percent identity can be determined using a genetic information processing software program, such as GENETYX Ver.7 (Genetyx Corporation), DINASIS Pro (Hitachi Software Engineering Co., Ltd.), or Vector NTI (Infomax Inc.).

By stating herein "deletion, substitution, insertion or addition of one or several nucleotides" in relation to nucleotide sequence, it is meant that in a nucleotide sequence of interest, one or several nucleotides (e.g., 30%, preferably 25%, 20%, 15%, 10%, 5%, 3%, 2% or 1%, of nucleotides with respect to the total length of the nucleotide sequence) are deleted, one or several nucleotides are substituted by any other nucleotides, any other nucleotides are inserted, and/or any other nucleotides are added. It is preferable that such a nucleotide deletion, substitution, insertion or addition should not give rise to a frame shift in an amino acid coding sequence.

As referred to herein, the percent identity between two nucleotide sequences can be determined by visual inspection and mathematical calculation. Alternatively, the percent identity can be determined using a computer program. Examples of such sequence comparison computer programs include the BLASTN program, version 2.2.7, available on the website of the National Library of Medicine (http://www.ncbi.nlm.nih.gov/blast/bl2seq/bls.html) (Altschul, et al. (1990) J. Mol. Biol., 215: 403-10), or the WU-BLAST 2.0 algorithm. Standard default parameter settings for WU-BLAST 2.0 are found and available on the following website: http://blast.wustl.edu.

As referred to herein, "under stringent conditions" means that hybridization takes place under moderately or highly stringent conditions. To be specific, the moderately stringent conditions can be easily determined by those having ordinary skill in the art on the basis of, for example, the length of DNA. Basic conditions are described in Sambrook, et al., Molecular Cloning: A Laboratory Manual, 3rd ed., ch.6-7, Cold Spring Harbor Laboratory Press, 2001. The moderately stringent conditions include hybridization under the conditions of preferably 1×SSC to 6×SSC at 42°C to 55°C, more preferably 1×SSC to 3×SSC at 45°C to 50°C, most preferably 2×SSC at 50°C. In the case of using a hybridization solution containing, for example, about 50% formamide, a temperature around 5 to 15°C lower than the foregoing should be adopted. Washing is also carried out under the conditions of 0.5×SSC to 6×SSC at 40°C to 60°C. In the process of hybridization and washing, generally 0.05% to 0.2% SDS, preferably about 0.1% SDS, may be added. Likewise, the highly stringent conditions can be easily determined by those having ordinary skill in the art on the basis of, for example, the length of DNA. Generally, the highly stringent (high stringent) conditions include hybridization and/or washing at a higher temperature and/or a lower salt concentration than those adopted under the moderately stringent conditions. For example, hybridization is carried out under the conditions of 0.1×SSC to 2×SSC at 55°C to 65°C, more preferably 0.1×SSC to 1×SSC at 60°C to 65°C, most preferably 0.2×SSC at 63°C. Washing is carried out under the conditions of 0.2×SSC to 2×SSC at 50°C to 68°C, more preferably 0.2×SSC at 60 to 65°C.

Antigens may be obtained by separating and purifying them from fish using a combination of protein purification methods well known to those skilled in the art. Also, antigens may be obtained by expressing them as recombinant proteins using a genetic recombination technique well known to those skilled in the art and by separating and purifying them using protein purification methods well known to those skilled in the art.

Exemplary protein purification methods include: solubility-based purification methods such as salt precipitation and solvent precipitation; purification methods based on molecular weight difference, such as dialysis, ultrafiltration, gel filtration and SDS-PAGE; charge-based purification methods such as ion exchange chromatography and hydroxylapatite chromatography; specific affinity-based purification methods such as affinity chromatography; purification methods based on hydrophobicity difference, such as reverse-phase high-performance liquid chromatography; and purification methods based on isoelectric point difference, such as isoelectric focusing.

Preparation of a protein by a genetic recombination technique is carried out by preparing an expression vector comprising an antigen-encoding nucleic acid, introducing the expression vector into appropriate host cells by gene transfer or genetic transformation, culturing the host cells under suitable conditions for expression of a recombinant protein, and recovering the recombinant protein expressed in the host cells.

The "vector" refers to a nucleic acid that can be used to introduce a nucleic acid attached thereto into host cells. The "expression vector" is a vector that can induce the expression of a protein encoded by a nucleic acid introduced thereby. Exemplary vectors include plasmid vectors, viral vectors, and the like. Those skilled in the art can select an appropriate expression vector for the expression of a recombinant protein depending on the type of host cells to be used. To facilitate purification, an affinity tags such as 6 His residues may be included. Moreover, a protein may be synthesized so that a signal sequence is included and the protein is secreted out of cells.

The "host cells" refers to cells that undergo gene transfer or genetic transformation by a vector. The host cells can be appropriately selected by those skilled in the art depending on the type of the vector to be used. The host cells can be derived from prokaryotes such as Escherichia coli (E. coli). When prokaryotic cells like E. coli are used as host cells, the antigen of the present invention may be designed to contain an N-terminal methionine residue in order to facilitate the expression of a recombinant protein in the prokaryotic cells. The N-terminal methionine can be cleaved from the recombinant protein after expression. Also, the host cells may be cells derived from eukaryotes, such as single-cell eukaryotes like yeast, plant cells and animal cells (e.g., human cells, monkey cells, hamster cells, rat cells, murine cells or insect cells) or silkworm.

Gene transfer or genetic transformation of an expression vector into host cells can be carried out as appropriate by following a technique known to those skilled in the art. Those skilled in the art can make possible the expression of a recombinant protein by selecting suitable conditions for the expression of the recombinant protein as appropriate depending on the type of host cells and culturing the host cells under the selected conditions. Then, those skilled in the art can homogenize the host cells having the expressed recombinant protein, and separate and purify an antigen expressed as the recombinant protein from the resulting homogenate by using an appropriate combination of such protein purification methods as mentioned above.

### Diagnostic kit and method

The present invention provides a method for providing an indicator for diagnosing a fish allergy in a subject, the method comprising the steps of:
(i) contacting a sample obtained from the subject with an antigen, wherein the sample is a solution comprising an IgE antibody;
(ii) detecting binding between an IgE antibody present in the sample obtained from the subject and the antigen; and
(iii) when the binding between the IgE antibody in the subject and the antigen is detected, an indicator of the fact that the subject has a fish allergy is provided;
wherein the antigen is at least one of the proteins identified as the aforementioned antigens (1) to (9) or at least one of the proteins identified as the aforementioned antigens (10) to (14).

The sample obtained from a subject is a solution containing an IgE antibody, as collected from the subject. Examples of such solutions include blood, saliva, sputum, snivel, urine, sweat, and tear. The sample obtained from the subject may be subjected to pretreatment for increasing the concentration of an IgE antibody in the sample before being contacted with an antigen. The pretreatment of a sample may involve, for example, collection of the serum and the plasma from the blood. Furthermore, Fab portions that are binding portions with the antigens may be purified. In a particularly preferred embodiment, the step (i) mentioned above is carried out by contacting an IgE antibody present in the serum obtained from a subject with an antigen.

The IgE antibody may be the IgE antibody itself or mast cells or the like to which the IgE antibody is bound.

Detection of contact and binding between the sample obtained from a subject and an antigen can be carried out by using a known method. Examples of such methods that can be used include detection by ELISA (Enzyme-Linked Immunosorvent Assay), sandwich immunoassay, immunoblotting, immunoprecipitation, or immunochromatography. These are all techniques involving contacting and binding an IgE antibody from a subject with an antigen and detecting the IgE antibody specifically bound to the antigen. Examples thereof include methods involving bringing an enzyme-labeled secondary antibody in contact, adding a substrate (usually a chromogenic or luminescent reagent) of the enzyme, and detecting the product of the enzymatic reaction; methods involving bringing a biotinylated secondary antibody in contact, adding an avidin-conjugated dye, and detecting the dye; and methods involving detecting a fluorescently labeled secondary antibody. Also, detection by a measurement method that permits the evaluation of binding between an antigen and an IgE antibody, such as surface plasmon resonance (SPR), can be used. A plurality of antigen-specific IgE antibodies may be mixed.

The antigen may be provided as an isolated antigen in a state immobilized on a carrier. In this case, the steps (i) and (ii) mentioned above can be carried out using ELISA, sandwich immunoassay, immunochromatography, surface plasmon resonance, or the like. Also, the step (i) mentioned above is carried out by contacting the sample obtained from a subject with an antigen-immobilized surface. The isolated antigen may be obtained by separating and purifying it from fish using a combination of protein purification methods well known to those skilled in the art, or by preparing it using a genetic recombination technique. Moreover, the antibody that binds to the antigen may be in an immobilized state.

The antigen may be in an unimmobilized state to a carrier. In this case, the steps (i) and (ii) mentioned above can be carried out by flow cytometry or the like and the presence of the antigen bound to an antibody may be confirmed using a laser beam. Examples include basophil activation test (BAT) in which an antigen binds to an antibody and the amount of the surface antigen CD203c which is expressed when basophils in the sample are activated is measured, and the like. Another example is histamine release test (HRT) in which it is examined whether histamine is released or not by bringing an antigen in contact with hemocytes via the binding to an antibody in a sample.

The antigen may be transferred from a state separated by two-dimensional electrophoresis and detected by immunoblotting. The two-dimensional electrophoresis is a technique for separating a protein sample by performing isoelectric focusing in the first dimension and performing SDS-PAGE (SDS-polyacrylamide gel electrophoresis) in the second dimension. The conditions for two-dimensional electrophoresis are not particularly limited as long as the conditions permit the separation of the antigen of the present invention. For example, the conditions for two-dimensional electrophoresis as described above in the subsection titled "Identification of antigens" can be adopted. Also, the electrophoresis conditions may be determined by reference to the descriptions in PTLs 1 to 4 mentioned above. For example, two-dimensional electrophoresis can be carried out under the conditions that satisfy at least one selected from the group consisting of the following requirements:
(A) the isoelectric focusing gels used in the first dimension should have a gel-strip length of 5 to 10 cm and a gel pH range of 3 to 10, and the pH gradient of the gels in the direction of electrophoresis should be as follows: where the gel-strip length up to pH 5 is taken as "a", that length from pH 5 to 7 as "b", and that length above pH 7 as "c", the relations "a < b" and "b > c" are satisfied;
(B) in the case of (A), when the total gel-strip length is taken as 1, "a" should be in the range of 0.15 to 0.3, "b" should be in the range of 0.4 to 0.7, and "c" should be in the range of 0.15 to 0.3;
(C) in the first dimensional isoelectric focusing, a constant voltage step should be performed by applying a constant voltage ranging from 100 V to 600 V per gel strip containing a sample, and after the electrophoresis variation width during electrophoresis for 30 minutes falls within the range of 5 µA, a voltage-increasing step should be started at which the voltage is increased from the aforementioned constant voltage;
(D) in the case of (C), the final voltage at the voltage-increasing step should be in the range of 3000 V to 6000 V;
(E) the isoelectric focusing gels used in the first dimension should have a longitudinal gel-strip length of 5 to 10 cm, and the electrophoresis gels used in the second dimension should have a gel concentration at the distal end in the direction of electrophoresis, which is in the range of 3 to 6%; and
(F) in the case of (E), the electrophoresis gels used in the second dimension should have a gel concentration at the proximal end in the direction of electrophoresis, which is set to a higher value than that at the distal end in the direction of electrophoresis.

The aforementioned antigens (1) to (9) and the aforementioned antigens (10) to (14) are antigens which specifically bind to IgE antibodies in patients with an allergy to fish. Therefore, when binding between an IgE antibody from a subject and the antigen is detected, an indicator of the fact that the subject has a fish allergy is provided.

The present invention further provides a kit for diagnosing an allergy to a fish, comprising at least one of the aforementioned antigens (1) to (9) or at least one of the aforementioned antigens (10) to (14). The diagnostic kit of this invention may be used in the aforementioned method for providing an indicator for diagnosing an allergy to a fish or in a diagnostic method as described later. The diagnostic kit of this invention comprises at least one of the aforementioned antigens (1) to (9) or at least one of the aforementioned antigens (10) to (14) and may comprise a chromogenic substrate or luminescent substrate serving as a substrate of an enzyme-labeled anti-IgE antibody and the enzyme or a biotinylated anti-IgE antibody and an avidin-conjugated dye that binds to the biotin. Also, a fluorescent-labeled anti-IgE antibody may be used. In the diagnostic kit of this invention, the antigen may be provided in a state immobilized on a carrier. The diagnostic kit of this invention may also be provided together with instructions on the procedure for diagnosis or a package containing said instructions.

In another embodiment, the diagnostic kit comprises a companion diagnostic agent for an allergy to fish. The companion diagnostic agent is used to analyze the reactivity of a pharmaceutical product for the purpose of identifying a patient in which the pharmaceutical product is expected to work or a patient at risk for serious side effects from the pharmaceutical product, or optimizing a therapy using the pharmaceutical product. Here, examples of optimizing a therapy include determination of the dosage and administration, decision of stopping the administration, determination of the allergen component to be used for the induction of immune tolerance, and the like.

The present invention further provides a composition for diagnosing an allergy to a fish, comprising at least one of the aforementioned antigens (1) to (9) or at least one of the aforementioned antigens (10) to (14). The diagnostic composition of this invention can be used in a diagnostic method as described below. The diagnostic composition of this invention may further comprise a pharmaceutically acceptable carrier and/or additives commonly used with the antigen of this invention depending on the need.

In one embodiment, the present invention provides a method for diagnosing an allergy to a fish in a subject, the method comprising:
(i) contacting a sample obtained from the subject with an antigen;
(ii) detecting binding between an IgE antibody present in the sample obtained from the subject and the antigen; and
(iii) when the binding between the IgE antibody in the subject and the antigen is detected, diagnosing the subject as being allergic to a fish;
wherein the antigen is at least one of the proteins identified as the aforementioned antigens (1) to (9) or at least one of the proteins identified as the aforementioned antigens (10) to (14). In this method, each of the steps (i) and (ii) is performed as described above regarding each of the corresponding steps of the method for providing an indicator for diagnosing an allergy to a fish.

In another embodiment, the present invention provides a method for diagnosing an allergy to a fish in a subject, the method comprising administering to the subject at least one of the aforementioned antigens (1) to (9) or at least one of the aforementioned antigens (10) to (14). This method may be performed in the form of a skin test characterized by applying the antigen onto the skin. Examples of the skin test include various forms of tests, such as: a prick test in which a diagnostic composition is applied onto the skin and then a tiny prick to such an extent as not to provoke bleeding is made in the skin to allow an antigen to infiltrate into the skin, thereby observing a skin reaction; a scratch test in which a diagnostic composition is applied onto the skin and then the skin is lightly scratched to observe a reaction; a patch test in which a diagnostic composition in the form of cream, ointment, etc. is applied onto the skin to observe a reaction; and an intracutaneous test in which an antigen is administered intracutaneously to observe a reaction. The method for infiltrating a diagnostic composition into the skin in a prick test or a scratch test may be a method involving bringing a tip of a lancet in contact with a diagnostic composition and wounding the skin by pricking the skin with the contact site to infiltrate the composition. If a skin reaction such as swelling occurs in a skin portion to which the antigen has been applied, the subject of interest is diagnosed as having an allergy to a fish. The amount of the antigen to be applied to the skin in such tests can be, for example, not more than 100 µg per dose.

In the allergic diagnosis, a load test for the purpose of identifying the antigen is often performed. At least one of the aforementioned antigens (1) to (9) or at least one of the aforementioned antigens (10) to (14) may be used as an active ingredient of a load test for diagnosing an allergy to fish. Here, the antigen protein to be used in the load test may be a protein that has been expressed and purified and may be a protein that has been expressed in a food and a food material, for example, rice-based vaccine produced by transforming rice with a gene of a cedar pollen antigen to express the antigen protein therein.

In yet another embodiment, the present invention provides at least one of the aforementioned antigens (1) to (9) or at least one of the aforementioned antigens (10) to (14) for use in the diagnosis of an allergy to a fish. Here, the present invention encompasses the provision of at least one of the aforementioned antigens (1) to (9) or (10) to (14) mixed with a known antigen.

In still another embodiment, the present invention provides a use of at least one of the aforementioned antigens (1) to (9) or at least one of the aforementioned antigens (10) to (14) for the production of a diagnostic agent for an allergy to a fish.

### Pharmaceutical composition and treatment method

The present invention provides a pharmaceutical composition comprising at least one of the aforementioned antigens (1) to (9) or at least one of the aforementioned antigens (10) to (14).

In one embodiment, the aforementioned pharmaceutical composition is used for the treatment of an allergy to fish.

The present invention also provides a method for treating an allergy to fish, the method comprising administering at least one of the aforementioned antigens (1) to (9) or at least one of the aforementioned antigens (10) to (14) to a patient in need of a treatment for an allergy to a fish.

In another embodiment, the present invention provides at least one of the aforementioned antigens (1) to (9) or at least one of the aforementioned antigens (10) to (14) for use in the treatment of an allergy to fish. In yet another embodiment, the present invention provides use of at least one of the aforementioned antigens (1) to (9) or at least one of the aforementioned antigens (10) to (14) for the production of a therapeutic agent for an allergy to fish.

In the process of allergy treatment, a hyposensitization therapy aiming at inducing immunological tolerance by administering an antigen to a patient is often adopted. At least one of the aforementioned antigens (1) to (9) or at least one of the aforementioned antigens (10) to (14) can be used as an active ingredient for a hyposensitization therapy for allergy to fish. Here, the antigen protein to be used in the hyposensitization therapy may be a protein that has been expressed and purified and may be a protein that has been expressed in a food and a food material, for example, rice-based vaccine produced by transforming rice with a gene of a cedar pollen antigen to express the antigen protein therein.

The pharmaceutical composition of the present invention can be administered by common administration routes. Examples of common administration routes include oral, sublingual, percutaneous, intracutaneous, subcutaneous, intravascular, intranasal, intramuscular, intraperitoneal, and intrarectal administrations.

The pharmaceutical composition of the present invention can be used as a pharmaceutical composition to which a commonly used pharmaceutically acceptable adjuvant or excipient or any other additives (e.g., stabilizer, solubilizer, emulsifier, buffer, preservative, colorant) are added by a conventional method together with the antigen of this invention depending on the need. The dosage form of the pharmaceutical composition can be selected by those skilled in the art as appropriate depending on the administration route. The pharmaceutical composition can be in the form of, for example, tablet, capsule, troche, sublingual tablet, parenteral injection, intranasal spray, poultice, solution, cream, lotion, or suppository. The administration dose, frequency and/or period of the pharmaceutical composition of this invention can be selected by a physician as appropriate depending on the administration route and the patient's condition and characteristics such as age and body weight. For example, the pharmaceutical composition may be administered to an adult patient at a dose of not more than 100 µg per dose. The administration interval can be, for example, once daily, once weekly, twice monthly, once per three months or so. The administration period can be, for example, several weeks to several years. The pharmaceutical composition may be administered in such a manner that the dose is increased in incremental steps over the administration period.

### Tester

The present invention provides a tester comprising an antibody for at least one of the aforementioned antigens (1) to (9) or at least one of the aforementioned antigens (10) to (14).

The antibody can be prepared by a conventional method: For example, the antibody may be prepared by immunizing a mammal such as rabbit with one of the aforementioned antigens (1) to (9) or one of the aforementioned antigens (10) to (14). The antibody may be an IgE antibody, a polyclonal antibody, a monoclonal antibody, or an antigen-binding fragment thereof (e.g., Fab, F(ab')₂, Fab').

Further, in the aforementioned tester, the antibody may be provided in a form bound to a carrier. The type of the carrier is not particularly limited as long as it is usable for detection of binding between an antibody and an antigen. Any given carrier known to those skilled in the art can be used.

Examples of a method for determining the presence or absence of an antigen include:
- A method in which a prepared tester comprising an IgE antibody is contacted with a sample obtained from a food or food material, and ELISA or the like method is, for example, used to detect whether there is a binding between the IgE antibody and an antigen in the sample, and if the binding between the IgE antibody and the antigen is detected, it is determined that the antibody remains in the food or food material, of interest.
- A method in which proteins are extracted from a food or food material, and the proteins are subjected to electrophoresis, and it is detected with an antibody whether there is a spot band of the antigen.
- A method in which a food or food material is infiltrated into a filter paper or the like and an antibody solution is reacted with the filter paper to detect an antigen present in the food.

The present invention encompasses, in another embodiment, a tester for determining the presence or absence of an antigen for an allergy to fish in an object of interest, the tester comprising a primer having a nucleotide sequence complementary to at least one part of one of the nucleotide sequences set forth in SEQ ID NO: 1, 4, 9, 18, 25, 32, 42, 55, 60, 69, 109, 120, 137, or 143. Without limiting, the primer has a nucleotide sequence complementary to preferably 12 residues, 15 nucleotides, 20 nucleotides, or 25 nucleotides in the sequence of a 3' terminal or middle portion of at least one part of one sequence of the nucleotide sequences set forth in, for example, SEQ ID NO: 1, 4, 9, 18, 25, 32, 42, 55, 60, 69, 109, 120, 137, or 143. Particularly when mRNA is targeted, the tester comprises a poly A tail-complementarity primer. In a preferred embodiment, the tester comprising the primer may further comprise a primer comprising a nucleotide sequence of a 5' terminal portion of at least one sequence of the nucleotide sequences set forth in SEQ ID NO: 1, 4, 9, 18, 25, 32, 42, 55, 60, 69, 109, 120, 137, or 143, preferably a nucleotide sequence consisting of 12 nucleotides, 15 nucleotides, 20 nucleotides, 25 nucleotides.

For example, the presence or absence of the antigen is determined by amplifying DNA or cDNA by polymerase chain reaction (PCR) including RT-PCR by using the aforementioned primer with DNA or mRNA obtained from fish as a template and comparing the amplified DNA or cDNA sequence(s) with SEQ ID NO: 1, 4, 9, 18, 25, 32, 42, 55, 60, 69, 109, 120, 137, or 143. Examples of the method of the amplification of mRNA by PCR include RACE and the like. In the determination, the antigen is determined to be present when the comparison between the amplified DNA or cDNA and SEQ ID NO: 1, 4, 9, 18, 25, 32, 42, 55, 60, 69, 109, 120, 137, or 143 indicates the presence of point mutation(s) encoding the same amino acid(s), or when the amino acid sequence encoded by the amplified DNA or cDNA has 70% or more, preferably 80, 90, 95, 98, or 99% or more identity with an amino acid sequence of SEQ ID NO: 2, 5, 10, 19, 26, 33, 43, 56, 61, 70, 110, 121, 138, or 144, even if the nucleotide sequence of the amplified DNA or cDNA is modified from any of the nucleotide sequences of SEQ ID NO: 1, 4, 9, 18, 25, 32, 42, 55, 60, 69, 109, 120, 137, or 143 by insertion, deletion, substitution, or addition of nucleotide(s).

In one embodiment, the aforementioned tester is used to determine the presence or absence of an antigen in food materials (fish or fish eggs) or in products of interest in a food production line. The tester may also be used for quality inspection of production lines and pre-shipment products by manufacturers, or may be used for self-checking of the presence or absence of an antigen in a food or food material of interest by consumers.

### Allergen-free food and the like

The present invention provides fish or fish eggs in which at least one of the aforementioned antigens (1) to (9) or at least one of the aforementioned antigens (10) to (14) is eliminated or reduced, a processed product of the fish or the fish eggs or fish which lay the fish eggs or have hatched from the eggs.

The method for eliminating or reducing the antigen of the present invention in fish, fish eggs, the processed product of the fish or the fish eggs or the fish which lay the fish eggs or have hatched from the eggs is not limited. The elimination or reduction of the antigen of the present invention may be conducted by any method, as long as the method permits the elimination or reduction of the antigen.

For example, the fish or fish eggs in which the antigen of the present invention is eliminated or reduced may be obtained by preparing fish or fish eggs in which the expression of the antigen of the present invention is knocked out, using a gene knock-out technique.

As the gene knock-out technique, there can be used any methods known to those skilled in the art. For example, Oishi, et al. (Scientific Reports, Vol.6, Article number: 23980, 2016, doi:10.1038/srep23980) describes that the genome editing technique CRISPER/Cas9 is applied to primordial germ cells to obtain individual animals deficient in an allergen gene. The fish or fish eggs devoid of the antigen of this invention may also be obtained by using the same technique. Moreover, fish or fish eggs in which the antigen of the present invention is eliminated or reduced may be obtained by mating by artificial insemination with fish or fish eggs that do not contain the antigen or contains a low content of the antigen. The artificial mating of fish or fish eggs can be performed by a conventional method.

The processed product of the fish or fish eggs in which the antigen of the present invention is eliminated or reduced may be a processed product made from fish in which the antigen of the present invention is eliminated or reduced. In the case of using ordinary fish or fish eggs as a source ingredient, a treatment for removing or reducing the antigen of the present invention is performed before or after preparation of a processed product of fish or fish eggs. Examples of a method of eliminating or reducing the antigen of the present invention in a processed product of fish or fish eggs made from ordinary fish or fish eggs include methods for eliminating the protein component in a food or a food material, such as elution with a high pressure treatment and a neutral salt solution and hot steam and methods for hydrolysis, denaturation, or amino acid modification (chemical modification or elimination of side chains) by heat treatment and acid treatment. The fish in which the antigen of the present invention is eliminated or reduced may be fish that have hatched and grown from the aforementioned fish eggs in which the antigen of the present invention is eliminated or reduced. Moreover, the fish eggs in which the antigen of the present invention is eliminated or reduced may be those obtained from the aforementioned fish in which the antigen of the present invention is eliminated or reduced.

### EXAMPLES

The following describes examples of the present invention. The technical scope of this invention is not limited by these examples.

### Example 1: Confirmation of a protein pattern

Proteins contained in salmon were investigated using a two-dimensional electrophoresis method described below.

### Protein extraction

The extraction and the purification of proteins contained in salmon were performed as follows. A solubilization agent (Mammalian Lysis Buffer (MCLI), SIGMA) was added to flesh of salmon and proteins were extracted, and then a urea buffer was added to obtain a protein extract. The composition of the urea buffer is as follows.
30 mM Tris
2 M Thiourea
7 M Urea
4% (w/v) CHAPS:
3 - [(3 -cholamidopropyl)dimethylammonio]propanesulfonate
Moderate amount of dilute hydrochloric acid

Distilled water was added and the total volume was adjusted to 100 mL. pH was 8.5.

Subsequently, 25 µg each in terms of protein weight was mixed to obtain an extract.

Thereafter, the precipitation procedure was repeated twice using a 2D-CleanUP Kit (produced by GE). In the first round of precipitation, the collected liquid protein extract was precipitated by adding TCA (trichloroacetic acid) thereto and the precipitated product produced by this procedure (TCA-precipitated product) was collected. In the second round of precipitation, the TCA-precipitated product collected above was further precipitated by adding acetone thereto and the precipitated product (sample) produced by this procedure was collected.

### Preparation of a sample solution

Part of the collected sample (40 µg on a protein weight basis) was dissolved in 150 µL of a DeStreak Rehydration Solution (produced by GE), which is a swelling buffer for first-dimensional isoelectric focusing gels, thereby obtaining a sample solution for first-dimensional isoelectric focusing (sample solution for swelling). The constituents of the DeStreak Rehydration Solution are as mentioned below.
7M thiourea
2M urea
4% (w/v) of CHAPS
0.5% (v/v) IPG buffer; produced by GE
Moderate amount of BPB (bromophenol blue)

### Impregnation of the sample into first-dimensional isoelectric focusing gels

First-dimensional isoelectric focusing gel strips (Immobiline Drystrip IPG gels (pH3-10NL); produced by GE) were immersed in 140 µL of the foregoing sample solution for first-dimensional isoelectric focusing (sample solution for swelling) and impregnated with the solution at room temperature overnight.

In this example, an IPGphor electrophoresis system produced by GE was used.

An electrophoresis tray was filled with silicone oil. Filter paper moisten with water was positioned at both ends of the gel strips impregnated with the sample, and the gel strips were set in the electrophoresis tray such that the gel strips were covered with silicone oil. Electrodes were placed on the gel strips with the filter paper intervening therebetween.

The maximum current of the isoelectric focusing system was set to 75 µA per gel strip, and the first-dimensional isoelectric focusing was carried out according to the following voltage program: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

### SDS equilibration of isoelectric focusing gels

After the aforementioned first-dimensional isoelectric focusing was done, the gel strips were taken out of the isoelectric focusing system, immersed in an equilibration buffer containing a reducing agent, and shaken at room temperature for 15 minutes. The constituents of the equilibration buffer containing the reducing agent are as mentioned below.
100 mM Tris-HCl (pH 8.0)
6M urea
30% (v/v) glycerol
2% (w/v) SDS
1% (w/v) DTT

Next, the equilibration buffer containing the reducing agent was removed, and then the gel strips were immersed in an equilibration buffer containing an alkylating agent and shaken at room temperature for 15 minutes to obtain SDS-equilibrated gels. The constituents of the equilibration buffer containing the alkylating agent are as mentioned below.
100 mM Tris-HCl (pH 8.0)
6M urea
30% (v/v) glycerol
2% (w/v) SDS
2.5% (w/v) iodoacetamide

### Second-dimensional SDS-PAGE

In this example, the XCell SureLock Mini-Cell electrophoresis system produced by Life Technologies was used. The second-dimensional electrophoresis gels used were NuPAGE 4-12% Bis-Tris Gels produced by Life Technologies. Also, an electrophoresis buffer composed of the following constituents was prepared and used.
50 mM MOPS
50 mM Tris base
0.1% (w/v) SDS
1 mM EDTA

Further, an agarose solution for gel adhesion was used in this example, which was prepared by dissolving 0.5% (w/v) Agarose S (produced by Nippon Gene Co., Ltd.) and a moderate amount of BPB (bromophenol blue) in the electrophoresis buffer.

SDS-PAGE wells were washed well with the electrophoresis buffer, and then the buffer used for the washing was removed. Next, the washed wells were charged with the fully dissolved agarose solution for gel adhesion. Next, the SDS-equilibrated gel strips were immersed in agarose and closely adhered to second-dimensional electrophoresis gels using tweezers. After it was confirmed that agarose was fully fixed with the gels being closely adhered to each other, electrophoresis was performed at a constant voltage of 200 V for about 45 minutes.

### Fluorescent staining of gels

The gels were fluorescently stained with SYPRO Ruby (produced by Life Technologies).

First, an airtight container to be used was washed well in advance with 98% (v/v) ethanol. The electrophoresed second-dimensional electrophoresis gel strips were taken out of the SDS-PAGE system, placed onto the washed airtight container, and treated twice by immersion in 50% (v/v) methanol and 7% (v/v) aqueous solution containing acetic acid for 30 minutes. Then, a further immersion treatment was done for 10 minutes, with the solution being replaced by water. Next, the second-dimensional electrophoresis gel strips were immersed in 40 mL of SYPRO Ruby and shaken at room temperature overnight. Thereafter, the SYPRO Ruby was removed, and then the second-dimensional electrophoresis gel strips were washed with water and shaken in 10% (v/v) methanol and 7% (v/v) aqueous solution containing acetic acid for 30 minutes. Further shaking was done for at least 30 minutes, with the solution being replaced by water.

### Analysis

The second-dimensional electrophoresis gels obtained through the foregoing series of treatments were subjected to fluorescent image scanning on Typhoon 9500 (produced by GE). The left panel of Fig. 1A illustrates the result of two-dimensional electrophoresis of proteins contained in flesh of salmon. Molecular weight marker bands are found at the left of the panels. The positions of the bands indicate particular molecular weights (KDa).

### Example 2: Identification of antigens by immunoblotting (1)

Identification of antigens by immunoblotting was carried out by taking all the steps up to the step of "Second-dimensional SDS-PAGE" as described above in Example 1, followed by the steps of "Transfer to membrane", "Immunoblotting" and "Analysis" as described below.

### Transfer to membrane

Transfer to membrane was done using the following transfer system and transfer buffer.
Transfer system: XCell SureLock Mini-Cell and XCell II Blot Module (produced by Life Technologies)
Transfer buffer: NuPAGE Transfer Buffer (X20) (produced by Life Technologies), used in a form diluted 20-fold with milliQ water.

To be specific, proteins in the second-dimensional electrophoresis gels were transferred to a membrane (PVDF membrane) according to the following procedure.
(1) The PVDF membrane was immersed in 100% methanol followed by milliQ water, and then moved into the transfer buffer to hydrophilize the PVDF membrane.
(2) After sponge, filter paper, the second-dimensional electrophoresis gels prepared by second-dimensional SDS-PAGE, the hydrophilized PVDF membrane, filter paper, and sponge were put in place in this order, the transfer system was energized at a constant voltage of 30 V for one hour.

### Immunoblotting

Immunoblotting of the membrane was carried out using, as a primary antibody, a serum sample from a patient 1 with a fish allergy or a serum sample from a non-fish-allergic subject. This Fish-allergic patient 1 is a patient diagnosed as immediate-type allergy to fish. This patient developed allergic symptoms to salmon, horse mackerel, sea eel, black porgy, mackerel, sea bream, cod, and young yellowtail and was positive in a prick test.

Immunoblotting of the membrane was carried out according to the following procedure.
(1) The transferred membrane was shaken in a 5% skim milk/PBST solution (a PBS buffer containing 0.1% Tween 20 nonionic surfactant) at room temperature for one hour.
(2) The membrane was left to stand in a solution of 5% serum as a primary antibody in 5% skim milk/PBST at room temperature for one hour.
(3) The membrane was washed with a PBST solution (5 min. × 3 times).
(4) The membrane was left to stand in a solution of 1:5000 dilution of the secondary antibody, anti-human IgE-HRP (horseradish peroxidase), with a 5% skim milk/PBST solution at room temperature for one hour.
(5) The membrane was washed with a PBST solution (5 min. × 3 times).
(6) The membrane was left to stand in Pierce Western Blotting Substrate Plus (produced by Thermo Fisher Scientific) for 5 minutes.

### Analysis

The membrane obtained through the foregoing series of treatments was subjected to fluorescent image scanning on Typhoon 9500 (produced by GE).

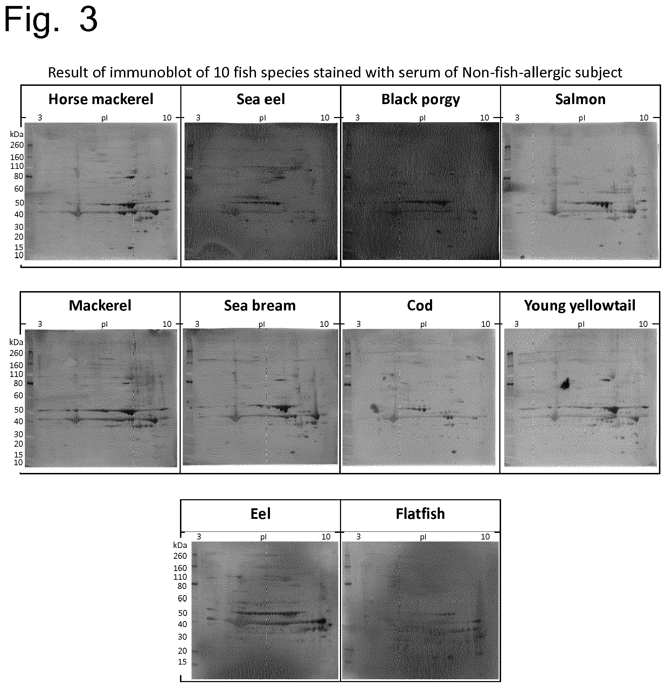

The immunoblot obtained with the serum from Fish-allergic patient 1 was compared with that obtained with the control serum from Non-fish-allergic subject. Nine spots that are different from those with the serum of Non-fish-allergic subject (Fig. 3) and different from the known salmon allergen proteins were detected on an immunoblot of the proteins contained in flesh of salmon with the serum of Fish-allergic patient 1 (Fig. 1A, right panel). The detected spots are indicated in the immunoblot (Fig. 1A, right panel).

The molecular weights and isoelectric points of the 9 spots are as follows.
Spot 1: Molecular weight 90 to 120 kDa, pI 3.0 to 6.0
Spot 2: Molecular weight 120 to 160 kDa, pI 4.0 to 7.0
Spot 3: Molecular weight 90 to 120 kDa, pI 5.0 to 8.0
Spot 4: Molecular weight 90 to 120 kDa, pI 6.0 to 8.0
Spot 5: Molecular weight 60 to 90 kDa, pI 3.0 to 6.0
Spot 6: Molecular weight 70 to 100 kDa, pI 5.0 to 7.0
Spot 7: Molecular weight 40 to 70 kDa, pI 4.0 to 7.0
Spot 8: Molecular weight 40 to 70 kDa, pI 4.0 to 7.0
Spot 9: Molecular weight 20 to 50 kDa, pI 4.0 to7.0

### Example 3: Mass spectrometry and identification of antigens (1)

The amino acid sequences of the antigens that form the above nine protein spots were identified by mass spectroscopy.

To be specific, protein extraction and mass spectroscopy were done by the following procedure.
(1) Flesh of salmon was subjected to protein extraction, two-dimensional electrophoresis and transfer to membrane by following the procedures described in Example 1 and 2, and the resulting membrane was stained by shaking in a solution of 0.008% Direct blue in 40% ethanol and 10% acetic acid.
(2) Then, the membrane was decolorized by repeating a 5-minute treatment with 40% ethanol and 10% acetic acid three times, washed with water for 5 minutes, and then dried by air.
(3) A protein spot of interest was cut out with a clean cutter blade and put into a centrifugal tube. The cut membrane was subjected to hydrophilization with 50 µL of methanol, followed by washing with 100 µL of water twice and then centrifugal cleaning. Thereafter, 20 µL of 20 mM NH₄HCO₃ and 50% acetonitrile were added.
(4) 1 µL of 1 pmol/µl lysyl endopeptidase (produced by WAKO) was added, and the solution was left to stand at 37°C for 60 minutes and then collected in a new centrifugal tube. After 20 µL of 20 mM NH₄HCO₃ and 70% acetonitrile were added to the membrane, the membrane was immersed therein at room temperature for 10 minutes, and the resulting solution was further collected. The solution was dissolved with 0.1% formic acid and 10 µL of 4% acetonitrile and transferred to a tube.
(5) The collected solution was dried under reduced pressure, dissolved with 15 µL of solution A (a 0.1% formic acid/4% acetonitrile solution), and analyzed by mass spectroscopy (ESI-TOF6600, produced by AB Sciex).
(6) Identification of proteins based on the MS data obtained with the mass spectrometer was done by searching NCBI or UniProt.

### Results

The mass spectrometry of the spots resulted in the detection of the following amino acid sequences.
Spot 1: the amino acid sequences set forth in SEQ ID NO: 3
Spot 2: the amino acid sequences set forth in SEQ ID NO: 6 to 8
Spot 3: the amino acid sequences set forth in SEQ ID NO: 11 to 17
Spot 4: the amino acid sequences set forth in SEQ ID NO: 20 to 24
Spot 5: the amino acid sequences set forth in SEQ ID NO: 27 to 31
Spot 6: the amino acid sequences set forth in SEQ ID NO: 34 to 41
Spot 7: the amino acid sequences set forth in SEQ ID NO: 44 to 54
Spot 8: the amino acid sequences set forth in SEQ ID NO: 57 to 59
Spot 9: the amino acid sequences set forth in SEQ ID NO: 62 to 68

Furthermore, the spots were identified as the following proteins by the analysis of the MS data obtained from the mass spectrometer for spot 1 at NCBI and for spots 2 to 9 with UniProt.
Spot 1: Alpha-actinin-3 (NCBI protein accession number XP_014051545.1, DNA accession number XM_014196070.1) (amino acid sequence: SEQ ID NO: 2, encoding nucleotide sequence: SEQ ID NO: 1)
Spot 2: EEF1A2 binding protein-like (UniProt protein accession number B5RI29, DNA accession number ACH85270.1) (amino acid sequence: SEQ ID NO: 5, encoding nucleotide sequence: SEQ ID NO: 4)
Spot 3: Alpha-1,4-glucan phosphorylase (UniProt protein accession number B5DG55, DNA accession number ACH70729.1) (amino acid sequence: SEQ ID NO: 10, encoding nucleotide sequence: SEQ ID NO: 9)
Spot 4: Elongation factor 2 (UniProt protein accession number C0H9N2, DNA accession number ACN10751.1) (amino acid sequence: SEQ ID NO: 19, encoding nucleotide sequence: SEQ ID NO: 18)
Spot 5: Heat shock cognate 70 kDa protein (UniProt protein accession number B5X3U6, DNA accession number ACI33977.1) (amino acid sequence: SEQ ID NO: 26, encoding nucleotide sequence: SEQ ID NO: 25)
Spot 6: Serotransferrin (UniProt protein accession number P79815, DNA accession number BAA13759.1) (amino acid sequence: SEQ ID NO: 33, encoding nucleotide sequence: SEQ ID NO: 32)
Spot 7: Myosin binding protein H-like (UniProt protein accession number B5DG45, DNA accession number ACH70719.1) (amino acid sequence: SEQ ID NO: 43, encoding nucleotide sequence: SEQ ID NO: 42)
Spot 8: Desmin (Fragment) (UniProt protein accession number Q8UWF1, DNA accession number CAC83053.1) (amino acid sequence: SEQ ID NO: 56, encoding nucleotide sequence: SEQ ID NO: 55)
Spot 9: Capping protein (Actin filament) muscle Z-line beta (UniProt protein accession number B5DFX6, DNA accession number ACH70650.1) (amino acid sequence: SEQ ID NO: 61, encoding nucleotide sequence: SEQ ID NO: 60)

### Example 4: Confirmation of antigens in other fish species (1)

Using flesh of the 9 fish species, horse mackerel, sea eel, black porgy, mackerel, sea bream, cod, young yellowtail, eel, and flatfish, other than salmon, antigens were confirmed by the same procedure as the procedure described in Example 1 and Example 2.

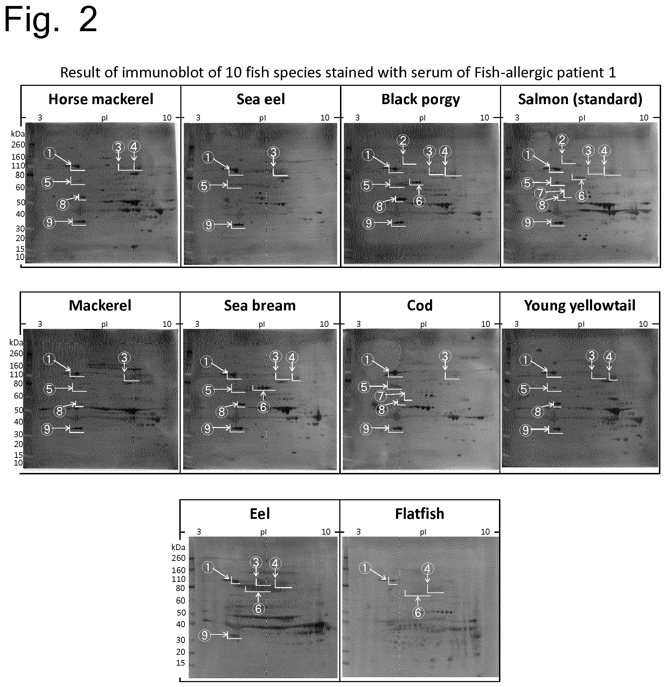

Immunoblots obtained with the serum from Fish-allergic patient 1 was compared with that obtained with the control serum from Non-fish-allergic subject. Spots that are different from those with the serum from Non-fish-allergic subject (Fig. 3) and correspond to a part of the 9 spots obtained in salmon were detected on immunoblots (Fig. 2) of proteins contained in flesh of each of the 9 fish species stained with the serum from Fish-allergic patient 1.

Spots detected in each fish species are as follows.
Horse mackerel: Spots 1, 3-5, 8, and 9
Sea eel: Spots 1, 3, 5, and 9
Black porgy: Spots 1-6, 8, and 9
Mackerel: Spots 1, 3, 5, 8, and 9
Sea bream: Spots 1, 3-6, 8, and 9
Cod: Spots 1, 3, 5, 7-9
Young yellowtail: Spots 1, 3-5, 8, 9
Eel: Spots 1, 3, 4, 6, 9
Flatfish: Spots 1, 4, 6

### Example 5: Confirmation of antigens by immunoblotting (2)

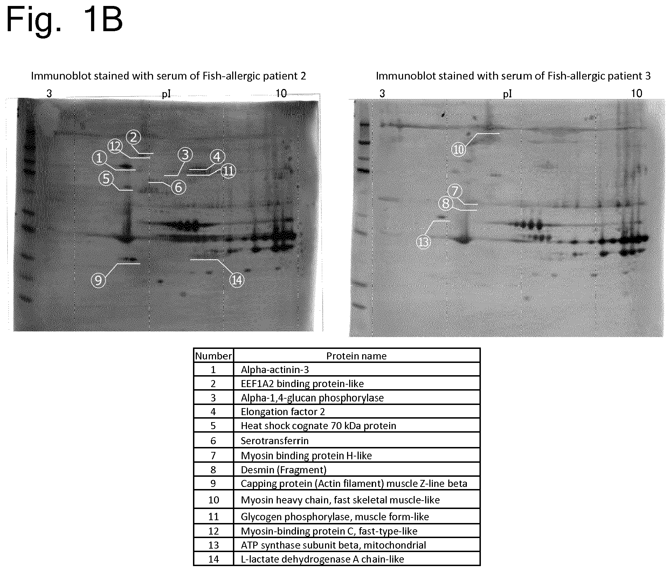

An immunoblot obtained with serum from a fish-allergic patient was compared with that obtained with the control serum from Non-fish-allergic subject in the same way as in Example 2. Three new spots (Spots 11, 12, 14) that are different from those with the serum from Non-fish-allergic subject (Fig. 3) and different from the known salmon allergen proteins, other than 7 (spots 1-6, 9) of the 9 spots detected in Example 2, were detected on an immunoblot (Fig. 1B, left panel) of proteins contained in flesh of salmon stained with the serum from Fish-allergic patient 2. The total 10 spots detected are indicated in the immunoblot (Fig. 1B, left panel).

The molecular weights and isoelectric points of the 3 new spots detected are as follows.
Spot 11: Molecular weight 90 to 110, pI 6.5 to 7.0
Spot 12: Molecular weight 120 to 140, pI 5.0 to 6.0
Spot 14: Molecular weight 30 to 40, pI 6.5 to 7.5

### Example 6: Mass spectrometry and identification of antigens (2)

The amino acid sequences of the antigens that form the 3 spots newly detected were identified by mass spectroscopy in the same way as in Example 3.

The mass spectrometry of the spots resulted in the detection of the following amino acid sequences.
Spot 11: Amino acid sequences set forth in SEQ ID NOs: 111 to 119
Spot 12: Amino acid sequences set forth in SEQ ID NOs: 122 to 136
Spot 14: Amino acid sequences set forth in SEQ ID NOs: 145 to 149

Furthermore, the spots were identified as the following proteins by the analysis of the MS data obtained from the mass spectrometer for the spots at NCBI.
Spot 11: Glycogen phosphorylase, muscle form-like (NCBI protein accession number XP_013984904.1, DNA accession number XM_014129429.1) (amino acid sequence: SEQ ID NO: 110, encoding nucleotide sequence: SEQ ID NO: 109)
Spot 12: Myosin-binding protein C, fast-type-like (NCBI protein accession number XP_014014310.1, DNA accession number XM_014158835.1) (amino acid sequence: SEQ ID NO: 121, encoding nucleotide sequence: SEQ ID NO: 120)
Spot 14: L-lactate dehydrogenase A chain-like (NCBI protein accession number XP_014003141.1, DNA accession number XM_014147666.1) (amino acid sequence: SEQ ID NO: 144, encoding nucleotide sequence: SEQ ID NO: 143)

### Example 7: Confirmation of antigens by immunoblotting (3)

An immunoblot obtained with the serum from a fish-allergic patient was compared with that obtained with the control serum from Non-fish-allergic subject, in the same way as in Example 2. Two new spots (Spots 10, 13) that are different from those with the serum from Non-fish-allergic subject (Fig. 3) and different from the known salmon allergen proteins, other than 2 (Spots 7, 8) of the 9 spots detected in Example 2, were detected on an immunoblot (Fig. 1B, right panel) of proteins contained in flesh of salmon stained with the serum from Fish-allergic patient 3. The total 4 spots detected are indicated in the immunoblot (Fig. 1B, right panel).

The molecular weights and isoelectric points of the 2 new spots detected are as follows.
Spot 10: Molecular weight 200 to 230, pI 4.5 to 5.5
Spot 13: Molecular weight 45 to 55, pI 4.0 to 5.5

### Example 8: Mass spectrometry and identification of antigens (3)

The amino acid sequences of the antigens that form the 2 spots newly detected were identified by mass spectroscopy in the same way as in Example 3.

The mass spectrometry of the spots resulted in the detection of the following amino acid sequences.
Spot 10: Amino acid sequences set forth in SEQ ID NOs: 71 to 108
Spot 13: Amino acid sequences set forth in SEQ ID NOs: 139 to 142

Furthermore, the spots were identified as the following proteins by the analysis of the MS data obtained from the mass spectrometer for the spots at NCBI.
Spot 10: Myosin heavy chain, fast skeletal muscle-like (NCBI protein accession number XP_014039990.1, DNA accession number XM 014184515.1) (amino acid sequence: SEQ ID NO: 70, encoding nucleotide sequence: SEQ ID NO: 69)
Spot 13: ATP synthase subunit beta, mitochondrial (NCBI protein accession number XP_014007238.1, DNA accession number XM_014151763.1) (amino acid sequence: SEQ ID NO: 138, encoding nucleotide sequence: SEQ ID NO: 137)

### Example 9: Confirmation of antigens in other fish species (2)

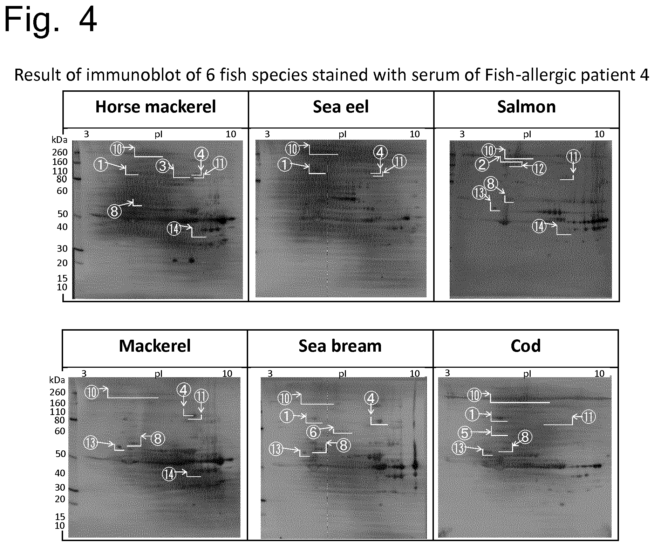

Using flesh of the 6 fish species, horse mackerel, sea eel, salmon, mackerel, sea bream, and cod, the antigen was confirmed in the same way as in Example 4. Immunoblots obtained with the serum from Fish-allergic patient 4 was compared with that obtained with the control serum from Non-fish-allergic subject. Spots that are different from those with the serum from Non-fish-allergic subject (Fig. 3) and correspond to a part of the 14 spots obtained in salmon with the serum from Fish-allergic patients 1 to 3 were detected on immunoblots (Fig. 4) of proteins contained in flesh of each of the 6 fish species stained with the serum from Fish-allergic patient 4.

Spots detected in each fish species are as follows.
Horse mackerel: Spots 1, 3, 4, 8, 10, 11, and 14
Sea eel: Spots 1, 4, 10, and 11
Salmon: Spots 2, 8, and 10-14
Mackerel: Spots 4, 8, 10, 11, 13, and 14
Sea bream: Spots 1, 4, 6, 8, 10, and 13
Cod: Spots 1, 5, 8, 10, 11, and 13

### INDUSTRIAL APPLICABILITY

The present invention can provide novel antigens of an allergy to fish, methods and kits for diagnosing an allergy to fish, a pharmaceutical composition comprising the antigen, and fish or fish eggs in which the antigen is eliminated or reduced, a processed product of the fish or fish eggs, or fish which lay the fish eggs or have hatched from the eggs. The present invention can further provide a tester for determining the presence or absence of a fish antigen in an object of interest.

## Claims

1. A kit for diagnosing a fish allergy, the kit comprising, as an antigen, at least one protein defined in any one of the following (1) to (9):
(1)
(1A) Alpha-actinin-3 or a variant thereof, which is a protein defined below in any of (1A-a) to (1A-e) which is an antigen for the fish allergy:
(1A-a) a protein comprising an amino acid sequence with deletion, substitution, insertion or addition of one or several amino acids in SEQ ID NO: 2;
(1A-b) a protein comprising an amino acid sequence having at least 70% identity to the amino acid sequence of SEQ ID NO: 2;
(1A-c) a protein comprising an amino acid sequence encoded by a nucleotide sequence with deletion, substitution, insertion or addition of one or several nucleotides in SEQ ID NO: 1;
(1A-d) a protein comprising an amino acid sequence encoded by a nucleotide sequence having at least 70% identity to the nucleotide sequence of SEQ ID NO: 1; or
(1A-e) a protein comprising an amino acid sequence encoded by a nucleic acid that hybridizes under stringent conditions with a nucleic acid having a nucleotide sequence complementary to the nucleotide sequence of SEQ ID NO: 1; or
(1B) a protein comprising an amino acid sequence of SEQ ID NO: 2 or 3;
(2)
(2A) EEF1A2 binding protein-like or a variant thereof, which is a protein defined below in any of (2A-a) to (2A-e) which is an antigen for the fish allergy:
(2A-a) a protein comprising an amino acid sequence with deletion, substitution, insertion or addition of one or several amino acids in SEQ ID NO: 5;
(2A-b) a protein comprising an amino acid sequence having at least 70% identity to the amino acid sequence of SEQ ID NO: 5;
(2A-c) a protein comprising an amino acid sequence encoded by a nucleotide sequence with deletion, substitution, insertion or addition of one or several nucleotides in SEQ ID NO: 4;
(2A-d) a protein comprising an amino acid sequence encoded by a nucleotide sequence having at least 70% identity to the nucleotide sequence of SEQ ID NO: 4; or
(2A-e) a protein comprising an amino acid sequence encoded by a nucleic acid that hybridizes under stringent conditions with a nucleic acid having a nucleotide sequence complementary to the nucleotide sequence of SEQ ID NO: 4; or
(2B) a protein comprising at least one amino acid sequence selected from the group consisting of SEQ ID NOs: 5-8;
(3)
(3A) Alpha-1,4-glucan phosphorylase or a variant thereof, which is a protein defined below in any of (3A-a) to (3A-e) which is an antigen for the fish allergy:
(3A-a) a protein comprising an amino acid sequence with deletion, substitution, insertion or addition of one or several amino acids in SEQ ID NO: 10;
(3A-b) a protein comprising an amino acid sequence having at least 70% identity to the amino acid sequence of SEQ ID NO: 10;
(3A-c) a protein comprising an amino acid sequence encoded by a nucleotide sequence with deletion, substitution, insertion or addition of one or several nucleotides in SEQ ID NO: 9;
(3A-d) a protein comprising an amino acid sequence encoded by a nucleotide sequence having at least 70% identity to the nucleotide sequence of SEQ ID NO: 9; or
(3A-e) a protein comprising an amino acid sequence encoded by a nucleic acid that hybridizes under stringent conditions with a nucleic acid having a nucleotide sequence complementary to the nucleotide sequence of SEQ ID NO: 9; or
(3B) a protein comprising at least one amino acid sequence selected from the group consisting of SEQ ID NOs: 10-16;
(4)
(4A) Elongation factor 2 or a variant thereof, which is a protein defined below in any of (4A-a) to (4A-e) which is an antigen for the fish allergy:
(4A-a) a protein comprising an amino acid sequence with deletion, substitution, insertion or addition of one or several amino acids in SEQ ID NO: 19;
(4A-b) a protein comprising an amino acid sequence having at least 70% identity to the amino acid sequence of SEQ ID NO: 19;
(4A-c) a protein comprising an amino acid sequence encoded by a nucleotide sequence with deletion, substitution, insertion or addition of one or several nucleotides in SEQ ID NO: 18;
(4A-d) a protein comprising an amino acid sequence encoded by a nucleotide sequence having at least 70% identity to the nucleotide sequence of SEQ ID NO: 18; or
(4A-e) a protein comprising an amino acid sequence encoded by a nucleic acid that hybridizes under stringent conditions with a nucleic acid having a nucleotide sequence complementary to the nucleotide sequence of SEQ ID NO: 18; or
(4B) a protein comprising at least one amino acid sequence selected from the group consisting of SEQ ID NOs: 19-24;
(5)
(5A) Heat shock cognate 70 kDa protein or a variant thereof, which is a protein defined below in any of (5A-a) to (5A-e) which is an antigen for the fish allergy:
(5A-a) a protein comprising an amino acid sequence with deletion, substitution, insertion or addition of one or several amino acids in SEQ ID NO: 26;
(5A-b) a protein comprising an amino acid sequence having at least 70% identity to the amino acid sequence of SEQ ID NO: 26;
(5A-c) a protein comprising an amino acid sequence encoded by a nucleotide sequence with deletion, substitution, insertion or addition of one or several nucleotides in SEQ ID NO: 25;
(5A-d) a protein comprising an amino acid sequence encoded by a nucleotide sequence having at least 70% identity to the nucleotide sequence of SEQ ID NO: 25; or
(5A-e) a protein comprising an amino acid sequence encoded by a nucleic acid that hybridizes under stringent conditions with a nucleic acid having a nucleotide sequence complementary to the nucleotide sequence of SEQ ID NO: 25; or
(5B) a protein comprising at least one amino acid sequence selected from the group consisting of SEQ ID NOs: 26-31;
(6)
(6A) Serotransferrin or a variant thereof, which is a protein defined below in any of (6A-a) to (6A-e) which is an antigen for the fish allergy:
(6A-a) a protein comprising an amino acid sequence with deletion, substitution, insertion or addition of one or several amino acids in SEQ ID NO: 33;
(6A-b) a protein comprising an amino acid sequence having at least 70% identity to the amino acid sequence of SEQ ID NO: 33;
(6A-c) a protein comprising an amino acid sequence encoded by a nucleotide sequence with deletion, substitution, insertion or addition of one or several nucleotides in SEQ ID NO: 32;
(6A-d) a protein comprising an amino acid sequence encoded by a nucleotide sequence having at least 70% identity to the nucleotide sequence of SEQ ID NO: 32; or
(6A-e) a protein comprising an amino acid sequence encoded by a nucleic acid that hybridizes under stringent conditions with a nucleic acid having a nucleotide sequence complementary to the nucleotide sequence of SEQ ID NO: 32; or
(6B) a protein comprising at least one amino acid sequence selected from the group consisting of SEQ ID NOs: 33-41;
(7)
(7A) Myosin binding protein H-like or a variant thereof, which is a protein defined below in any of (7A-a) to (7A-e) which is an antigen for the fish allergy:
(7A-a) a protein comprising an amino acid sequence with deletion, substitution, insertion or addition of one or several amino acids in SEQ ID NO: 43;
(7A-b) a protein comprising an amino acid sequence having at least 70% identity to the amino acid sequence of SEQ ID NO: 43;
(7A-c) a protein comprising an amino acid sequence encoded by a nucleotide sequence with deletion, substitution, insertion or addition of one or several nucleotides in SEQ ID NO: 42;
(7A-d) a protein comprising an amino acid sequence encoded by a nucleotide sequence having at least 70% identity to the nucleotide sequence of SEQ ID NO: 42; or
(7A-e) a protein comprising an amino acid sequence encoded by a nucleic acid that hybridizes under stringent conditions with a nucleic acid having a nucleotide sequence complementary to the nucleotide sequence of SEQ ID NO: 42; or
(7B) a protein comprising at least one amino acid sequence selected from the group consisting of SEQ ID NOs: 43-54,
(8)
(8A) Desmin (Fragment) or a variant thereof, which is a protein defined below in any of (8A-a) to (8A-e) which is an antigen for the fish allergy:
(8A-a) a protein comprising an amino acid sequence with deletion, substitution, insertion or addition of one or several amino acids in SEQ ID NO: 56;
(8A-b) a protein comprising an amino acid sequence having at least 70% identity to the amino acid sequence of SEQ ID NO: 56;
(8A-c) a protein comprising an amino acid sequence encoded by a nucleotide sequence with deletion, substitution, insertion or addition of one or several nucleotides in SEQ ID NO: 55;
(8A-d) a protein comprising an amino acid sequence encoded by a nucleotide sequence having at least 70% identity to the nucleotide sequence of SEQ ID NO: 55; or
(8A-e) a protein comprising an amino acid sequence encoded by a nucleic acid that hybridizes under stringent conditions with a nucleic acid having a nucleotide sequence complementary to the nucleotide sequence of SEQ ID NO: 55; or
(8B) a protein comprising at least one amino acid sequence selected from the group consisting of SEQ ID NOs: 56-59;
(9)
(9A) Capping protein (Actin filament) muscle Z-line beta or a variant thereof, which is a protein defined below in any of (9A-a) to (9A-e) which is an antigen for the fish allergy:
(9A-a) a protein comprising an amino acid sequence with deletion, substitution, insertion or addition of one or several amino acids in SEQ ID NO: 61;
(9A-b) a protein comprising an amino acid sequence having at least 70% identity to the amino acid sequence of SEQ ID NO: 61;
(9A-c) a protein comprising an amino acid sequence encoded by a nucleotide sequence with deletion, substitution, insertion or addition of one or several nucleotides in SEQ ID NO: 60;
(9A-d) a protein comprising an amino acid sequence encoded by a nucleotide sequence having at least 70% identity to the nucleotide sequence of SEQ ID NO: 60; or
(9A-e) a protein comprising an amino acid sequence encoded by a nucleic acid that hybridizes under stringent conditions with a nucleic acid having a nucleotide sequence complementary to the nucleotide sequence of SEQ ID NO: 60; or
(9B) a protein comprising at least one amino acid sequence selected from the group consisting of SEQ ID NOs: 61-68.

2. A kit for diagnosing a fish allergy, the kit comprising, as an antigen, at least one protein defined in any one of the following (10) to (14):
(10)
(10A) Myosin heavy chain, fast skeletal muscle-like or a variant thereof, which is a protein defined below in any of (10A-a) to (10A-e) which is an antigen for the fish allergy:
(10A-a) a protein comprising an amino acid sequence with deletion, substitution, insertion or addition of one or several amino acids in SEQ ID NO: 70;
(10A-b) a protein comprising an amino acid sequence having at least 70% identity to the amino acid sequence of SEQ ID NO: 70;
(10A-c) a protein comprising an amino acid sequence encoded by a nucleotide sequence with deletion, substitution, insertion or addition of one or several nucleotides in SEQ ID NO: 69;
(10A-d) a protein comprising an amino acid sequence encoded by a nucleotide sequence having at least 70% identity to the nucleotide sequence of SEQ ID NO: 69; or
(10A-e) a protein comprising an amino acid sequence encoded by a nucleic acid that hybridizes under stringent conditions with a nucleic acid having a nucleotide sequence complementary to the nucleotide sequence of SEQ ID NO: 69; or
(10B) a protein comprising at least one amino acid sequence selected from the group consisting of SEQ ID NOs: 70-108;
(11)
(11A) Glycogen phosphorylase, muscle form-like or a variant thereof, which is a protein defined below in any of (11A-a) to (11A-e) which is an antigen for the fish allergy:
(11A-a) a protein comprising an amino acid sequence with deletion, substitution, insertion or addition of one or several amino acids in SEQ ID NO: 110;
(11A-b) a protein comprising an amino acid sequence having at least 70% identity to the amino acid sequence of SEQ ID NO: 110;
(11A-c) a protein comprising an amino acid sequence encoded by a nucleotide sequence with deletion, substitution, insertion or addition of one or several nucleotides in SEQ ID NO: 109;
(11A-d) a protein comprising an amino acid sequence encoded by a nucleotide sequence having at least 70% identity to the nucleotide sequence of SEQ ID NO: 109; or
(1 1A-e) a protein comprising an amino acid sequence encoded by a nucleic acid that hybridizes under stringent conditions with a nucleic acid having a nucleotide sequence complementary to the nucleotide sequence of SEQ ID NO: 109; or
(11B) a protein comprising at least one amino acid sequence selected from the group consisting of SEQ ID NOs: 110-119;
(12)
(12A) Myosin-binding protein C, fast-type-like or a variant thereof, which is a protein defined below in any of (12A-a) to (12A-e) which is an antigen for the fish allergy:
(12A-a) a protein comprising an amino acid sequence with deletion, substitution, insertion or addition of one or several amino acids in SEQ ID NO: 121;
(12A-b) a protein comprising an amino acid sequence having at least 70% identity to the amino acid sequence of SEQ ID NO: 121;
(12A-c) a protein comprising an amino acid sequence encoded by a nucleotide sequence with deletion, substitution, insertion or addition of one or several nucleotides in SEQ ID NO: 120;
(12A-d) a protein comprising an amino acid sequence encoded by a nucleotide sequence having at least 70% identity to the nucleotide sequence of SEQ ID NO: 120; or
(12A-e) a protein comprising an amino acid sequence encoded by a nucleic acid that hybridizes under stringent conditions with a nucleic acid having a nucleotide sequence complementary to the nucleotide sequence of SEQ ID NO: 120; or
(12B) a protein comprising at least one amino acid sequence selected from the group consisting of SEQ ID NOs: 121-136;
(13)
(13A) ATP synthase subunit beta, mitochondrial or a variant thereof, which is a protein defined below in any of (13A-a) to (13A-e) which is an antigen for the fish allergy:
(13A-a) a protein comprising an amino acid sequence with deletion, substitution, insertion or addition of one or several amino acids in SEQ ID NO: 138;
(13A-b) a protein comprising an amino acid sequence having at least 70% identity to the amino acid sequence of SEQ ID NO: 138;
(13A-c) a protein comprising an amino acid sequence encoded by a nucleotide sequence with deletion, substitution, insertion or addition of one or several nucleotides in SEQ ID NO: 137;
(13A-d) a protein comprising an amino acid sequence encoded by a nucleotide sequence having at least 70% identity to the nucleotide sequence of SEQ ID NO: 137; or
(13A-e) a protein comprising an amino acid sequence encoded by a nucleic acid that hybridizes under stringent conditions with a nucleic acid having a nucleotide sequence complementary to the nucleotide sequence of SEQ ID NO: 137; or
(13B) a protein comprising at least one amino acid sequence selected from the group consisting of SEQ ID NOs: 138-142;
(14)
(14A) L-lactate dehydrogenase A chain-like or a variant thereof, which is a protein defined below in any of (14A-a) to (14A-e) which is an antigen for the fish allergy:
(14A-a) a protein comprising an amino acid sequence with deletion, substitution, insertion or addition of one or several amino acids in SEQ ID NO: 144;
(14A-b) a protein comprising an amino acid sequence having at least 70% identity to the amino acid sequence of SEQ ID NO: 144;
(14A-c) a protein comprising an amino acid sequence encoded by a nucleotide sequence with deletion, substitution, insertion or addition of one or several nucleotides in SEQ ID NO: 143;
(14A-d) a protein comprising an amino acid sequence encoded by a nucleotide sequence having at least 70% identity to the nucleotide sequence of SEQ ID NO: 143; or
(14A-e) a protein comprising an amino acid sequence encoded by a nucleic acid that hybridizes under stringent conditions with a nucleic acid having a nucleotide sequence complementary to the nucleotide sequence of SEQ ID NO: 143; or
(14B) a protein comprising at least one amino acid sequence selected from the group consisting of SEQ ID NOs: 144-149.

3. A composition for diagnosing a fish allergy, the composition comprising, as an antigen, at least one of proteins as defined in any of (1) to (9) of claim 1 or at least one of proteins as defined in any of (10) to (14) of claim 2.

4. A method for providing an indicator for diagnosing a fish allergy in a subject, the method comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject has a fish allergy is provided;
wherein the antigen is at least one of proteins as defined in any of (1) to (9) of claim 1 or at least one of proteins as defined in any of (10) to (14) of claim 2.

5. A pharmaceutical composition comprising at least one of proteins as defined in any of (1) to (9) of claim 1 or at least one of proteins as defined in any of (10) to (14) of claim 2.

6. The pharmaceutical composition according to claim 5 for the treatment of an allergy to a fish.

7. A fish or a processed product of fish in which an antigen is eliminated or reduced, wherein the antigen is at least one of proteins as defined in any of (1) to (9) of claim 1 or at least one of proteins as defined in any of (10) to (14) of claim 2.

8. A tester for determining the presence or absence of a fish antigen in an object of interest, the tester comprising an antibody that binds to at least one of proteins as defined in any of (1) to (9) of claim 1 or at least one of proteins as defined in any of (10) to (14) of claim 2.

9. A tester for determining the presence or absence of an antigen causing an allergy to fish in an object of interest, the tester comprising a primer having a nucleotide sequence complementary to at least one part of one of the nucleotide sequences set forth in SEQ ID NO: 1, 4, 9, 18, 25, 32, 42, 55, 60, 69, 109, 120, 137, or 143.

10. A fish-derived antigen, which is at least one of proteins as defined in any of (1) to (9) of claim 1 or at least one of proteins as defined in any of (10) to (14) of claim 2 and causes an allergy to a fish.
